# EUROPEAN PATENT APPLICATION

(11) **EP 4 741 380 A1**
(43) Date of publication of application: **13.05.2026**
(21) Application number: 25789362.8
(22) Date of filing: 02.04.2025
(51) Int. Cl.: C07D 261/04, C07D 261/06, C07D 413/10, A01N 43/80, A01P 7/02, A01P 7/04

(54) **ISOXAZOLINE COMPOUND AND USE**

(30) Priority: 16.04.2024 CN 202410456463; 13.02.2025 CN 202510160698
(71) Applicant: NANJING PETMEDICINE TECHNOLOGY CO., LTD., Jiangsu 210032 (CN)
(72) Inventor: LI, Yi, Nanjing, Jiangsu 210032 (CN); CHEN, Chun, Nanjing, Jiangsu 210032 (CN); LIN, Hongzhi, Nanjing, Jiangsu 210032 (CN)
(74) Representative: J A Kemp LLP
(86) International application number: PCT/CN2025/086768
(87) International publication number: WO 2025/218493

(57) **Abstract**

Provided are an isoxazoline compound and use thereof. The isoxazoline compound has a structure represented by Formula 1. The compound can be used for preventing and treating parasite infections and killing agricultural pests, has a good prevention and treatment effect, and exhibits good pharmacokinetic properties in mice.

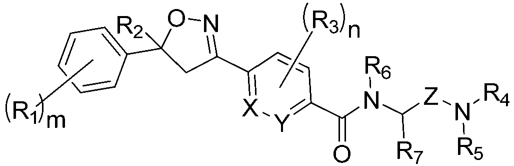

## Description

### TECHNICAL FIELD

The present application belongs to the technical field of pharmaceutical compounds and, in particular, relates to an isoxazoline compound and use thereof.

### BACKGROUND

Ectoparasites for animals cause great harm to the health of companion animals and the production performance of livestock and poultry. Effective control of the infection of animals with ectoparasites has long been a goal of the agricultural and veterinary sectors (WOODS D J, KNAUER C S. Discovery of veterinary antiparasitic agents in the 21st century: a view from industry [J]. Int J Parasitol, 2010, 40(10): 1177-81.). Isoxazoline insecticides are a new type of pest control drugs, and such drugs primarily act on the γ-aminobutyric acid (GABA)-gated chloride channels of invertebrates (MITA T, KIKUCHI T, MIZUKOSHI T, et al. Isoxazoline-substituted benzamide compound and noxious organism control agent [J]. WO Patent, 2005, 2005085216: A1. & OZOE Y, ASAHI M, OZOE F, et al. The antiparasitic isoxazoline A1443 is a potent blocker of insect ligand-gated chloride channels [J]. Biochemical and biophysical research communications, 2010, 391(1): 744-9.). The action sites of the isoxazoline insecticides differ from those of other insecticides acting on the chloride channels (such as dieldrin and fipronil), and the isoxazoline insecticides can overcome the resistance to existing insecticides. The Insecticide Resistance Action Committee (IRAC) classifies the isoxazoline insecticides into Group 30 (IRAC Group 30). After researchers from Nissan Chemical (Japan) and DuPont (USA) developed an insecticidal active ingredient, fluralaner, (CASIDA J E. Golden age of RyR and GABA-R diamide and isoxazoline insecticides: common genesis, serendipity, surprises, selectivity, and safety [J]. Chemical research in toxicology, 2015, 28(4): 560-6.), afoxolaner, sarolaner, and lotilaner were subsequently developed. Currently, the four isoxazoline drugs are used for ectoparasite control in pets, and the global annual sales of such antiparasitic drugs (including compound preparations) exceed 2 billion dollars. The isoxazoline drugs have become the mainstream products for routine parasite control in companion animals. Among the currently marketed "-laner" drugs, three share the same N-trifluoroethyl glycinamide tail chain. With an enhanced metabolic capability towards this fragment, insects may generate multi-drug resistance to "-laner" insecticides. Therefore, the development of a new generation of "-laner" insecticides that are safer, more convenient, more effective, and more environmentally friendly can provide new therapeutic options for pet owners while overcoming common resistance to antiparasitic drugs.

Additionally, isoxazoline compounds act on the GABA-gated chloride channels and also have a significant killing effect on agricultural pests, demonstrating a wide application potential in the field of pesticides.

### SUMMARY

The following is a summary of the subject matter described herein in detail. This summary is not intended to limit the scope of the claims.

In view of the defects in the existing art, an object of the present application is to provide an isoxazoline compound and use thereof.

To achieve the object, the present application adopts the technical solutions below.

In one aspect, the present application provides an isoxazoline compound. The isoxazoline compound has a structure represented by Formula I: 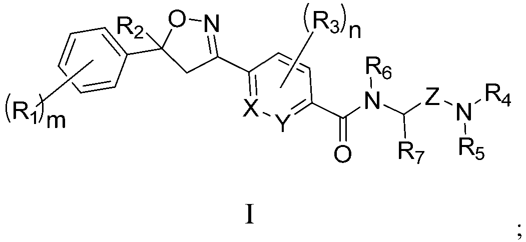 wherein
X is selected from -CH= or -N=;
Y is selected from -CH= or -N=;
Z is selected from 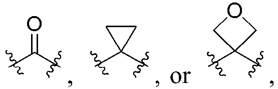 wherein the wavy line represents a linkage site of the group;
R₁ is selected from hydrogen, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, halogenated C₂ to C₆ alkynyl, halogen, cyano, nitro, -C(O)NRₐR_{b}, -C(O)Rₐ, -C(O)ORₐ, -ORₐ, -RₐOR_{b}, -OC(O)Rₐ, -OC(O)ORₐ, -OC(O)NRₐR_{b}, -NRₐR_{b}, -SRₐ, -S(O)Rₐ, -S(O)₂Rₐ, or a 3- to 10-membered saturated or unsaturated ring containing 0 to 3 heteroatoms, wherein the 3- to 10-membered saturated or unsaturated ring containing 0 to 3 heteroatoms is optionally substituted with 1 to 3 Rₐ;
R₂ is selected from hydrogen, halogen, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, or halogenated C₂ to C₆ alkynyl;
R₃ is selected from hydrogen, halogen, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, or halogenated C₂ to C₆ alkynyl;
R₄ is selected from hydrogen, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, halogenated C₂ to C₆ alkynyl, halogen, cyano, nitro, -C(O)NRₐR_{b}, -C(O)Rₐ, -C(O)ORₐ, -ORₐ, -RₐOR_{b}, -OC(O)Rₐ, -OC(O)ORₐ, -OC(O)NRₐR_{b}, -NRₐR_{b}, -SRₐ, -S(O)Rₐ, -S(O)₂Rₐ, or a 3- to 10-membered saturated or unsaturated ring containing 0 to 3 heteroatoms, wherein the 3- to 10-membered saturated or unsaturated ring containing 0 to 3 heteroatoms is optionally substituted with 1 to 3 Rₐ;
R₅ is selected from hydrogen, C₁ to C₆ alkyl, C₁ to C₆ alkyl substituted with 1 to 3 Rₐ, C₂ to C₆ alkenyl, C₂ to C₆ alkynyl, halogenated C₁ to C₆ alkyl, deuterated C₁ to C₆ alkyl, halogenated C₂ to C₆ alkenyl, halogenated C₂ to C₆ alkynyl, halogen, cyano, nitro, -C(O)NRₐR_{b}, -C(O)Rₐ, -C(O)ORₐ, -ORₐ, -RₐOR_{b}, -OC(O)Rₐ, -OC(O)ORₐ, -OC(O)NRₐR_{b}, -NRₐR_{b}, -SRₐ, -S(O)Rₐ, -S(O)₂Rₐ, or a 3- to 10-membered saturated or unsaturated ring containing 0 to 3 heteroatoms, wherein the 3- to 10-membered saturated or unsaturated ring containing 0 to 3 heteroatoms is optionally substituted with 1 to 3 Rₐ; or
R₄ and R₅, together with the nitrogen atom connecting them, form a 3- to 7-membered saturated or unsaturated ring optionally substituted with 1 to 8 Rₐ;
R₆ is selected from hydrogen, C₁ to C₆ alkyl, C₂ to C₆ alkenyl, halogenated C₁ to C₆ alkyl, C₁ to C₆ alkoxy, or deuterated C₁ to C₆ alkyl;
R₇ is selected from hydrogen, halogen, C₁ to C₆ alkyl, C₂ to C₆ alkenyl, C₂ to C₆ alkynyl, halogenated C₁ to C₆ alkyl, C₁ to C₆ alkoxy, deuterated C₁ to C₆ alkyl, halogenated C₂ to C₆ alkenyl, or halogenated C₂ to C₆ alkynyl;
Rₐ and R_{b} are each independently selected from hydrogen, deuterium, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, halogenated C₂ to C₆ alkynyl, halogen, cyano, nitro, amino, carboxyl, oxo, hydroxyl, hydroxyalkyl, alkoxy, halogenated alkoxy, deuterated alkoxy, C₃ to C₆ cycloalkyl, halogenated C₃ to C₆ cycloalkyl, alkoxy-substituted C₃ to C₆ cycloalkyl, C₃ to C₆ cycloalkyl substituted with 1 to 3 R_{c}, C₃ to C₆ heterocyclyl, halogenated C₃ to C₆ heterocyclyl, alkyl-substituted C₃ to C₆ heteroaryl, -S(O)₂R_{c}, -OR_{c}OR_{d}, -R_{c}OR_{d}, -C(O)R_{c}, or -OC(O)R_{c}, wherein R_{c} and R_{d} are each independently selected from halogen, C₁ to C₆ alkyl, or halogenated C₁ to C₆ alkyl;
m is 0, 1, 2, 3, 4, or 5; and
n is 0, 1, 2, 3, or 4.

In some embodiments, the isoxazoline compound has a structure represented by Formula Ia: 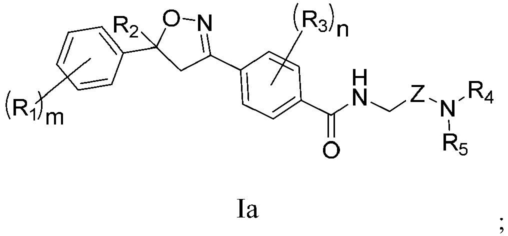 wherein Z, R₁, R₂, R₃, R₄, R₅, m, and n are defined the same as above.

In some other embodiments, the isoxazoline compound has a structure represented by Formula Ib: 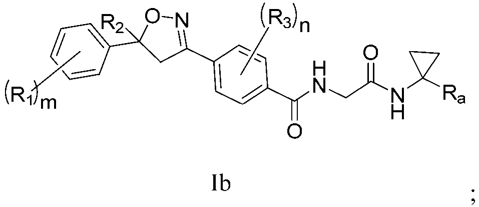
wherein R₁, R₂, R₃, m, and n are defined the same as above; and
Rₐ is selected from cyano or trifluoromethyl.

In some embodiments, R₁ is selected from hydrogen, halogen, C₁ to C₆ alkyl, C₁ to C₆ alkoxy, halogenated C₁ to C₆ alkyl, or halogenated C₁ to C₆ alkoxy.

In some other embodiments, R₁ is selected from hydrogen, fluorine, chlorine, methyl, methoxy, trifluoromethyl, or trifluoromethoxy.

In some other embodiments, R₂ is selected from hydrogen, halogen, C₁ to C₆ alkyl, or halogenated C₁ to C₆ alkyl.

In some other embodiments, R₂ is trifluoromethyl.

In some other embodiments, R₃ is selected from hydrogen, halogen, C₁ to C₆ alkyl, or halogenated C₁ to C₆ alkyl.

In some other embodiments, R₃ is methyl, chlorine, or fluorine.

In some other embodiments, R₄ is hydrogen.

In some other embodiments, R₅ is selected from 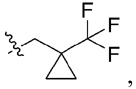 C₁ to C₆ alkyl, halogenated C₁ to C₆ alkyl, C₃ to C₆ cycloalkyl, halogenated C₃ to C₆ cycloalkyl, cyano-substituted C₃ to C₆ cycloalkyl, alkoxy-substituted C₃ to C₆ cycloalkyl, C₃ to C₆ cycloalkyl substituted with halogenated C₁ to C₆ alkyl, or C₃ to C₆ heterocyclyl substituted with halogenated C₁ to C₆ alkyl.

In some other embodiments, R₅ is selected from trifluoroethyl, cyclopropyl,

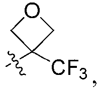

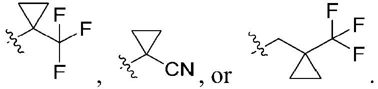

In some other embodiments, 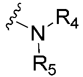 is selected from

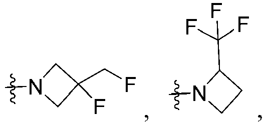

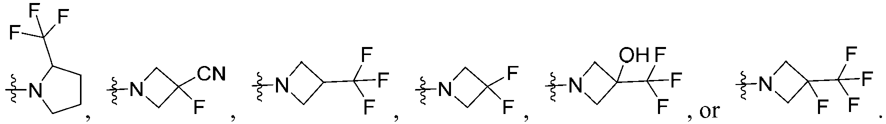

In some other embodiments, R₆ is selected from hydrogen or C₁ to C₆ alkyl.

In some other embodiments, R₇ is selected from hydrogen or C₁ to C₆ alkyl.

In some other embodiments, the isoxazoline compound is any one of the following compounds:

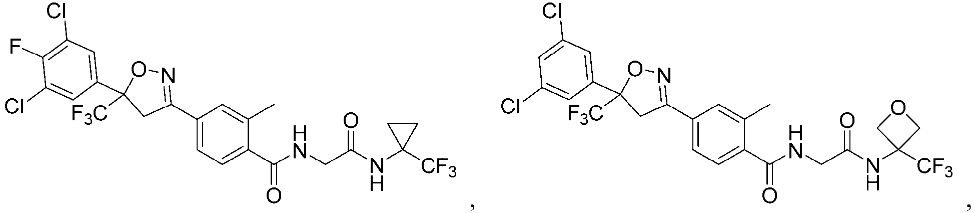

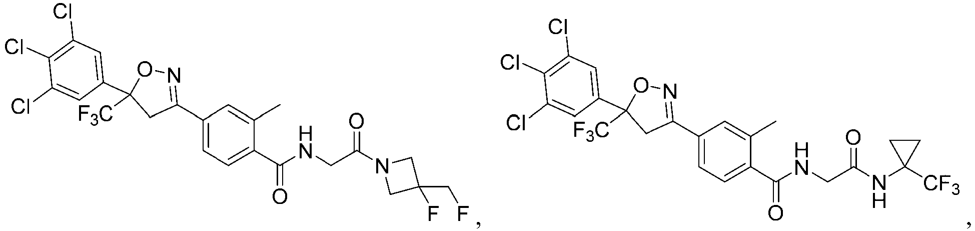

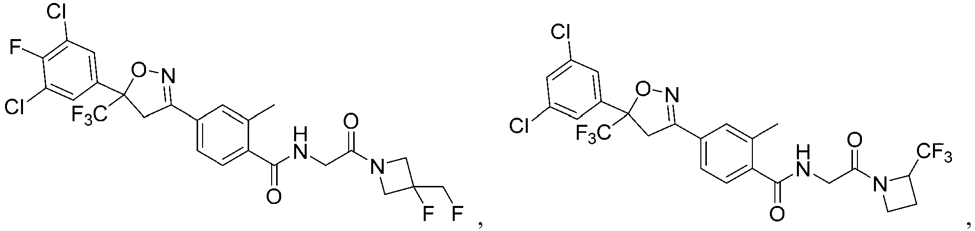

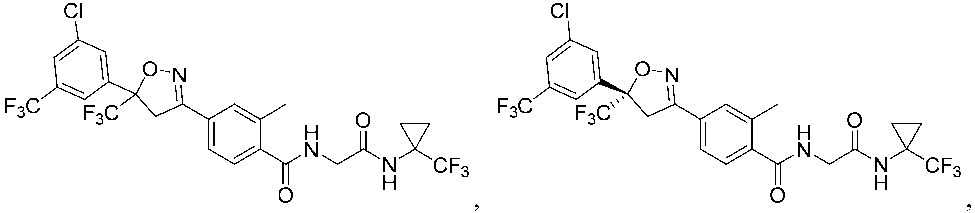

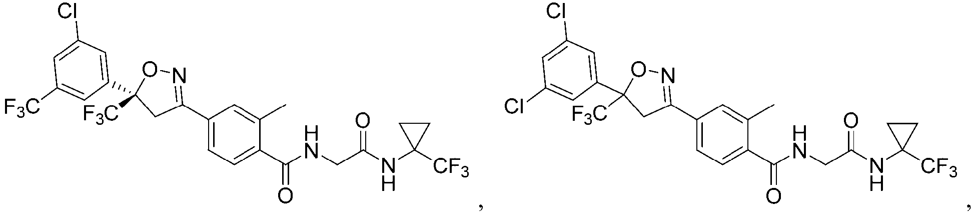

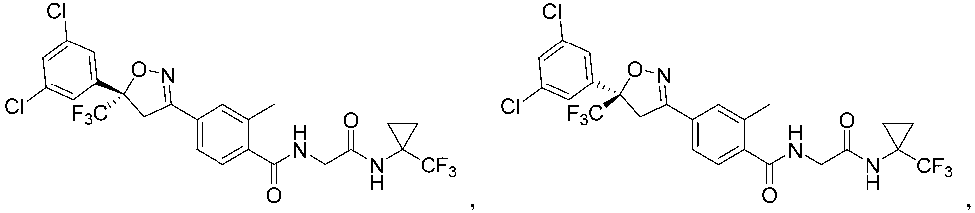

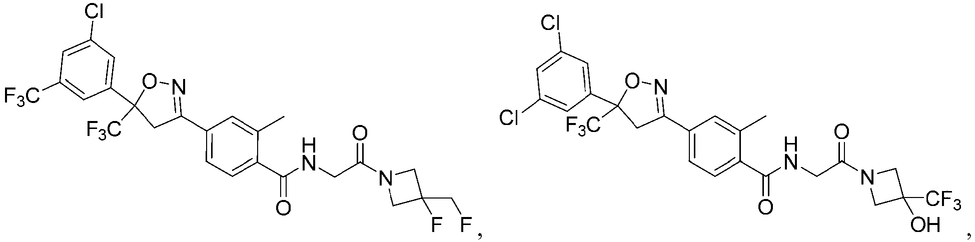

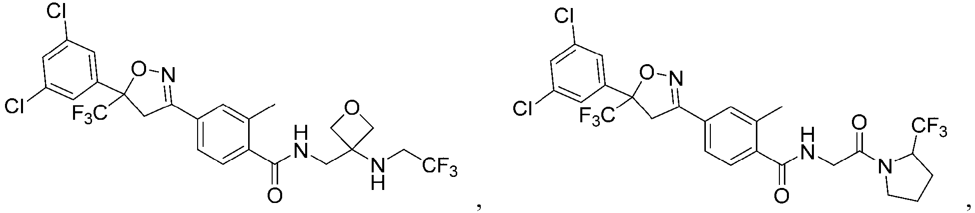

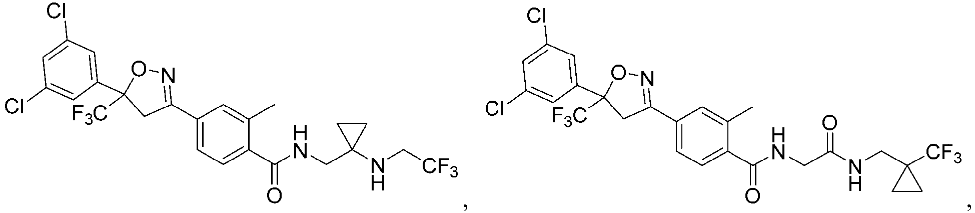

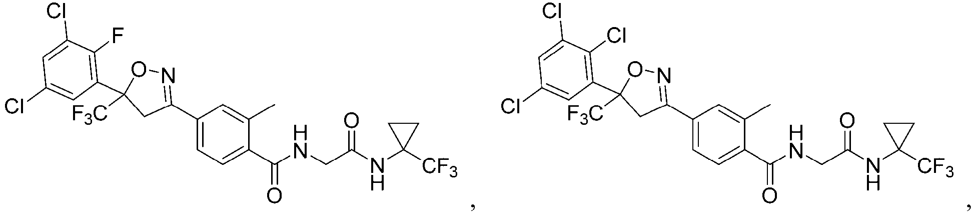

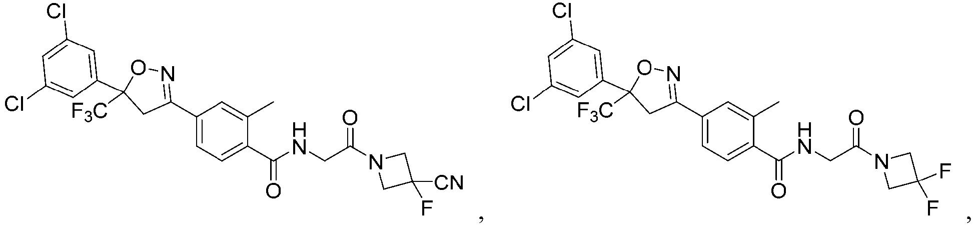

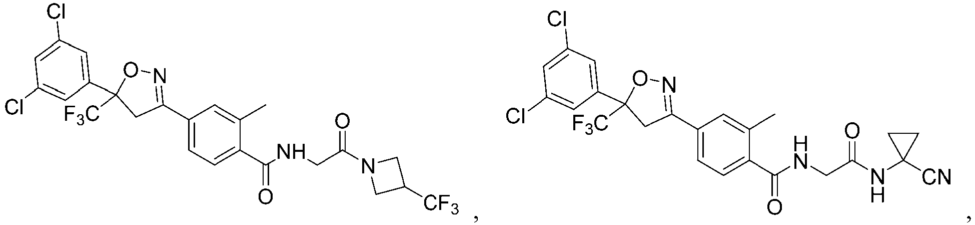

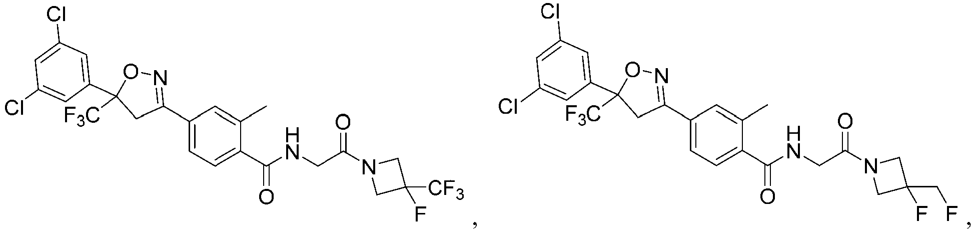

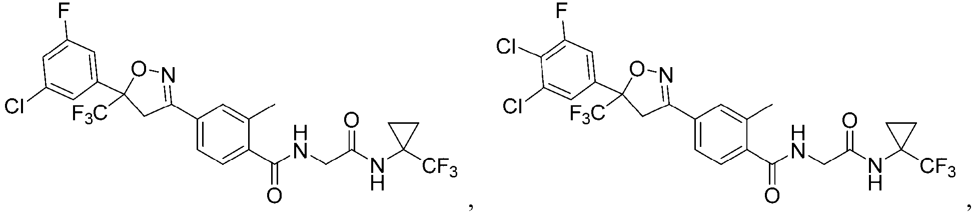

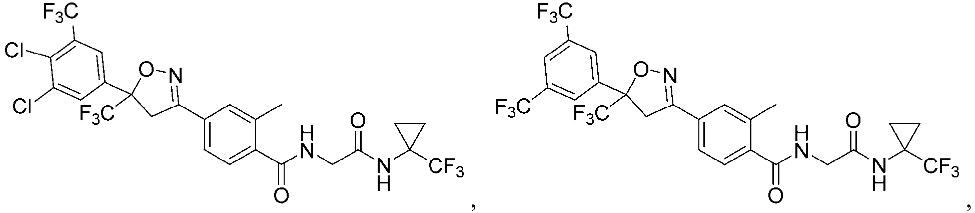

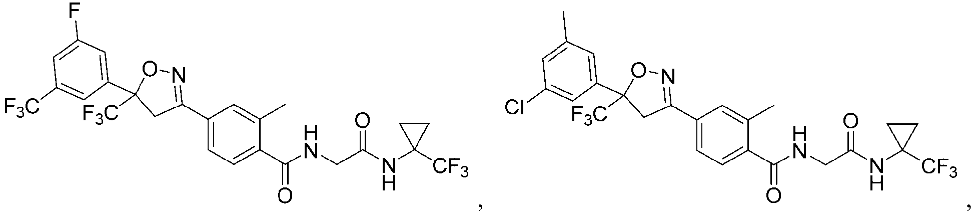

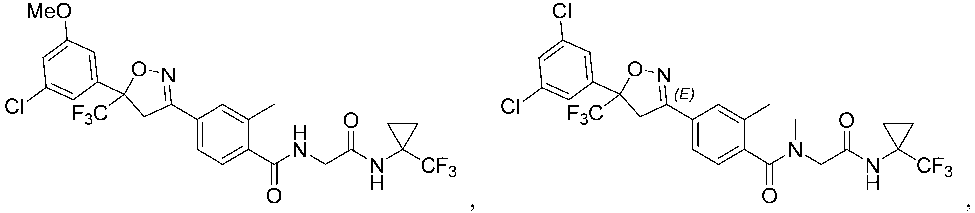

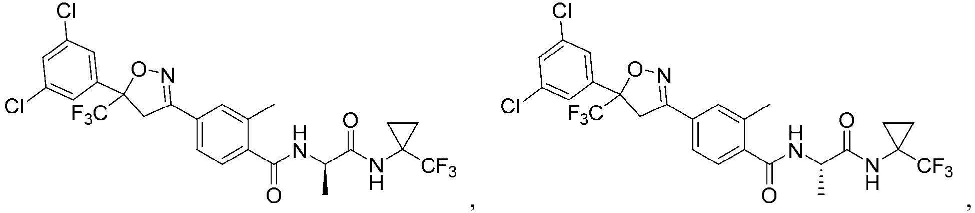

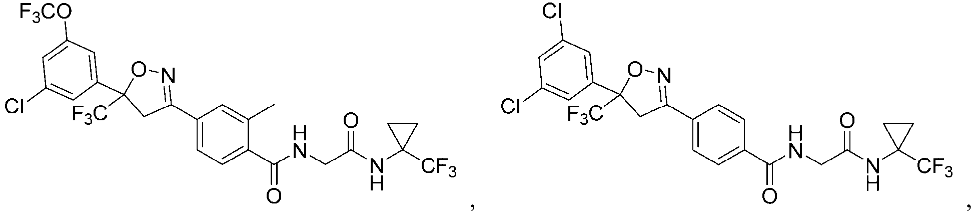

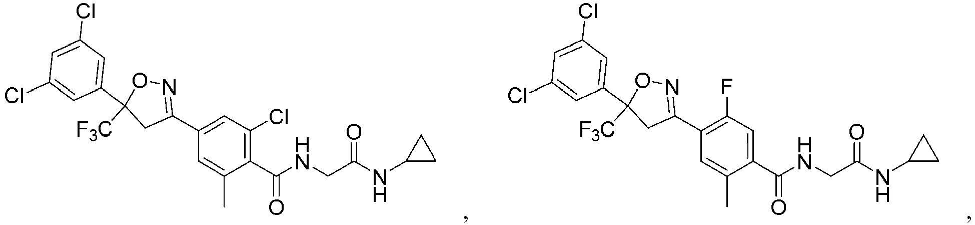

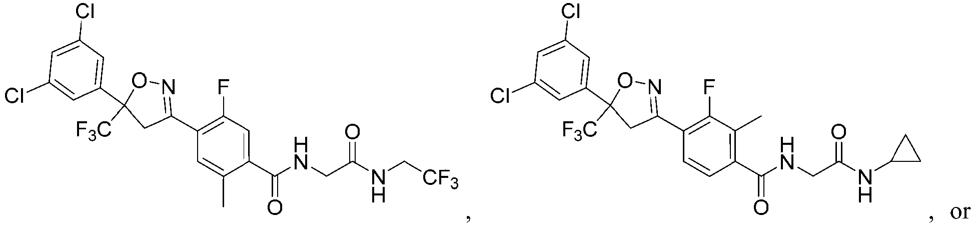

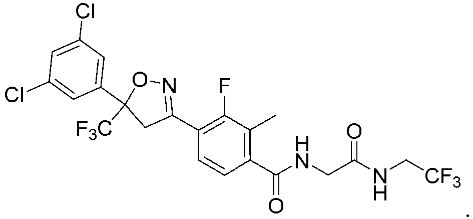

In further preferred embodiments, the isoxazoline compound is any one of the following compounds: 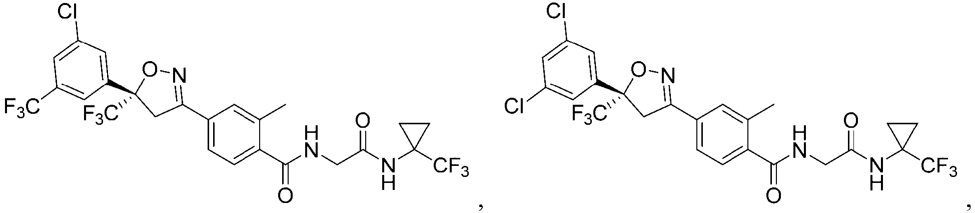 or

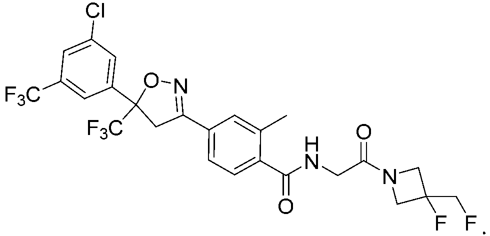

In another aspect, the present application further provides a tautomer, enantiomer, diastereomer, mesomer, racemate, or pharmaceutically acceptable salt of the preceding isoxazoline compound.

In another aspect, the present application further provides a pharmaceutical composition. The pharmaceutical composition comprises a therapeutically effective amount of the preceding isoxazoline compound or a tautomer, enantiomer, diastereomer, mesomer, racemate, or pharmaceutically acceptable salt thereof and a pharmaceutically acceptable carrier or excipient.

In another aspect, the present application provides use of the preceding isoxazoline compound, the tautomer, enantiomer, diastereomer, mesomer, racemate, or pharmaceutically acceptable salt thereof, or the preceding pharmaceutical composition for controlling an infection of a human or an animal with ectoparasites.

In another aspect, the present application provides a pesticide composition. The pesticide composition comprises an active ingredient and optionally at least one agromedically acceptable carrier and/or adjuvant, wherein the active ingredient is the preceding isoxazoline compound or the tautomer, enantiomer, diastereomer, mesomer, racemate, or pharmaceutically acceptable salt thereof, and a pharmaceutically acceptable carrier or excipient.

In another aspect, the present application provides a pesticide formulation made of the preceding isoxazoline compound or the tautomer, enantiomer, diastereomer, mesomer, racemate, or pharmaceutically acceptable salt thereof.

In another aspect, the present application further provides use of the preceding isoxazoline compound or the tautomer, enantiomer, diastereomer, mesomer, racemate, or pharmaceutically acceptable salt thereof for preparation of a product for controlling agricultural pests.

In further preferred embodiments, in the preceding use for preparation of the product for controlling agricultural pests, the agricultural pests are selected from at least one of mites, Lepidoptera, Diptera, Thysanoptera, Hemiptera, Isoptera, or Coleoptera.

### Terms

Unless otherwise stated, the terms used in the description and the claims have the meanings below.

The term "isomer" includes an enantiomeric form, a diastereomeric form, and a geometric (or conformational) isomeric form of a given structure. For example, the present application includes R and S configurations for each asymmetric center, Z and E double-bond isomers, Z and E conformational isomers, single stereochemical isomers as well as enantiomeric, diastereomeric, and geometric (or conformational) mixtures.

The term "pharmaceutically acceptable salt" refers to, for example, an acid addition salt and/or an alkali salt thereof. Suitable acid addition salts are formed by acids, which form non-toxic salts such as hydrochlorides/chlorides. Suitable alkali salts are formed by bases, which form non-toxic salts such as calcium and sodium salts. Hemi-salts of acids and bases may also be formed, such as hemisulfates and hemicalcium salts.

The term "therapeutically effective amount" refers to an amount of the compound of the present application that (i) treats a specific disease, condition, or disorder; (ii) attenuates, ameliorates, or eliminates one or more symptoms of a specific disease, condition, or disorder; or (iii) prevents or delays the onset of one or more symptoms of a specific disease, condition, or disorder described in the present application.

The term "pharmaceutically acceptable carrier or excipient" refers to a non-toxic carrier, adjuvant, or vehicle that does not impair the pharmacological activity of the compound formulated therewith.

The term "alkyl" refers to a saturated aliphatic hydrocarbyl group, which is a straight or branched chain group containing 1 to 20 carbon atoms, preferably alkyl containing 1 to 12 carbon atoms, and more preferably alkyl containing 1 to 6 carbon atoms. Non-limiting examples of lower alkyl containing 1 to 6 carbon atoms include methyl, ethyl, n-propyl, isopropyl, n-butyl, isobutyl, t-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, and 2,3-dimethylbutyl.

The term "alkenyl" refers to an aliphatic hydrocarbyl group having at least one carbon-carbon double bond, including a straight or branched chain group having at least one carbon-carbon double bond. In some embodiments, an alkenyl group has 2 to 20 carbon atoms, 2 to 10 carbon atoms, 2 to 6 carbon atoms, 3 to 6 carbon atoms, or 2 to 4 carbon atoms. For example, the term "C₂₋₆ alkenyl" includes an unsaturated straight or branched chain group having 2 to 6 carbon atoms (with at least one carbon-carbon double bond), including, but not limited to, vinyl, 1-propenyl, 2-propenyl (allyl), isopropenyl, 2-methyl-1-propenyl, 1-butenyl, and 2-butenyl.

The term "alkynyl" refers to an aliphatic hydrocarbyl group having at least one carbon-carbon triple bond, including a straight or branched chain group having at least one carbon-carbon triple bond. In some embodiments, an alkynyl group has 2 to 20 carbon atoms, 2 to 10 carbon atoms, 2 to 6 carbon atoms, or 3 to 6 carbon atoms. For example, "C₂₋₆ alkynyl" includes an unsaturated straight or branched chain group having 2 to 6 carbon atoms (with at least one carbon-carbon triple bond).

The term "alkoxy" refers to -O-alkyl and -O-unsubstituted cycloalkyl, wherein alkyl is defined as above. Non-limiting examples of alkoxy include methoxy, ethoxy, propoxy, butoxy, cyclopropoxy, cyclobutoxy, cyclopentoxy, and cyclohexoxy.

The term "cycloalkyl" refers to a saturated or partially unsaturated monocyclic or polycyclic hydrocarbon substituent. A cycloalkyl ring includes 3 to 20 carbon atoms, preferably 3 to 12 carbon atoms, more preferably 3 to 6 carbon atoms (e.g. 3, 4, 5, or 6 carbon atoms), and most preferably 5 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, and cyclooctyl. Polycyclic cycloalkyl includes spiro, fused, and bridged cycloalkyl groups.

The term "spirocycloalkyl" refers to a 5- to 20-membered polycyclic group with individual rings sharing one common carbon atom (called a spiro atom), wherein the rings may contain one or more double bonds, but no ring has a completely conjugated π-electron system. Spirocycloalkyl is preferably 6- to 14-membered and more preferably 7- to 10-membered (e.g., 7-, 8-, 9-, or 10-membered). According to the number of common spiro atoms shared between rings, spirocycloalkyl may be classified into monospirocycloalkyl, dispirocycloalkyl, or polyspirocycloalkyl, preferably monospirocycloalkyl and dispirocycloalkyl, and more preferably 4-membered/4-membered, 4-membered/5-membered, 4-membered/6-membered, 5-membered/5-membered, or 5-membered/6-membered monospirocycloalkyl.

The term "fused cycloalkyl" refers to a 5- to 20-membered full-carbon polycyclic group in which each ring in the system shares an adjacent pair of carbon atoms with another ring in the system, wherein one or more rings may contain one or more double bonds, but no ring has a completely conjugated π-electron system. Fused cycloalkyl is preferably 6- to 14-membered and more preferably 7- to 10-membered. According to the number of rings, fused cycloalkyl may be classified into bicyclic, tricyclic, tetracyclic, or polycyclic fused cycloalkyl, preferably bicyclic or tricyclic fused cycloalkyl, and more preferably 5-membered/5-membered or 5-membered/6-membered bicyclic fused cycloalkyl.

The term "bridged cycloalkyl" refers to a 5- to 20-membered full-carbon polycyclic group in which any two rings share two disconnected carbon atoms, wherein the rings may contain one or more double bonds, but no ring has a completely conjugated π-electron system. Bridged cycloalkyl is preferably 6- to 14-membered and more preferably 7- to 10-membered. According to the number of rings, bridged cycloalkyl may be classified into bicyclic, tricyclic, tetracyclic, or polycyclic bridged cycloalkyl, preferably bicyclic, tricyclic, or tetracyclic bridged cycloalkyl, and more preferably bicyclic or tricyclic bridged cycloalkyl.

The term "heterocyclyl" refers to a saturated or partially unsaturated monocyclic or polycyclic hydrocarbon substituent containing 3 to 20 ring atoms, wherein one or more ring atoms are heteroatoms selected from nitrogen, oxygen, or S(O)m (wherein m is an integer from 0 to 2) but exclude -O-O-, -O-S-, or -S-S- in the ring, with the remaining ring atoms being carbon. Preferably, heterocyclyl contains 3 to 12 ring atoms, wherein 1 to 4 ring atoms are heteroatoms; more preferably, heterocyclyl contains 3 to 8 ring atoms, wherein 1 to 3 ring atoms are heteroatoms; most preferably, heterocyclyl contains 5 to 6 ring atoms, wherein 1 to 2 or 1 to 3 ring atoms are heteroatoms. Non-limiting examples of monocyclic heterocyclyl include pyrrolidinyl, imidazolidinyl, tetrahydrofuryl, tetrahydropyranyl, tetrahydrothienyl, dihydroimidazolyl, dihydrofuryl, dihydropyrazolyl, dihydropyrrolyl, piperidinyl, piperazinyl, morpholinyl, thiomorpholinyl, and homopiperazinyl, preferably tetrahydropyryl, piperidinyl, and pyrrolidinyl. Polycyclic heterocyclyl includes spiroheterocyclyl, fused heterocyclyl, and bridged heterocyclyl.

The term "spiroheterocyclyl" refers to a 5- to 20-membered polycyclic heterocyclyl group with individual rings sharing one common atom (called a spiro atom), wherein one or more ring atoms are heteroatoms selected from nitrogen, oxygen, or S(O)m (wherein m is an integer from 0 to 2), with the remaining ring atoms being carbon. The rings may contain one or more double bonds, but no ring has a completely conjugated π-electron system. Spiroheterocyclyl is preferably 6- to 14-membered and more preferably 7- to 10-membered. According to the number of common spiro atoms shared between rings, spiroheterocyclyl may be classified into monospiroheterocyclyl, dispiroheterocyclyl, or polyspiroheterocyclyl, preferably monospiroheterocyclyl and dispiroheterocyclyl, and more preferably 4-membered/4-membered, 4-membered/5-membered, 4-membered/6-membered, 5-membered/5-membered, or 5-membered/6-membered monospiroheterocyclyl.

The term "fused heterocyclyl" refers to a 5- to 20-membered polycyclic heterocyclyl group in which each ring in the system shares an adjacent pair of atoms with another ring in the system, wherein one or more rings may contain one or more double bonds, but no ring has a completely conjugated π-electron system; and one or more ring atoms are heteroatoms selected from nitrogen, oxygen, or S(O)m (wherein m is an integer from 0 to 2), with the remaining ring atoms being carbon. Fused heterocyclyl is preferably 6- to 14-membered and more preferably 7-to 10-membered. According to the number of rings, fused heterocyclyl may be classified into bicyclic, tricyclic, tetracyclic, or polycyclic fused heterocyclyl, preferably bicyclic or tricyclic fused heterocyclyl, and more preferably 5-membered/5-membered or 5-membered/6-membered bicyclic fused heterocyclyl.

The term "bridged heterocyclyl" refers to a 5- to 14-membered polycyclic heterocyclyl group in which any two rings share two disconnected atoms, wherein the rings may contain one or more double bonds, but no ring has a completely conjugated π-electron system; and one or more ring atoms are heteroatoms selected from nitrogen, oxygen, or S(O)m (wherein m is an integer from 0 to 2), with the remaining ring atoms being carbon. Bridged heterocyclyl is preferably 6- to 14-membered and more preferably 7- to 10-membered. According to the number of rings, bridged heterocyclyl may be classified into bicyclic, tricyclic, tetracyclic, or polycyclic bridged heterocyclyl, preferably bicyclic, tricyclic, or tetracyclic bridged heterocyclyl, and more preferably bicyclic or tricyclic bridged heterocyclyl.

Heterocyclyl includes the preceding heterocyclyl (including monocyclic heterocyclyl, spiroheterocyclyl, fused heterocyclyl, and bridged heterocyclyl) fused to an aryl, heteroaryl, or cycloalkyl ring, wherein a ring joined to a parent structure is a heterocyclyl ring. Non-limiting examples include

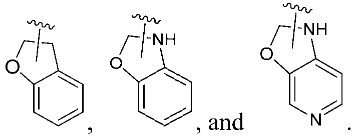

The term "aryl" refers to a 6- to 14-membered full-carbon monocyclic ring or polycyclic fused ring (i.e., rings sharing an adjacent pair of carbon atoms) group having a conjugated π-electron system and is preferably 6- to 10-membered, such as phenyl and naphthyl.

The term "heteroaryl" refers to a heteroaromatic system containing 1 to 4 heteroatoms and 5 to 14 ring atoms, wherein the heteroatoms are selected from oxygen, sulfur, and nitrogen. Heteroaryl is preferably 5- to 10-membered heteroaryl containing 1 to 3 heteroatoms and more preferably 5- or 6-membered heteroaryl containing 1 to 2 heteroatoms. Preferred examples include imidazolyl, furyl, thienyl, thiazolyl, pyrazolyl, oxazolyl, pyrrolyl, tetrazolyl, pyridyl, pyrimidinyl, thiadiazolyl, pyrazinyl, and pyridazinyl.

Heteroaryl includes the preceding heteroaryl fused to an aryl, heterocyclyl, or cycloalkyl ring, wherein a ring joined to a parent structure is a heteroaryl ring. Non-limiting examples include

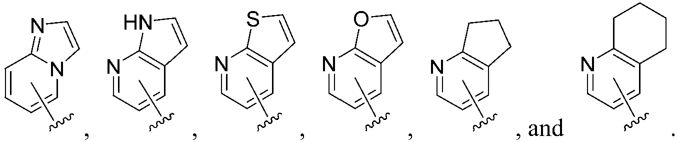

The term "saturated or unsaturated ring" includes the preceding aryl, heteroaryl, cycloalkyl, or heterocycloalkyl.

The term "hydroxyalkyl" refers to alkyl substituted with hydroxyl, wherein alkyl is defined as above.

The term "halogenated alkyl" refers to alkyl substituted with one or more halogens, wherein alkyl is defined as above.

The term "halogenated alkoxy" refers to alkoxy substituted with one or more halogens, wherein alkoxy is defined as above.

The term "deuterated alkyl" refers to alkyl substituted with one or more deuterium atoms, wherein alkyl is defined as above.

The term "deuterated alkoxy" refers to alkoxy substituted with one or more deuterium atoms, wherein alkoxy is defined as above.

The term "cycloalkyl alkyl" refers to alkyl substituted with one or more cycloalkyl groups, wherein cycloalkyl and alkyl are defined as above.

The term "cycloalkyl-oxy" refers to -O-cycloalkyl, wherein cycloalkyl is defined as above.

The term "heterocyclyl alkyl" refers to alkyl substituted with one or more heterocyclyl groups, wherein heterocyclyl and alkyl are defined as above.

The term "arylalkyl" refers to alkyl substituted with one or more aryl groups, wherein aryl and alkyl are defined as above.

The term "hydroxyl" refers to an -OH group.

The term "halogen" refers to fluorine, chlorine, bromine, or iodine.

The term "amino" refers to -NH₂.

The term "cyano" refers to -CN.

The term "nitro" refers to -NO₂.

The term "carboxyl" refers to -C(O)OH.

In the present application, a limited range of the number of carbon atoms in a group refers to any integer number of carbon atoms within the limited range. For example, C₁ to C₆ refers to that the number of carbon atoms in the group may be 1, 2, 3, 4, 5, or 6, C₂ to C₆ refers to that the number of carbon atoms in the group may be 2, 3, 4, 5, or 6, and another limited range of the number of carbon atoms in a group is interpreted similarly.

Compared with the existing art, the present application has the beneficial effects below.

The compound of the present application can be used for preventing and treating parasite infections, has a good prevention and treatment effect, and exhibits good pharmacokinetic properties in mice.

Other aspects can be understood after the detailed description is read and understood.

### DETAILED DESCRIPTION

Technical solutions of the present application are further described below through examples. It is to be understood by those skilled in the art that the examples described below are intended to facilitate understanding of the present application and are not to be construed as limiting the present application.

Some reaction reagents involved in the present application are abbreviated as follows:
DMF: N,N-dimethylformamide;
HATU: 2-(7-azabenzotriazol-1-yl)-*N*,*N*,*N'*,*N'*-tetramethyluronium hexafluorophosphate;
DIPEA: *N,N*-diisopropylethylamine;
DCM: dichloromethane;
T₄P: 1-butylphosphonic acid cyclic anhydride;
TFA: trifluoroacetic acid;
Pd(dppf)Cl₂: [1,1-bis(diphenylphosphino)ferrocene]palladium(II) dichloride;
THF: tetrahydrofuran;
NCS: N-chlorosuccinimide.

### Example 001 Preparation of

### 4-(5-(3,5-dichloro-4-fluorophenyl)-5-(trifluoromethyl)-4,5-dihydroisoxazol-3-yl)-2-methyl-N-(2-oxo-2-((1-(trifluoromethyl)cyclopropyl)amino)ethyl)benzamide (1)

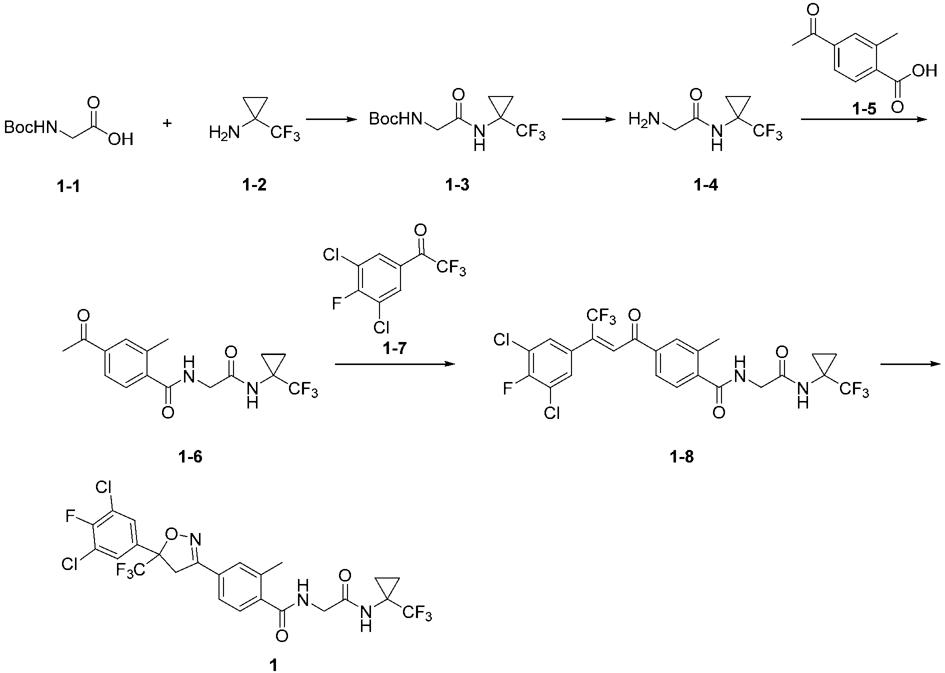

**Step 1:** Compound **1-1** (20.0 g, 114 mmol, 1 eq.) was dissolved in DMF (150 mL), added with HATU (52.1 g, 137 mmol, 1.2 eq.) and DIPEA (44.3 g, 343 mmol, 3 eq.), and stirred at 25°C for 0.5 h. Then, a hydrochloride of Compound 1-2 (20.3 g, 126 mmol, 1.1 eq.) was added and stirred at 25°C for 12 h. The reaction was quenched with water (300 mL) and extracted with ethyl acetate (200 mL × 3). Organic phases were washed with saturated brine (200 mL × 2), dried over anhydrous magnesium sulfate, filtered, and concentrated under reduced pressure to obtain a crude product, Compound **1-3** (30.0 g with a yield of 93%).

**Step 2:** Compound **1-3** (30.0 g, 106 mmol, 1 eq.) was dissolved in DCM (150 mL), added with a dioxane solution of hydrogen chloride (4 M, 150 mL), and stirred at 25°C for 3 h. After the reaction was completed, the system was concentrated under reduced pressure and triturated with ethyl acetate (30 mL) at room temperature to obtain a hydrochloride of Compound **1-4** (18.0 g with a yield of 77%). ¹H NMR (400 MHz, DMSO-*d*₆) δ 9.34 (s, 1H), 8.21 (s, 3H), 3.55 (s, 2H), 1.32-1.24 (m, 2H), 1.10-1.00 (m, 2H).

**Step 3:** Compound **1-5** (5.00 g, 28.1 mmol, 1 eq.) was dissolved in DMF (50 mL), added with HATU (12.8 g, 33.7 mmol, 1.2 eq.) and DIPEA (10.9 g, 84.1 mmol, 3 eq.), and stirred at 25°C for 0.5 h. Then, Compound **1-4** (7.36 g, 33.7 mmol, 1.2 eq.) was added and stirred at 25°C for 0.5 h. The reaction solution was poured into water (50 mL) and extracted with ethyl acetate (10 mL × 3). Organic phases were washed with saturated brine (10 mL × 2), dried over anhydrous magnesium sulfate, filtered, and concentrated under reduced pressure. The crude product was separated and purified through column chromatography (SiO₂, ethyl acetate/n-heptane, 0-60%) to obtain Compound **1-6** (4.20 g with a yield of 78%). MS-ESI: calcd for [M+H]⁺ 343, found 343.

**Step 4:** Compound **1-6** (297 mg, 1.14 mmol, 1.3 eq.) and Compound **1-7** (300 mg, 876 µmol, 1 eq.) were dissolved in acetonitrile (3 mL), added with K₂CO₃ (12.1 mg, 87.6 µmol, 0.1 eq.), and stirred at 95°C for 16 h. The reaction solution was concentrated under reduced pressure, added with water (10 mL), and extracted with ethyl acetate (10 mL × 3). Organic phases were washed with saturated brine (10 mL × 2), dried over anhydrous magnesium sulfate, filtered, and concentrated under reduced pressure. The crude product was separated and purified through column chromatography (SiO₂, ethyl acetate/n-heptane, 0-40%) to obtain Compound **1-8** (140 mg with a yield of 27%). MS-ESI: calcd for [M+H]⁺ 585, found 585.

**Step 5:** Tetrabutylammonium bromide (5.3 mg, 23.9 µmol, 0.1 eq.), NH₂OH.HCl (49.8 mg, 718 µmol, 3 eq.), and NaOH (38.2 mg, 957 µmol, 4 eq.) were dissolved in H₂O (0.5 mL), added to a solution of Compound **1-8** (140 mg, 239 µmol, 1 eq.) in THF (2.0 mL), and stirred at 25°C for 12 h. The reaction was quenched with NH₄Cl (5 mL) and extracted with ethyl acetate (10 mL × 3). Organic phases were washed with saturated brine (10 mL × 2), dried over anhydrous magnesium sulfate, filtered, and concentrated under reduced pressure. The crude product was separated and purified through preparative high-performance liquid chromatography (column: Xtimate C18, 30 × 150 mm I.D., 5 µm; mobile phase: [0.1% formic acid aqueous solution-acetonitrile]; gradient: (66%-86%, 10 min, 100%, 2 min, 66%, 3 min)) to obtain Compound **1** (6.8 mg with a yield of 5%). ¹H NMR (400 MHz, CDCl₃) δ 7.61 (d, *J* = 6.0 Hz, 2H), 7.58-7.46 (m, 3H), 6.77 (s, 1H), 6.64 (t, *J* = 4.8 Hz, 1H), 4.16-4.05 (m, 3H), 3.71 (d, *J =* 17.6 Hz, 1H), 2.50 (s, 3H), 1.43-1.36 (m, 2H), 1.22-1.14 (m, 2H). MS-ESI: calcd for [M+H]⁺ 600, found 600.

### Example 002 Preparation of 4-(5-(3,5-dichlorophenyl)-5-(trifluoromethyl)-4,5-dihydroisoxazol-3-yl)-2-methyl-N-(2-oxo-2-(( 3-(trifluoromethyl)oxetan-3-yl)amino)ethyl)benzamide (2)

### Synthesis route of fragment 2-3

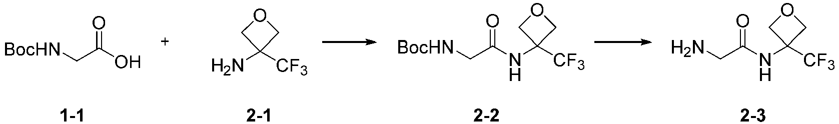

**Step 1:** T₄P (3.04 g, 4.22 mmol, 50% ethyl acetate solution, 1.5 eq.) and DIPEA (1.09 g, 8.45 mmol, 3 eq.) were added to a solution of Compound **1-1** (493 mg, 2.81 mmol, 1 eq.) and Compound **2-1** (500 mg, 2.81 mmol, 1 eq.) in DCM (5 mL) and stirred at 25°C for 2 h. After the reaction was completed, the system was added with water (50 mL) and extracted with ethyl acetate (50 × 3 mL). Organic phases were washed with saturated NaHCO₃ (50 × 2 mL), dried over anhydrous magnesium sulfate, filtered, and concentrated under reduced pressure to obtain a crude product, Compound **2-2** (780 mg with a yield of 92%).

**Step 2:** TFA (298 mg, 2.61 mmol, 2 mL, 1 eq.) was added to a solution of Compound **2-2** (780 mg, 2.61 mmol, 1 eq.) in DCM (10 mL) and stirred at 25°C for 5 h. The reaction mixture was concentrated under reduced pressure to obtain a crude product, a trifluoroacetate of Compound **2-3** (800 mg with a yield of 97%).

### Synthesis route of Compound 2

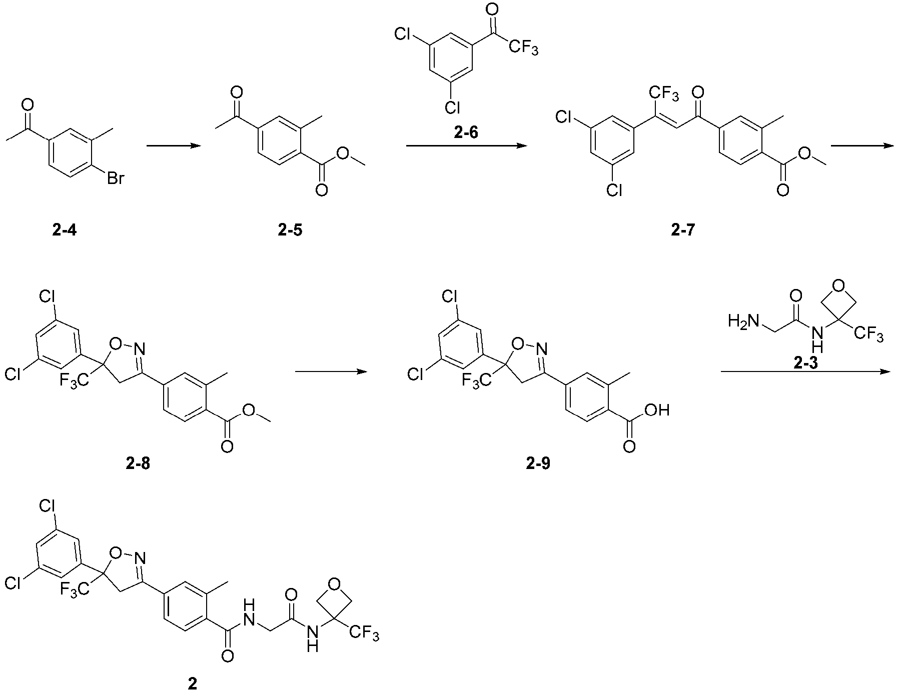

**Step 1:** Pd(dppf)Cl₂ (344 mg, 469 µmol, 0.02 eq.) and triethylamine (7.12 g, 70.3 mmol, 3 eq.) were added to a solution of Compound **2-4** (5.00 g, 23.5 mmol, 1 eq.) in MeOH (30 mL) and DMF (30 mL) and stirred at 80°C for 24 h under a 15 psi CO atmosphere. After the reaction was completed, the system was concentrated under reduced pressure. The crude product was separated and purified through column chromatography (SiO₂, ethyl acetate/n-heptane, 0-10%) to obtain Compound **2-5** (4.2 g with a yield of 93%). ¹H NMR (400 MHz, CDCl₃) δ 7.98 (d, *J =* 8.0 Hz, 1H), 7.85-7.77 (m, 2H), 3.94 (s, 3H), 2.69-2.48 (m, 6H).

**Step 2:** Cs₂CO₃ (678 mg, 2.08 mmol, 0.1 eq.) was added to a solution of Compound **2-5** (4.00 g, 20.8 mmol, 1 eq.) and Compound **2-6** (6.07 g, 24.9 mmol, 1.2 eq.) in toluene (50 mL) and trifluoromethylbenzene (50 mL), stirred at 110°C for 12 h, and quenched with H₂O (10 mL). The system was extracted with ethyl acetate (10 mL × 2). Organic phases were washed with saturated brine (15 mL), dried over anhydrous magnesium sulfate, filtered, and concentrated under reduced pressure. The crude product was separated and purified through column chromatography (SiO₂, ethyl acetate/n-heptane, 0-5%) to obtain Compound **2-7** (4.00 g with a yield of 46%). ¹H NMR (400 MHz, CDCl₃) δ 8.02-7.91 (m, 1H), 7.74-7.63 (m, 2H), 7.43-7.38 (m, 1H), 7.34 (t, *J* = 2.0 Hz, 1H), 7.17 (d, *J=* 1.6 Hz, 2H), 3.94 (s, 3H), 2.64 (s, 3H).

**Step 3:** NaOH (419 mg, 10.5 mmol, 3 eq.) was added to a solution of NH₂OH.HCl (486 mg, 6.99 mmol, 2 eq.) and tetrabutylammonium bromide (112 mg, 349 µmol, 0.1 eq.) in H₂O (4 mL) and stirred at 25°C for 0.5 h. Then, a solution of Compound **2-7** (1.46 g, 3.49 mmol, 1 eq.) in THF (20 mL) was added and stirred at 25°C for 3 h. After the reaction was completed, the reaction was quenched with NH₄Cl (5 mL), added with H₂O (20 mL), and extracted with ethyl acetate (20 mL × 2). Organic phases were washed with saturated brine (25 mL), drying over anhydrous magnesium sulfate, and filtered and concentrated under reduced pressure. The crude product was separated and purified through column chromatography (SiO₂, ethyl acetate/n-heptane, 0-7%) to obtain Compound **2-8** (1.01 g with a yield of 66%). ¹H NMR (400 MHz, CDCl₃) δ 7.98 (d, *J =* 8.8 Hz, 1H), 7.59-7.49 (m, 4H), 7.45 (t, *J =* 1.6 Hz, 1H), 4.14-4.06 (m, 1H), 3.93 (s, 3H), 3.73 (d, *J=* 17.2 Hz, 1H), 2.65 (s, 3H).

**Step 4:** Compound **2-8** (297 mg, 1.14 mmol, 1.3 eq.) was dissolved in THF (3 mL) and H₂O (1 mL), added with LiOH.H₂O (291 mg, 6.94 mmol, 3 eq.), and stirred at 25°C for 6 h. The reaction solution was adjusted to a pH of 5-7 with 1 M HCl and extracted with ethyl acetate (10 mL × 3). Organic phases were washed with saturated brine (10 mL × 2), dried over anhydrous magnesium sulfate, filtered, and concentrated under reduced pressure to obtain a crude product, Compound **2-9** (960 mg with a yield of 99%). ¹H NMR (400 MHz, CDCl₃) δ 8.18-8.08 (m, 1H), 7.64-7.57 (m, 2H), 7.54 (d, *J =* 1.6 Hz, 2H), 7.46 (t, *J =* 1.6 Hz, 1H), 4.16-4.09 (m, 1H), 3.75 (d, *J =* 17.2 Hz, 1H), 2.71 (s, 3H).

**Step 5:** HATU (65.4 mg, 172 µmol, 1.2 eq.) and DIPEA (55.6 mg, 430 µmol, 3 eq.) were added to a solution of Compound **2-9** (60.0 mg, 143 µmol, 1 eq.) in DMF (1 mL) and stirred at 25°C for 10 min. Compound **2-3** (70 mg, 224 µmol, 1.56 eq.) was added to the mixture and stirred at 25°C for 10 min. The system was added with water (10 mL) and extracted with ethyl acetate (10 mL × 3). Organic phases were washed with saturated brine (10 mL × 2), dried over anhydrous magnesium sulfate, filtered, and concentrated under reduced pressure. The obtained crude product was purified through preparative thin-layer chromatography (SiO₂, ethyl acetate) to obtain Compound **2** (13.7 mg with a yield of 16%). ¹H NMR (400 MHz, CDCl₃) δ 7.61-7.51 (m, 5H), 7.50-7.41 (m, 2H), 6.75 (t, *J =* 5.2 Hz, 1H), 4.99-4.63 (m, 4H), 4.24 (d, *J =* 5.6 Hz, 2H), 4.11 (d, *J=* 17.2 Hz, 1H), 3.72 (d, *J=* 17.2 Hz, 1H), 2.48 (s, 3H). MS-ESI: calcd for [M+H]⁺ 598, found 598.

### Example 003 Preparation of N-(2-(3-fluoro-3-(fluoromethyl)azetidin-1-yl)-2-oxoethyl)-2-methyl-4-(5-(3,4,5-trichlorophenyl) -5-(trifluoromethyl)-4,5-dihydroisoxazol-3-yl)benzamide (3)

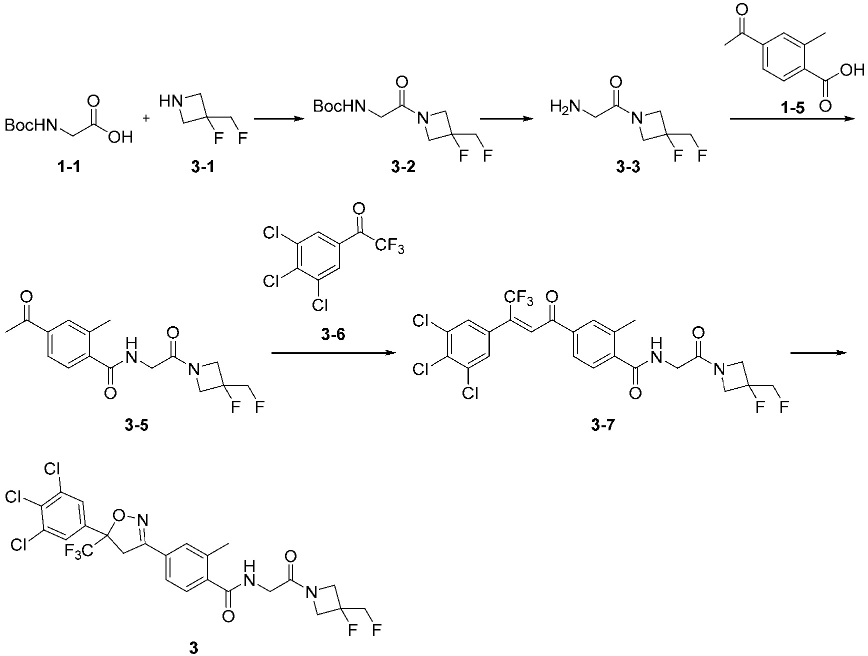

**Step 1:** Compound **1-1** (3.80 g, 21.7 mmol, 1.0 eq.), a hydrochloride of Compound **3-1** (4.67 g, 32.5 mmol, 1.5 eq.), T₄P (23.4 g, 32.5 mmol, 50% ethyl acetate solution, 1.5 eq.), and DIEA (11.2 g, 86.8 mmol, 4 eq.) were dissolved in dichloromethane (30 mL) and reacted at 20°C for 1 h. The reaction was quenched with water (30 mL) and extracted with ethyl acetate (30 mL × 2). Organic phases were combined, washed with saturated brine (20 mL × 2), and concentrated under reduced pressure to obtain a crude product, Compound **3-2** (5.40 g with a yield of 94%).

**Step 2:** Compound **3-2** (6.00 g, 22.7 mmol, 1.0 eq.) was dissolved in dichloromethane (6 mL), and trifluoroacetic acid (12.9 g, 114 mmol, 4.0 eq.) was added dropwise and reacted at 40°C for 16 h. The system was concentrated under reduced pressure to obtain a crude product, a trifluoroacetate of Compound **3-3** (6.31 g with a yield of 100%).

**Step 3:** Compound **1-5** (2.50 g, 14.0 mmol, 1.0 eq.), Compound **3-3** (5.85 g, 21.0 mmol, 1.5 eq.), DIEA (7.25 g, 56.1 mmol, 4.0 eq.), and HATU (8.00 g, 21.0 mmol, 1.5 eq.) were dissolved in DMF (15 mL) and stirred at 20°C for 1 h. The system was added with water (10 mL) and ethyl acetate (10 mL) and separated into layers. The aqueous phase was extracted with ethyl acetate (20 mL × 3). Organic phases were combined, washed with saturated brine (20 mL × 2), dried over anhydrous magnesium sulfate, filtered, and concentrated under reduced pressure. The crude product was separated and purified through column chromatography (SiO₂, ethyl acetate) to obtain Compound **3-5** (3.00 g with a yield of 66%). ¹H NMR (400 MHz, CDCl₃) δ 7.92-7.72 (m, 2H), 7.61-7.39 (m, 1H), 6.77-6.47 (m, 1H), 4.79-4.56 (m, 2H), 4.49-4.08 (m, 6H), 2.63 (s, 3H), 2.53 (s, 3H).

**Step 4:** Compound **3-5** (250 mg, 770 µmol, 1.0 eq.), Compound **3-6** (320 mg, 1.16 mmol, 1.5 eq.), and Cs₂CO₃ (25.0 mg, 77.1 µmol, 0.1 eq.) were added to toluene (3 mL) and trifluoromethylbenzene (3 mL) and stirred at 115°C for 16 h. The reaction solution was concentrated under reduced pressure, added with water (10 mL), and extracted with ethyl acetate (10 mL × 3). Organic phases were washed with saturated brine (10 mL × 2), dried over anhydrous magnesium sulfate, filtered, and concentrated under reduced pressure. The crude product was separated and purified through column chromatography (SiO₂, ethyl acetate/n-heptane, 0-35%) to obtain Compound 3-7 (180 mg with a yield of 40%). ¹H NMR (400 MHz, CDCl₃) δ 7.78-7.64 (m, 2H), 7.56-7.50 (m, 1H), 7.47-7.42 (m, 1H), 7.35-7.30 (m, 2H), 6.64-6.58 (m, 1H), 4.84-4.57 (m, 2H), 4.47-4.03 (m, 6H), 2.58-2.43 (m, 3H).

**Step 5:** Tetrabutylammonium bromide (9.9 mg, 30.8 µmol, 0.1 eq.), NaOH (49.3 mg, 1.23 mmol, 4 eq.), and NH₂OH.HCl (64.2 mg, 925 µmol, 3 eq.) were dissolved in H₂O (0.5 mL), stirred at 25°C for 0.5 h, added to a solution of Compound **3-7** (180 mg, 308 µmol, 1 eq.) in THF (2.0 mL), and stirred at 25°C for 0.5 h. The reaction was quenched with NH₄Cl (5 mL) and extracted with ethyl acetate (10 mL × 3). Organic phases were washed with saturated brine (10 mL × 2), dried over anhydrous magnesium sulfate, filtered, and concentrated under reduced pressure. The crude product was purified through preparative thin-layer chromatography (SiO₂, ethyl acetate) to obtain Compound **3** (24.6 mg with a yield of 13%). ¹H NMR (400 MHz, CDCl₃) δ 7.67 (s, 2H), 7.58-7.47 (m, 3H), 6.62 (t, *J=* 4.4 Hz, 1H), 4.81-4.68 (m, 1H), 4.66-4.57 (m, 1H), 4.41 (d, *J=* 17.6 Hz, 2H), 4.26 (d, *J =* 18.0 Hz, 2H), 4.15-4.05 (m, 3H), 3.71 (d, *J =* 17.2 Hz, 1H), 2.51 (s, 3H). MS-ESI: calcd for [M+H]⁺ 598, found 598.

### Example 004 Preparation of 2-methyl-N-(2-oxo-2-((1-(trifluoromethyl)cyclopropyl)amino)ethyl)-4-(5-(3,4,5-trichlorophenyl) -5-(trifluoromethyl)-4,5-dihydroisoxazol-3-yl)benzamide (4)

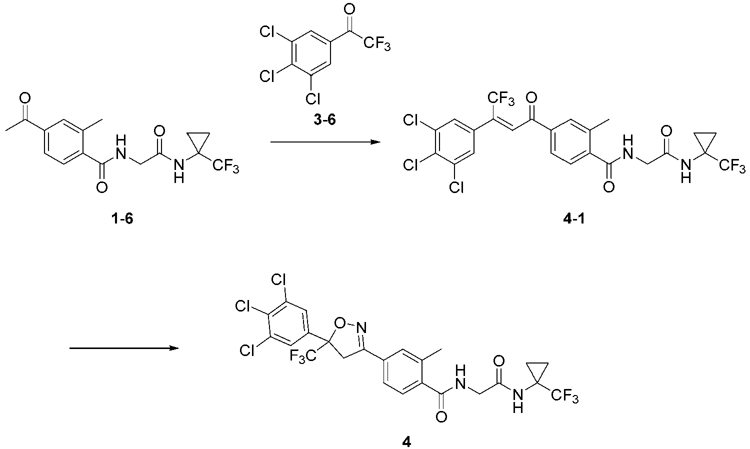

**Step 1:** K₂CO₃ (4.04 mg, 29.2 µmol, 0.1 eq.) was added to a solution of Compound **3-6** (105 mg, 379 µmol, 1.3 eq.) and Compound **1-6** (100 mg, 292 µmol, 1 eq.) in acetonitrile (3 mL) and stirred at 95°C for 16 h. After the reaction was completed, the mixture was concentrated under reduced pressure, diluted with water (10 mL), and extracted with ethyl acetate (10 mL × 3). Organic phases were washed with saturated brine (10 mL × 2), dried over anhydrous magnesium sulfate, filtered, and concentrated under reduced pressure. The crude product was separated and purified through column chromatography (SiO₂, ethyl acetate/n-heptane, 0-50%) to obtain Compound **4-1** (110 mg with a yield of 62%). MS-ESI: calcd for [M+H]⁺ 601, found 601.

**Step 2:** Tetrabutylammonium bromide (5.35 mg, 16.6 µmol, 0.1 eq.), NH₂OH.HCl (34.6 mg, 498 µmol, 3 eq.), and NaOH (26.5 mg, 664 µmol, 4 eq.) were dissolved in H₂O (0.5 mL) and stirred at 25°C for 0.5 h. A solution of Compound **4-1** (100 mg, 166 µmol, 1 eq.) in THF (2 mL) was added to the mixture and stirred at 25°C for 0.5 h. After the reaction was completed, the reaction was quenched with NH₄Cl (5 mL) and extracted with ethyl acetate (10 mL × 2). Combined organic phases were washed with saturated brine (10 mL), dried over anhydrous magnesium sulfate, filtered, and concentrated under reduced pressure. The crude product was separated and purified through preparative thin-layer chromatography (SiO₂, ethyl acetate) to obtain Compound **4** (19.3 mg with a yield of 19%). ¹H NMR (400 MHz, CDCl₃) δ 7.67 (s, 2H), 7.59-7.44 (m, 3H), 6.98 (s, 1H), 6.74 (t, *J =* 5.2 Hz, 1H), 4.23-3.98 (m, 3H), 3.71 (d, *J= 17.2* Hz, 1H), 2.49 (s, 3H), 1.41-1.34 (m, 2H), 1.20-1.10 (m, 2H). MS-ESI: calcd for [M+H]⁺ 616, found 616.

### Example 005 Preparation of 4-(5-(3,5-dichloro-4-fluorophenyl)-5-(trifluoromethyl)-4,5-dihydroisoxazol-3-yl)-N-(2-(3-fluoro-3-(fluoromethyl)azetidin-1-yl)-2-oxoethyl)-2-methylbenzamide (5)

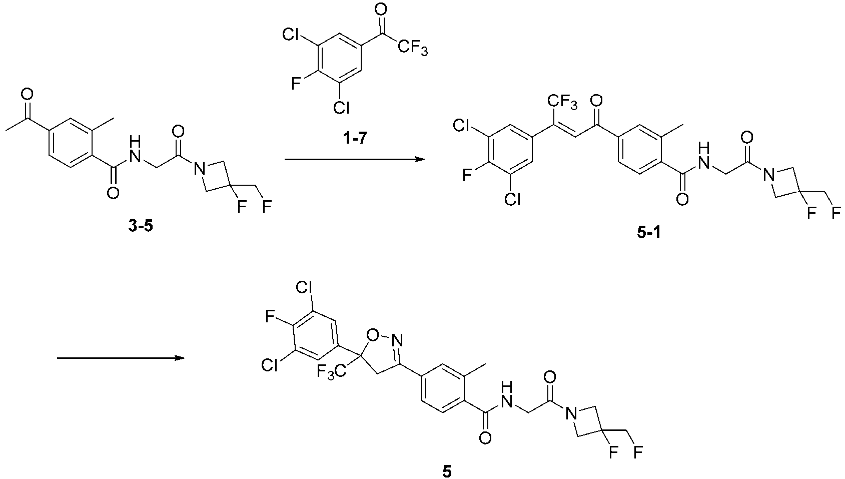

**Step 1:** Compound **3-5** (250 mg, 770 µmol, 1.0 eq.), Compound **1-7** (301 mg, 1.16 mmol, 1.5 eq.), and Cs₂CO₃ (25 mg, 77.1 µmol, 0.1 eq.) were added to toluene (3 mL) and trifluoromethylbenzene (3 mL) and stirred at 115°C for 16 h. The reaction solution was concentrated under reduced pressure, added with water (10 mL), and extracted with ethyl acetate (10 mL × 3). Organic phases were washed with saturated brine (10 mL × 2), dried over anhydrous magnesium sulfate, filtered, and concentrated under reduced pressure. The crude product was separated and purified through column chromatography (SiO₂, ethyl acetate/n-heptane, 0-50%) to obtain Compound **5-1** (200 mg with a yield of 46%). ¹H NMR (400 MHz, CDCl₃) δ 7.74-7.64 (m, 2H), 7.56-7.50 (m, 1H), 7.45-7.39 (m, 1H), 7.28-7.23 (m, 2H), 6.70-6.51 (m, 1H), 4.82-4.53 (m, 2H), 4.50-4.01 (m, 6H), 2.52 (s, 3H).

**Step 2:** NH₂OH.HCl (47.7 mg, 687 µmol, 3 eq.), NaOH (36.6 mg, 916 µmol, 4 eq.), and tetrabutylammonium bromide (7.4 mg, 22.9 µmol, 0.1 eq.) were dissolved in H₂O (0.5 mL), added to a solution of Compound **3-7** (130 mg, 229 µmol, 1 eq.) in THF (2.0 mL), and stirred at 25°C for 12 h. The reaction was quenched with NH₄Cl (5 mL) and extracted with ethyl acetate (10 mL × 3). Combined organic phases were washed with saturated brine (10 mL × 2), dried over anhydrous magnesium sulfate, filtered, and concentrated under reduced pressure. The crude product was separated and purified through preparative high-performance liquid chromatography (column: Xtimate C18, 30 × 150 mm I.D., 5 µm; mobile phase: [0.1% formic acid aqueous solution-acetonitrile]; gradient: (62%-82%, 10 min, 100%, 2 min, 62%, 3 min)) to obtain Compound 5 (13.7 mg with a yield of 10%). ¹H NMR (400 MHz, CDCl₃) δ 7.61 (d, *J =* 6.0 Hz, 2H), 7.56 (s, 1H), 7.52 (d, *J=* 2.4 Hz, 2H), 6.66 (t, *J=* 4.4 Hz, 1H), 4.81-4.68 (m, 1H), 4.67-4.58 (m, 1H), 4.42 (d, *J =* 17.6 Hz, 2H), 4.26 (d, *J* = 18.0 Hz, 2H), 4.14-4.02 (m, 3H), 3.71 (d, *J =* 17.2 Hz, 1H), 2.51 (s, 3H). MS-ESI: calcd for [M+H]⁺ 582, found 582.

### Example 006 Preparation of 4-(5-(3,5-dichlorophenyl)-5-(trifluoromethyl)-4,5-dihydroisoxazol-3-yl)-2-methyl-N-(2-oxo-2-(2 -(trifluoromethyl)azetidin-1-yl)ethyl)benzamide (6)

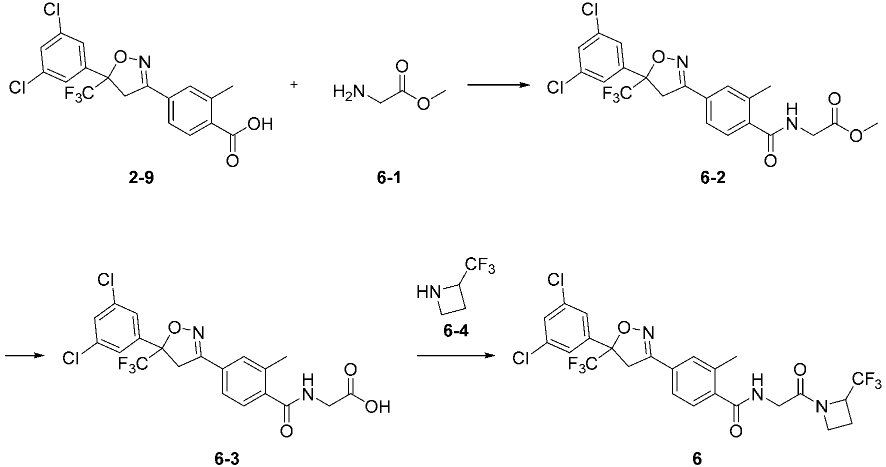

**Step 1:** HATU (436 mg, 1.14 mmol, 1.2 eq.) and DIPEA (370 mg, 2.86 mmol, 499 µL, 3 eq.) were added to a solution of Compound **2-9** (400 mg, 956 µmol, 1 eq.) and Compound **6-1** (144 mg, 1.14 mmol, 1.2 eq.) in DMF (10 mL) and stirred at 20°C for 2 h. After the reaction was completed, the system was added with H₂O (10 mL) and extracted with ethyl acetate (10 mL × 2). Combined organic phases were washed with saturated brine (10 mL × 2), dried over anhydrous magnesium sulfate, filtered, and concentrated under reduced pressure to obtain a crude product. The crude product was purified through column chromatography (SiO₂, ethyl acetate/n-heptane, 0-25%) to obtain Compound **6-2** (450 mg with a yield of 96%). MS-ESI: calcd for [M+H]⁺ 489, found 489.

**Step 2:** LiOH.H₂O (115 mg, 2.75 mmol, 3 eq.) was added to a solution of Compound **6-2** (450 mg, 919 µmol, 1 eq.) in THF (4 mL) and H₂O (1 mL) and stirred at 20°C for 5 h. THF was spin-removed, and the system was acidified to a pH of about 4 with 1 N HCl and extracted with ethyl acetate (15 mL × 2). Combined organic phases were washed with saturated brine (15 mL), dried over magnesium sulfate, filtered, and concentrated under reduced pressure to obtain Compound **6-3** (400 mg with a yield of 91%). MS-ESI: calcd for [M+H]⁺ 475, found 475.

**Step 3:** HATU (48.0 mg, 126 µmol, 1.2 eq.) and DIPEA (40.7 mg, 315 µmol, 3 eq.) were added to a solution of Compound **6-3** (50.0 mg, 105 µmol, 1 eq.) in DMF (2 mL) and stirred at 25°C for 10 min. A hydrochloride of Compound **6-4** (20.3 mg, 126 µmol, 1.2 eq.) was added to the mixture and stirred at 25°C for 10 min. After the reaction was completed, the system was diluted with water (10 mL) and extracted with ethyl acetate (10 mL × 3). Combined organic phases were washed with saturated brine (10 mL × 2), dried over anhydrous magnesium sulfate, filtered, and concentrated under reduced pressure. The crude product was purified through preparative thin-layer chromatography (SiO₂, ethyl acetate) to obtain Compound 6 (5.6 mg with a yield of 9%). ¹H NMR (400 MHz, CDCl₃) δ 7.56 (s, 1H), 7.53 (d, *J =* 5.2 Hz, 4H), 7.45 (t, *J =* 1.6 Hz, 1H), 6.67-6.55 (m, 1H), 5.01-4.70 (m, 1H), 4.44-4.15 (m, 3H), 4.11 (d, *J =* 17.6 Hz, 1H), 4.05-3.85 (m, 1H), 3.72 (d, *J =* 17.2 Hz, 1H), 2.82-2.62 (m, 1H), 2.52 (s, 3H), 2.50-2.43 (m, 1H). MS-ESI: calcd for [M+H]⁺ 582, found 582.

### Example 007 Preparation of 4-(5-(3-chloro-5-(trifluoromethyl)phenyl)-5-(trifluoromethyl)-4,5-dihydroisoxazol-3-yl)-2-methy 1-N-(2-oxo-2-((1-(trifluoromethyl)cyclopropyl)amino)ethyl)benzamide (7)

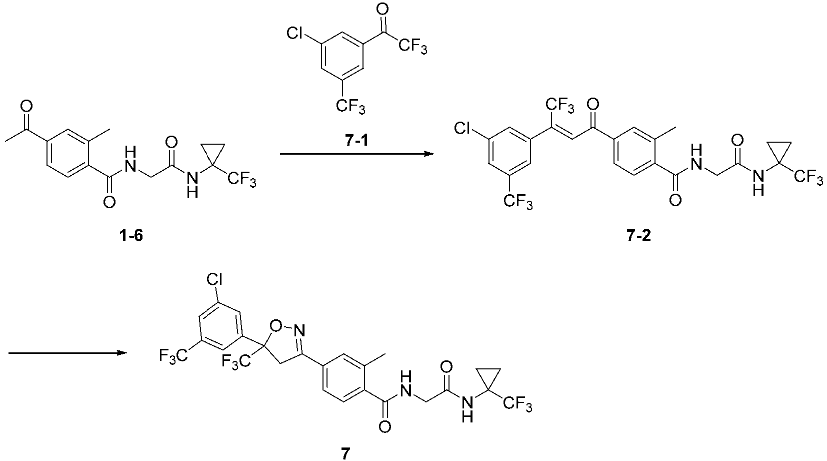

**Step 1:** Cs₂CO₃ (57.1 mg, 175 µmol, 0.2 eq.) was added to a solution of Compound **7-1** (315 mg, 1.13 mmol, 1.3 eq.) and Compound **1-6** (300 mg, 876 µmol, 1 eq.) in toluene (5 mL) and trifluoromethylbenzene (5 mL) and stirred at 110°C for 12 h. The mixture was concentrated under reduced pressure, diluted with water (10 mL), and extracted with ethyl acetate (10 mL × 2). Organic phases were concentrated under reduced pressure, dissolved in toluene (5 mL), added with sulfoxide chloride (312 mg, 2.62 mmol, 3 eq.), and stirred at 70°C for 4 h. The reaction was concentrated under reduced pressure, quenched with water (10 mL), and extracted with ethyl acetate (10 mL × 2). Combined organic phases were washed with saturated brine (10 mL), dried over anhydrous magnesium sulfate, filtered, and concentrated under reduced pressure to obtain a crude product. The crude product was separated and purified through column chromatography (SiO₂, ethyl acetate/n-heptane, 0-40%) to obtain Compound **7-2** (170 mg with a yield of 32%). MS-ESI: calcd for [M+H]⁺ 601, found 601.

**Step 2:** Tetrabutylammonium bromide (5.3 mg, 28.2 µmol, 0.1 eq.), NH₂OH.HCl (58.9 mg, 848 µmol, 3 eq.), and NaOH (45.2 mg, 1.13 mmol, 4 eq.) were dissolved in H₂O (0.5 mL), stirred at 25°C for 0.5 h, added with a solution of Compound **7-2** (170 mg, 282 µmol, 1 eq.) in THF (2.0 mL), and stirred at 25°C for 0.5 h. The reaction was quenched with NH₄Cl (5 mL) and extracted with ethyl acetate (10 mL × 3). Organic phases were washed with saturated brine (10 mL × 2), dried over anhydrous magnesium sulfate, filtered, concentrated under reduced pressure, and purified through preparative thin-layer chromatography (SiO₂, ethyl acetate) to obtain Compound 7 (19.0 mg with a yield of 11%). ¹H NMR (400 MHz, CDCl₃) δ 7.85 (s, 1H), 7.78 (s, 1H), 7.72 (s, 1H), 7.61-7.47 (m, 3H), 7.00 (s, 1H), 6.76 (t, *J =* 5.2 Hz, 1H), 4.22-4.05 (m, 3H), 3.75 (d, *J* = 17.6 Hz, 1H), 2.50 (s, 3H), 1.42-1.30 (m, 2H), 1.22-1.06 (m, 2H). MS-ESI: calcd for [M+H]⁺ 616, found 616.

### Example 008 and Example 009 Preparation of (S)-4-(5-(3-chloro-5-(trifluoromethyl)phenyl)-5-(trifluoromethyl)-4,5-dihydroisoxazol-3-yl)-2-m ethyl-N-(2-oxo-2-((1-(trifluoromethyl)cyclopropyl)amino)ethyl)benzamide (8) and (R)-4-(5-(3-chloro-5-(trifluoromethyl)phenyl)-5-(trifluoromethyl)-4,5-dihydroisoxazol-3-yl)-2-m ethyl-N-(2-oxo-2-((1-(trifluoromethyl)cyclopropyl)amino)ethyl)benzamide (9)

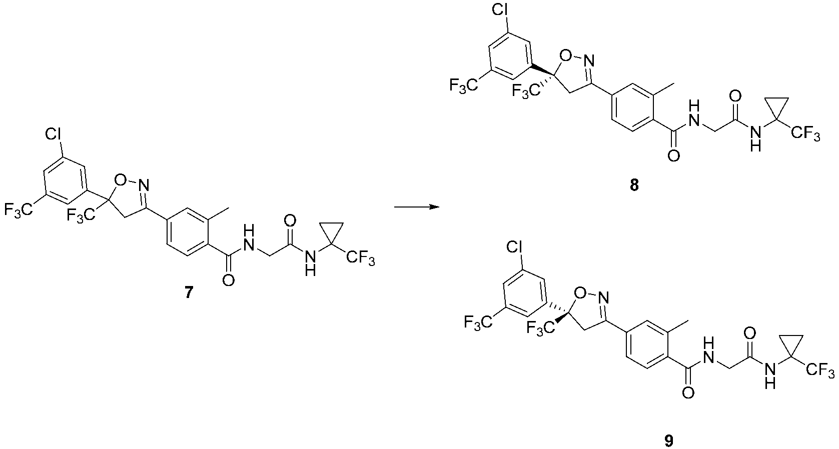

**Step 1:** Compound **7** (500 mg) was separated and purified through supercritical fluid chromatography (column: ChiralCel OJ, 250 × 50 mm I.D., 10 µm; mobile phase: [A is CO₂ and B is methanol (0.1% NH₃H₂O)]; gradient: (B 20%)) to obtain Compound **8** (41.6 mg with a yield of 8%). ¹H NMR (400 MHz, CDCl₃) δ 7.85 (s, 1H), 7.78 (s, 1H), 7.72 (s, 1H), 7.60-7.46 (m, 3H), 6.92 (s, 1H), 6.71 (t, *J =* 5.2 Hz, 1H), 4.23-4.04 (m, 3H), 3.75 (d, *J =* 17.2 Hz, 1H), 2.50 (s, 3H), 1.42-1.34 (m, 2H), 1.24-1.06 (m, 2H). MS-ESI: calcd for [M+H]⁺ 616, found 616. Compound **9** (37.0 mg with a yield of 7%). ¹H NMR (400 MHz, CDCl₃) δ 7.85 (s, 1H), 7.78 (s, 1H), 7.72 (s, 1H), 7.60-7.46 (m, 3H), 6.92 (s, 1H), 6.71 (t, *J =* 5.2 Hz, 1H), 4.23-4.04 (m, 3H), 3.75 (d, *J =* 17.2 Hz, 1H), 2.51 (s, 3H), 1.43-1.33 (m, 2H), 1.24-1.06 (m, 2H). MS-ESI: calcd for [M+H]⁺ 616, found 616.

### Example 010 Preparation of 4-(5-(3,5-dichlorophenyl)-5-(trifluoromethyl)-4,5-dihydroisoxazol-3-yl)-2-methyl-N-(2-oxo-2-(( 1-(trifluoromethyl)cyclopropyl)amino)ethyl)benzamide (10)

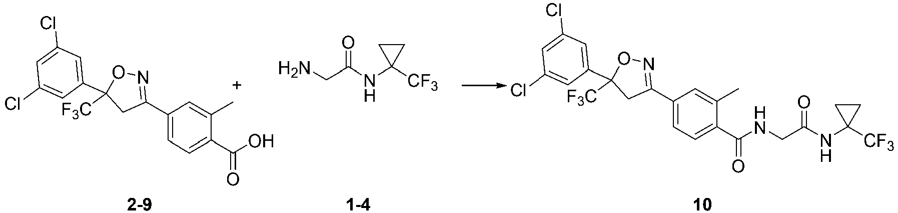

**Step 1:** HATU (54.5 mg, 143 µmol, 1.2 eq.) and DIPEA (46.3 mg, 358 µmol, 3 eq.) were added to a solution of Compound **2-9** (50.0 mg, 119 µmol, 1 eq.) in DMF (1 mL) and stirred at 25°C for 10 min. A hydrochloride of Compound **1-4** (26.1 mg, 143 µmol, 1.2 eq.) was added and stirred at 25°C for 10 min. The reaction was quenched with water (10 mL) and extracted with ethyl acetate (10 mL × 3). Combined organic phases were washed with saturated brine (10 mL × 2), dried over anhydrous magnesium sulfate, filtered, concentrated under reduced pressure, and purified through preparative thin-layer chromatography (SiO₂, ethyl acetate) to obtain Compound **10** (27.3 mg with a yield of 39%). ¹H NMR (400 MHz, CDCl₃) δ 7.63-7.47 (m, 5H), 7.46 (t, *J=* 1.6 Hz, 1H), 6.81 (s, 1H), 6.65 (t, *J* = 5.2 Hz, 1H), 4.18-4.03 (m, 3H), 3.72 (d, *J* = 17.6 Hz, 1H), 2.50 (s, 3H), 1.43-1.33 (m, 2H), 1.22-1.13 (m, 2H). MS-ESI: calcd for [M+H]⁺ 582, found 582.

### Example 011 and Example 012 Preparation of (S)-4-(5-(3,5-dichlorophenyl)-5-(trifluoromethyl)-4,5-dihydroisoxazol-3-yl)-2-methyl-N-(2-oxo-2-((1-(trifluoromethyl)cyclopropyl)amino)ethyl)benzamide (11) and (R)-4-(5-(3,5-dichlorophenyl)-5-(trifluoromethyl)-4,5-dihydroisoxazol-3-yl)-2-methyl-N-(2-oxo-2-((1-(trifluoromethyl)cyclopropyl)amino)ethyl)benzamide (12)

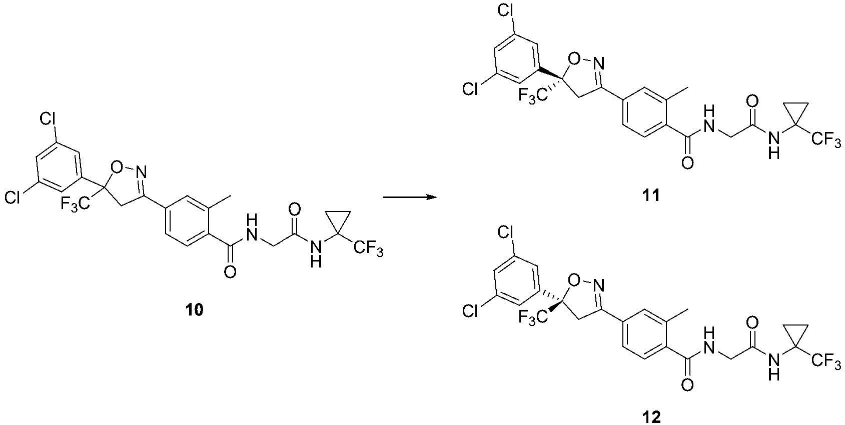

**Step 1:** Compound **10** (300 mg) was separated and purified through supercritical fluid chromatography (column: ChiralCel OJ, 250 × 50 mm I.D., 10 µm; mobile phase: [A for CO₂ and B for methanol]; gradient: (B 20%)) to obtain Compound **11** (14.9 mg with a yield of 5%). ¹H NMR (400 MHz, CDCl₃) δ 7.59-7.42 (m, 6H), 6.85 (s, 1H), 6.85 (t,, *J* = 5.2 Hz, 1H), 4.19-3.95 (m, 3H), 3.72 (d, *J =* 17.6 Hz, 1H), 2.50 (s, 3H), 1.41-1.32 (m, 2H), 1.22-1.12 (m, 2H). MS-ESI: calcd for [M+H]⁺ 582, found 582.

Compound **12** (31.6 mg with a yield of 11%). ¹H NMR (400 MHz, CDCl₃) δ 7.59-7.42 (m, 6H), 6.85 (s, 1H), 6.67 (t, *J =* 5.2 Hz, 1H), 4.19-3.95 (m, 3H), 3.72 (d, *J =* 17.6 Hz, 1H), 2.50 (s, 3H), 1.41-1.32 (m, 2H), 1.22-1.12 (m, 2H). MS-ESI: calcd for [M+H]⁺ 582, found 582.

### Example 013 Preparation of 4-(5-(3-chloro-5-(trifluoromethyl)phenyl)-5-(trifluoromethyl)-4,5-dihydroisoxazol-3-yl)-N-(2-(3-fluoro-3-(fluoromethyl)azetidin-1-yl)-2-oxoethyl)-2-methylbenzamide (13)

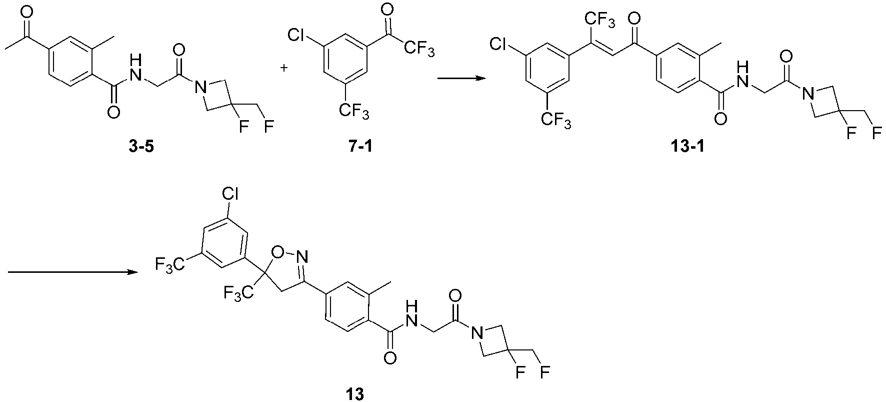

**Step 1:** Compound **3-5** (250 mg, 770 µmol, 1.0 eq.), Compound **7-1** (320 mg, 1.16 mmol, 1.5 eq.), and Cs₂CO₃ (25 mg, 77.1 µmol, 0.1 eq.) were added to toluene (3 mL) and trifluoromethylbenzene (3 mL) and stirred at 115°C for 16 h. The reaction solution was concentrated under reduced pressure, added with water (5 mL), and extracted with ethyl acetate (5 mL × 3). Combined organic phases were washed with water (5 mL) and saturated brine (5 mL), dried over anhydrous magnesium sulfate, filtered, and concentrated under reduced pressure. The crude product was separated and purified through column chromatography (SiO₂, ethyl acetate/n-heptane, 0-50%) to obtain Compound **13-1** (150 mg with a yield of 33%). ¹H NMR (400 MHz, CDCl₃) δ 7.72-7.65 (m, 2H), 7.64-7.60 (m, 1H), 7.53-7.41 (m, 4H), 6.72-6.43 (m, 1H), 4.91-4.50 (m, 2H), 4.47-3.94 (m, 6H), 2.50 (s, 3H).

**Step 2:** NH₂OH.HCl (53.6 mg, 772 µmol, 3 eq.), NaOH (41.1 mg, 1.02 mmol, 4 eq.), and tetrabutylammonium bromide (8.3 mg, 25.7 µmol, 0.1 eq.) were dissolved in H₂O (0.5 mL), stirred at 25°C for 0.5 h, added to a solution of Compound **13-1** (150 mg, 257 µmol, 1 eq.) in THF (2.0 mL), and stirred at 25°C for 0.5 h. The reaction was quenched with NH₄Cl (5 mL) and extracted with ethyl acetate (10 mL × 3). Organic phases were washed with saturated brine (10 mL × 2), dried over anhydrous magnesium sulfate, filtered, and concentrated under reduced pressure. The crude product was purified through preparative thin-layer chromatography (SiO₂, ethyl acetate) to obtain Compound **13** (26.3 mg) with a yield of 17%. ¹H NMR (400 MHz, CDCl₃) δ = 7.91-7.66 (m, 3H), 7.64-7.46 (m, 3H), 6.62 (t, *J=* 4.4 Hz, 1H), 4.80-4.57 (m, 2H), 4.41 (d, *J=* 17.6 Hz, 2H), 4.26 (d, *J =* 18.0 Hz, 2H), 4.16 (d, *J =* 17.6 Hz, 1H), 4.10 (t*, J =* 3.6 Hz, 2H), 3.75 (d, *J* = 17.6 Hz, 1H), 2.51 (s, 3H). MS-ESI: calcd for [M+H]⁺ 598, found 598.

### Example 014 Preparation of 4-(5-(3,5-dichlorophenyl)-5-(trifluoromethyl)-4,5-dihydroisoxazol-3-yl)-N-(2-(3-hydroxy-3-(trifl uoromethyl)azetidin-1-yl)-2-oxoethyl)-2-methylbenzamide (14)

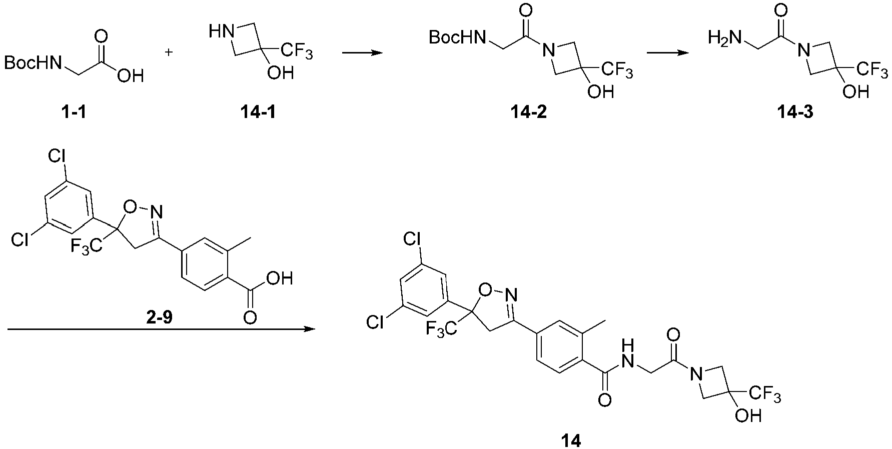

**Step 1:** DIPEA (1.09 g, 8.44 mmol, 3 eq.) and T₄P (3.04 g, 4.22 mmol, 50% ethyl acetate solution, 1.5 eq.) were added to a solution of a hydrochloride of Compound **14-1** (500 mg, 2.81 mmol, 1 eq.) and Compound **1-1** (493 mg, 2.81 mmol, 1 eq.) in DCM (3 mL) and stirred at 25°C for 2 h. The system was diluted with water (20 mL) and extracted with ethyl acetate (20 mL × 3). Combined organic phases were washed with NaHCO₃ (20 mL × 2), dried over anhydrous magnesium sulfate, filtered, and concentrated under reduced pressure to obtain a crude product, Compound **14-2** (600 mg with a yield of 71%). ¹H NMR (400 MHz, CDCl₃) δ 5.24 (s, 1H), 4.43 (d, *J* = 10.0 Hz, 1H), 4.34 (d, *J=* 11.2 Hz, 1H), 4.22 (d, *J =* 10.4 Hz, 1H), 4.08 (d, *J* = 11.6 Hz, 1H), 3.95-3.66 (m, 3H), 1.47 (s, 9H).

**Step 2:** TFA (1 mL) was added to a solution of Compound **14-2** (605 mg, 2.02 mmol, 1 eq.) in DCM (5 mL) and stirred at 25°C for 3 h. After the reaction was completed, the system was concentrated under reduced pressure to obtain a crude product, a trifluoroacetate of Compound **14-3** (600 mg with a yield of 94%).

**Step 3:** HATU (65.4 mg, 172 µmol, 1.2 eq.) and DIPEA (55.6 mg, 430 µmol, 3 eq.) were added to a solution of Compound **2-9** (60.0 mg, 143 µmol, 1 eq.) in DMF (1 mL) and stirred at 25°C for 0.5 h. The trifluoroacetate of Compound **14-3** (60.0 mg, 192 µmol, 1.34 eq.) was added and stirred at 25°C for 0.5 h. The system was diluted with water (20 mL) and extracted with ethyl acetate (20 mL × 3). Organic phases were washed with saturated brine (20 mL × 2), dried over anhydrous magnesium sulfate, filtered, concentrated under reduced pressure, and separated and purified through preparative thin-layer chromatography (SiO₂, ethyl acetate) to obtain Compound 14 (13.4 mg with a yield of 20%). ¹H NMR (400 MHz, CDCl₃) δ 7.59-7.48 (m, 5H), 7.45 (t, *J=* 1.6 Hz, 1H), 6.67 (t, *J=* 4.8 Hz, 1H), 4.48 (d, *J=* 10.0 Hz, 1H), 4.35 (d, *J =* 11.2 Hz, 1H), 4.31-4.04 (m, 5H), 4.03-3.91 (m, 1H), 3.73 (d, *J* = 17.6 Hz, 1H), 2.49 (s, 3H). MS-ESI: calcd for [M+H]⁺ 598, found 598.

### Example 015 Preparation of 4-(5-(3,5-dichlorophenyl)-5-(trifluoromethyl)-4,5-dihydroisoxazol-3-yl)-2-methyl-N-(3-((2,2,2-tr ifluoroethyl)amino)oxetan-3-yl)methyl)benzamide (15)

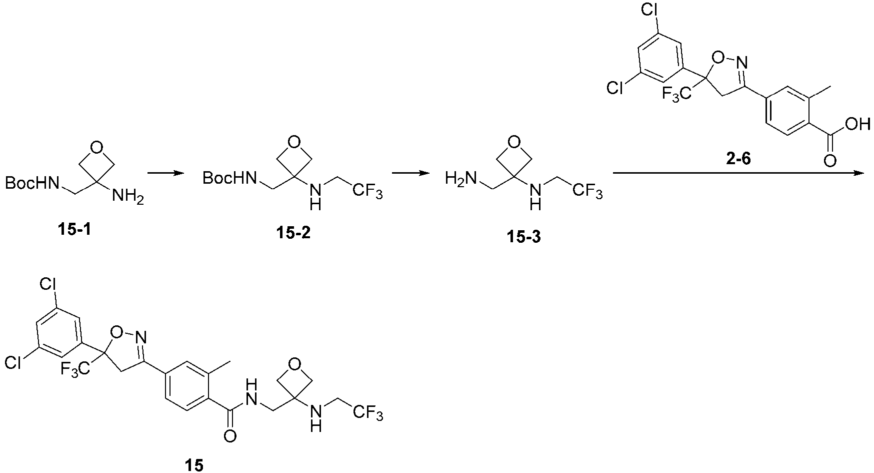

**Step 1:** Trifluoroethyl trifluoromethanesulfonate (860 mg, 3.70 mmol, 1.5 eq.) and triethylamine (500 mg, 4.94 mmol, 687 µL, 2 eq.) were added to a solution of Compound **15-1** (500 mg, 2.47 mmol, 1 eq.) in THF (40 mL) and reacted at 70°C for 12 h. After the reaction was completed, the system was concentrated under reduced pressure to obtain a crude product, Compound **15-2** (700 mg with a yield of 99%).

**Step 2:** TFA (3 mL) was added to a solution of Compound **15-2** (178 mg, 626 µmol, 1 eq.) in DCM (10 mL) and stirred at 25°C for 12 h. The reaction solution was concentrated under reduced pressure to obtain a crude product, a trifluoroacetate of Compound **15-3** (150 mg with a yield of 80%). ¹H NMR(400 MHz, DMSO-*d*₆) δ 7.79 (s, 3H), 4.4-4.5 (m, 2H), 4.36 (d, *J =* 7.2 Hz, 2H), 3.3-3.4 (m, 2H), 3.25 (d, *J =* 4.8 Hz, 2H).

**Step 3:** HATU (47.7 mg, 125 µmol, 1.5 eq.) and DIPEA (32.4 mg, 251 µmol, 43.7 µL, 3 eq.) were added to a solution of Compound **2-9** (35.0 mg, 83.6 µmol, 1 eq.) and Compound **15-3** (49.9 mg, 167 µmol, 2 eq.) in DMF (2 mL) and stirred at 25°C for 12 h. The reaction was quenched with H₂O (3 mL) and extracted with ethyl acetate (5 mL × 2). Combined organic phases were washed with saturated brine (10 mL), dried over anhydrous magnesium sulfate, filtered, and concentrated under reduced pressure. The obtained crude product was purified through preparative thin-layer chromatography (SiO₂, ethyl acetate) to obtain Compound **15** (27.0 mg with a yield of 55%). ¹H NMR (400 MHz, CDCl₃) δ 7.54 (d, *J =* 6.0 Hz, 4H), 7.45 (t, *J* = 2.0 Hz, 1H), 7.42 (d, *J =* 8.0 Hz, 1H), 6.15 (t, *J =* 5.2 Hz, 1H), 4.60-4.47 (m, 4H), 4.10 (d, *J =* 17.2 Hz, 1H), 3.89 (d, *J=* 5.6 Hz, 2H), 3.72 (d, *J=* 17.6 Hz, 1H), 3.43-3.24 (m, 2H), 2.48 (s, 3H), 2.09-1.93 (m, 1H). MS-ESI: calcd for [M+H]⁺ 584, found 584.

### Example 016 Preparation of 4-(5-(3,5-dichlorophenyl)-5-(trifluoromethyl)-4,5-dihydroisoxazol-3-yl)-2-methyl-N-(2-oxo-2-(2 -(trifluoromethyl)pyrrolidin-1-yl)ethyl)benzamide (16)

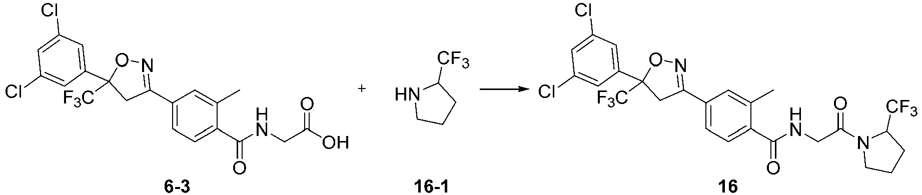

**Step 1:** HATU (48.0 mg, 126 µmol, 1.2 eq.) and DIPEA (40.7 mg, 315 µmol, 3 eq.) were added to a solution of Compound **6-3** (50.0 mg, 105 µmol, 1 eq.) in DMF (2 mL) and stirred at 25°C for 10 min. A hydrochloride of Compound **16-1** (21.9 mg, 125 µmol, 1.18 eq.) was added to the mixture and stirred at 25°C for 10 min. The reaction was quenched with water (10 mL) and extracted with ethyl acetate (10 mL × 3). Combined organic phases were washed with saturated brine (10 mL × 2), dried over anhydrous magnesium sulfate, filtered, and concentrated under reduced pressure. The crude product was purified through preparative thin-layer chromatography (SiO₂, ethyl acetate) to obtain Compound **16** (34.5 mg with a yield of 54%). ¹H NMR (400 MHz, CDCl₃) δ 7.57-7.50 (m, 5H), 7.45 (t, *J =* 1.6 Hz, 1H), 6.87-6.72 (m, 1H), 4.90-4.74 (m, 1H), 4.50-4.33 (m, 1H), 4.25-4.15 (m, 1H), 4.11 (d, *J =* 17.6 Hz, 1H), 3.72 (d, *J =* 17.2 Hz, 1H), 3.69-3.52 (m, 2H), 2.53 (s, 3H), 2.29-2.02 (m, 4H). MS-ESI: calcd for [M+H]⁺ 596, found 596.

### Example 017 Preparation of 4-(5-(3,5-dichlorophenyl)-5-(trifluoromethyl)-4,5-dihydroisoxazol-3-yl)-2-methyl-N-(1-((2,2,2-tr ifluoroethyl)amino)cyclopropyl)methyl)benzamide (17)

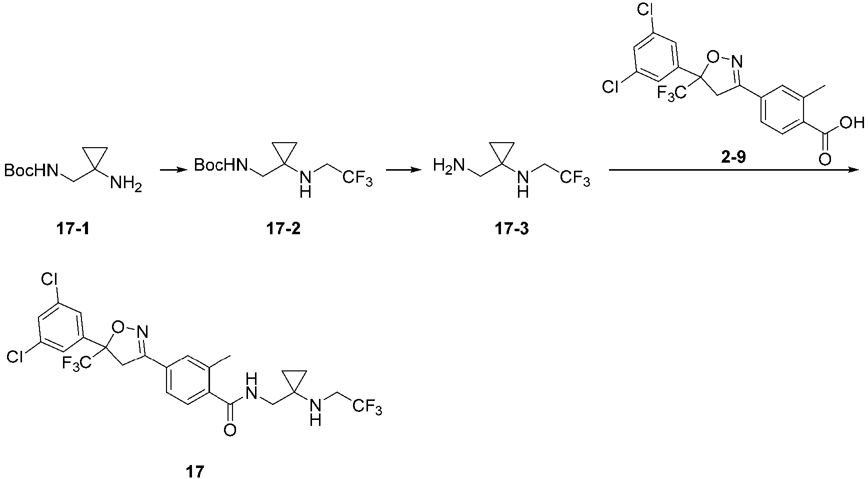

**Step 1:** Trifluoroethyl trifluoromethanesulfonate (934 mg, 4.02 mmol, 1.5 eq.) and triethylamine (543 mg, 5.37 mmol, 746 µL, 2 eq.) were added to a solution of Compound **17-1** (500 mg, 2.68 mmol, 1 eq.) in THF (10 mL) and stirred at 70°C for 12 h. The reaction solution was concentrated under reduced pressure to obtain a crude product, Compound **17-2** (700 mg with a yield of 97%).

**Step 2:** A dioxane solution of hydrogen chloride (4 M, 5 mL) was added to a solution of Compound **17-2** (250 mg, 931 µmol, 1 eq.) in dioxane (5 mL) and stirred at 25°C for 16 h. The reaction solution was concentrated under reduced pressure. The obtained crude product was triturated with ethyl acetate (15 mL) to obtain a hydrochloride of Compound **17-3** (176 mg with a yield of 92%). ¹H NMR (400 MHz, DMSO-*d*₆) δ 8.13 (s, 3H), 3.50-3.31 (m, 2H), 2.90-2.81 (m, 2H), 0.75-0.65 (m, 4H).

**Step 3:** HATU (47.7 mg, 125 µmol, 1.5 eq.) and DIPEA (32.4 mg, 251 µmol, 43.7 µL, 3 eq.) were added to a solution of Compound **2-9** (35 mg, 83.6 µmol, 1 eq.) and Compound **17-3** (34.2 mg, 167 µmol, 2 eq.) in DMF (2 mL) and stirred at 25°C for 1 h. The reaction was quenched with H₂O (3 mL) and extracted with ethyl acetate (5 mL × 2). Combined organic phases were washed with saturated brine (10 mL), dried over anhydrous magnesium sulfate, filtered, and concentrated under reduced pressure. The crude product was purified through preparative thin-layer chromatography (SiO₂, n-heptane/ethyl acetate, 1:1) to obtain Compound **17** (25.0 mg with a yield of 52%). ¹H NMR (400 MHz, CDCl₃) δ 7.58-7.51 (m, 4H), 7.51-7.41 (m, 2H), 6.14 (s, 1H), 4.11 (d, *J =* 17.6 Hz, 1H), 3.72 (d, *J =* 17.2 Hz, 1H), 3.45 (d, *J =* 5.6 Hz, 2H), 3.41-3.28 (m, 2H), 2.51 (s, 3H), 1.63 (s, 1H), 0.80-0.74 (m, 2H), 0.72-0.66 (m, 2H). MS-ESI: calcd for [M+H]⁺ 568, found 568.

### Example 018 Preparation of 4-(5-(3,5-dichlorophenyl)-5-(trifluoromethyl)-4,5-dihydroisoxazol-3-yl)-2-methyl-N-(2-oxo-2-(( 1-(trifluoromethyl)cyclopropyl)methyl)amino)ethyl)benzamide (18)

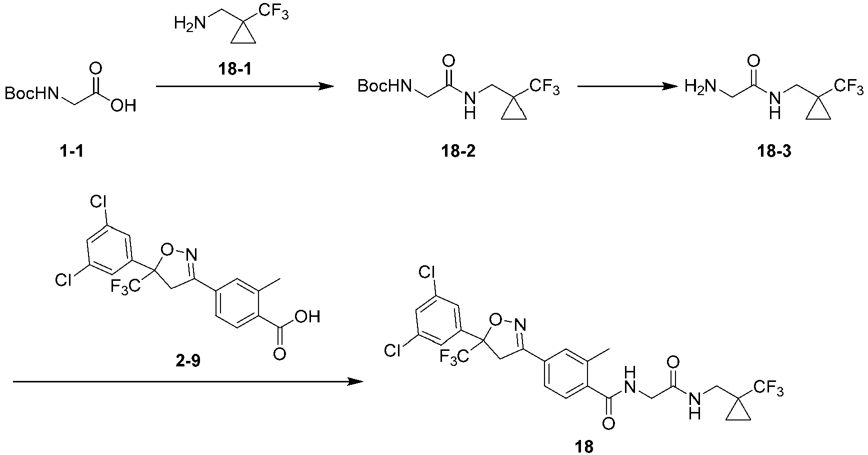

**Step 1:** Compound **1-1** (500 mg, 2.85 mmol, 1 eq.) and a hydrochloride of Compound **18-1** (601 mg, 3.43 mmol, 1.2 eq.) were dissolved in DMF (8 mL), added with HATU (1.63 g, 4.28 mmol, 1.5 eq.) and DIPEA (1.11 g, 8.56 mmol, 3 eq.), and stirred and reacted at 25°C for 12 h. The reaction was quenched with water (30 mL) and extracted with ethyl acetate (10 mL × 2). Organic phases were combined, washed with saturated brine (10 mL × 2), dried over anhydrous magnesium sulfate, filtered, and concentrated under reduced pressure to obtain a crude product, Compound **18-2** (800 mg with a yield of 95%).

**Step 2:** Compound **18-2** (800 mg, 2.70 mmol, 1 eq.) was dissolved in a dioxane solution of hydrogen chloride (4 M, 10 mL) and stirred and reacted at 25°C for 2 h. The reaction solution was concentrated under reduced pressure to obtain a crude product, a hydrochloride of Compound **18-3** (600 mg with a yield of 95%).

**Step 3:** Compound **2-9** (50 mg, 0.120 mmol, 1 eq.) and the hydrochloride of Compound **18-3** (41.7 mg, 0.179 mmol, 1.5 eq.) were dissolved in DMF (2 mL), added with HATU (68.2 mg, 0.179 mmol, 1.5 eq.) and DIPEA (46.4 mg, 0.359 mmol, 3 eq.), and stirred and reacted at 25°C for 2 h. The reaction was quenched with water (10 mL) and extracted with ethyl acetate (5 mL × 3). Organic phases were combined, washed with saturated brine (5 mL × 2), dried over anhydrous magnesium sulfate, filtered, and concentrated under reduced pressure. The obtained crude product was purified through preparative thin-layer chromatography (SiO₂, ethyl acetate) to obtain Compound **18** (48.7 mg with a yield of 68%). ¹H NMR (400 MHz, CDCl₃) δ 7.56-7.40 (m, 6H), 6.72 (t, *J=* 4.8 Hz, 1H), 6.55 (t, *J =* 5.2 Hz, 1H), 4.17-4.03 (m, 3H), 3.70 (d, *J* = 17.2 Hz, 1H), 3.52 (d, *J* = 6.0 Hz, 2H), 2.47 (s, 3H), 1.05-0.98 (m, 2H), 0.85-0.75 (s, 2H). MS-ESI: calcd for [M+H]⁺ 596, found 596.

### Example 019 Preparation of 4-(5-(3,5-dichloro-2-fluorophenyl)-5-(trifluoromethyl)-4,5-dihydroisoxazol-3-yl)-2-methyl-N-(2-oxo-2-((1-(trifluoromethyl)cyclopropyl)amino)ethyl)benzamide (19)

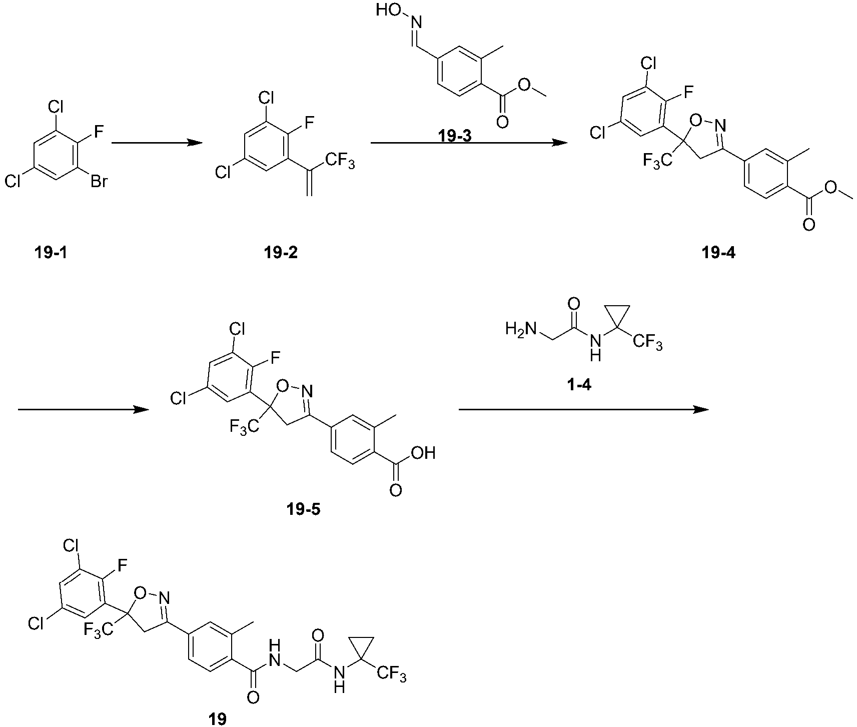

**Step 1:** Compound **19-1** (1.00 g, 4.10 mmol, 1 eq.) and 1-(trifluoromethyl)vinyl boronic acid hexylene glycol ester (1.10 g, 4.92 mmol, 1.2 eq.) were dissolved in dioxane (10 mL) and water (2 mL), added with Pd(dppf)Cl₂ (300.0 mg, 0.410 mmol, 0.1 eq.) and K₂CO₃ (1.70 g, 12.3 mmol, 3 eq.), and stirred and reacted at 100°C for 12 h under nitrogen protection. The reaction solution was concentrated under reduced pressure to obtain a crude product. The crude product was purified through column chromatography (SiO₂, n-heptane) to obtain Compound **19-2** (700 mg with a yield of 66%). ¹H NMR (400 MHz, CDCl₃) δ 7.46 (d, *J =* 6.0 Hz, 1H), 7.27-7.21 (m, 1H), 6.27 (s, 1H), 5.83 (s, 1H).

**Step 2:** Compound **19-3** (149 mg, 0.772 mmol, 1 eq.) was dissolved in DMF (5 mL), added with NCS (155 mg, 1.16 mmol, 1.5 eq.), and stirred and reacted at 25°C for 2 h. Then, Compound **19-2** (200 mg, 0.772 mmol, 1 eq.) and triethylamine (234 mg, 2.32 mmol, 3 eq.) were dissolved in DMF (2 mL), added dropwise to the above reaction solution, and stirred and reacted at 25°C for 12 h. The reaction was quenched with water (10 mL) and extracted with ethyl acetate (5 mL × 3). Organic phases were combined, washed with saturated brine (5 mL × 2), dried over anhydrous magnesium sulfate, filtered, and concentrated under reduced pressure. The obtained crude product was purified through column chromatography (SiO₂, n-heptane:ethyl acetate = 10:1) to obtain Compound **19-4** (100 mg with a yield of 29%). ¹H NMR (400 MHz, CDCl₃) δ 7.97 (d, *J=* 8.6 Hz, 1H), 7.74 (dd, *J=* 6.0, 2.8 Hz, 1H), 7.59-7.54 (m, 2H), 7.52 (dd, *J=* 6.0, 2.8 Hz, 1H), 4.18 (dd, *J =* 18.0, 2.2 Hz, 1H), 3.92 (s, 3H), 3.86 (dd, *J =* 18.0, 1.2 Hz, 1H), 2.64 (s, 3H).

**Step 3:** Compound **19-4** (100 mg, 0.222 mmol, 1 eq.) was dissolved in THF (1.5 mL), methanol (1 mL), and water (0.5 mL), added with lithium hydroxide monohydrate (28.0 mg, 0.666 mmol, 3 eq.), and stirred and reacted at 25°C for 12 h. The reaction solution was adjusted to a pH of about 3 with 1 M HCl and extracted with ethyl acetate (5 mL × 2). Organic phases were combined, washed with saturated brine (5 mL × 2), dried over anhydrous magnesium sulfate, filtered, and concentrated under reduced pressure to obtain Compound **19-5** (50 mg with a yield of 52%).

**Step 4:** Compound **19-5** (50 mg, 0.115 mmol, 1 eq.) and a hydrochloride of Compound **1-4** (50.1 mg, 0.229 mmol, 2 eq.) were dissolved in DMF (2 mL), added with HATU (131 mg, 0.344 mmol, 3 eq.) and DIPEA (44.4 mg, 0.344 mmol, 3 eq.), and stirred and reacted at 25°C for 2 h. The reaction was quenched with water (10 mL) and extracted with ethyl acetate (5 mL × 3). Organic phases were combined, washed with saturated brine (5 mL × 2), dried over anhydrous magnesium sulfate, filtered, and concentrated under reduced pressure. The obtained crude product was purified through preparative thin-layer chromatography (SiO₂, ethyl acetate) to obtain Compound **19** (30 mg with a yield of 44%). ¹H NMR (400 MHz, CDCl₃) δ 7.75-7.71 (m, 1H), 7.56-7.46 (m, 4H), 7.12 (s, 1H), 6.80 (t, *J =* 5.2 Hz, 1H), 4.20-4.11 (m, 3H), 3.84 (d, *J =* 16.6 Hz, 1H), 2.47 (s, 3H), 1.37-1.32 (m, 2H), 1.17-1.10 (m, 2H). MS-ESI: calcd for [M+H]⁺ 600, found 600.

### Example 020 Preparation of 2-methyl-N-(2-oxo-2-((1-(trifluoromethyl)cyclopropyl)amino)ethyl)-4-(5-(2,3,5-trichlorophenyl) -5-(trifluoromethyl)-4,5-dihydroisoxazol-3-yl)benzamide (20)

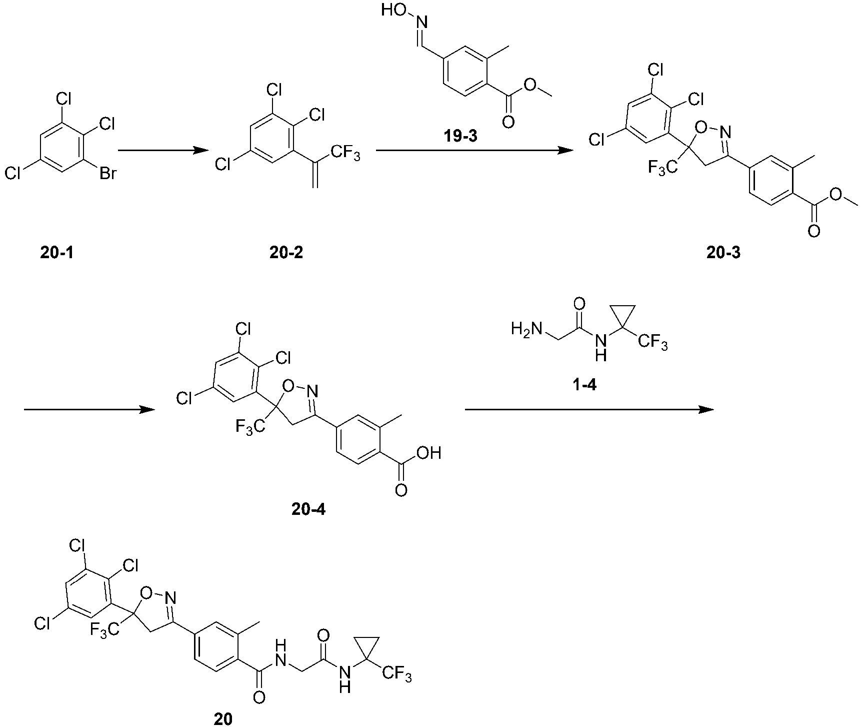

**Step 1:** Compound **20-1** (1.20 g, 4.61 mmol, 1 eq.) and 1-(trifluoromethyl)vinyl boronic acid hexylene glycol ester (1.23 g, 5.53 mmol, 1.2 eq.) were dissolved in dioxane (20 mL) and water (4 mL), added with Pd(dppf)Cl₂ (337.3 mg, 0.461 mmol, 0.1 eq.) and K₂CO₃ (1.91 g, 13.8 mmol, 3 eq.), and stirred and reacted at 100°C for 12 h under nitrogen protection. The reaction solution was concentrated under reduced pressure to obtain a crude product. The crude product was purified through column chromatography (SiO₂, n-heptane) to obtain Compound **20-2** (800 mg with a yield of 63%). ¹H NMR (400 MHz, CDCl₃) δ 7.54 (d, *J =* 2.4 Hz, 1H), 7.24 (d, *J=* 2.4 Hz, 1H), 6.26 (d, *J=* 1.6 Hz, 1H), 5.69 (d, *J=* 1.6 Hz, 1H).

**Step 2:** Compound **19-3** (140.3 mg, 0.726 mmol, 1 eq.) was dissolved in DMF (5 mL), added with NCS (145.4 mg, 1.09 mmol, 1.5 eq.), and stirred and reacted at 25°C for 2 h. After the reaction was completed, Compound **20-2** (200 mg, 0.726 mmol, 1 eq.) and triethylamine (220 mg, 2.18 mmol, 3 eq.) were dissolved in DMF (2 mL), added dropwise to the above reaction solution, and stirred and reacted at 25°C for 12 h. The reaction was quenched with water (10 mL) and extracted with ethyl acetate (5 mL × 3). Organic phases were combined, washed with saturated brine (5 mL × 2), dried over anhydrous magnesium sulfate, filtered, and concentrated under reduced pressure. The obtained crude product was purified through column chromatography (SiO₂, n-heptane:ethyl acetate = 10:1) to obtain Compound **20-3** (50 mg with a yield of 15%). ¹H NMR (400 MHz, CDCl₃) δ 7.97 (d, *J =* 8.6 Hz, 1H), 7.92 (d, *J =* 2.4 Hz, 1H), 7.60 (d, *J=* 2.4 Hz, 1H), 7.59-7.55 (m, 2H), 4.26-4.18 (m, 1H), 4.11-4.05 (m, 1H), 3.92 (s, 3H), 2.65 (s, 3H).

**Step 3:** Compound **20-3** (50 mg, 0.107 mmol, 1 eq.) was dissolved in THF (1.5 mL), methanol (1 mL), and water (0.5 mL), added with lithium hydroxide monohydrate (13.5 mg, 0.321 mmol, 3 eq.), and stirred and reacted at 25°C for 12 h. The reaction solution was adjusted to a pH of about 3 with 1 M HCl and extracted with ethyl acetate (5 mL × 2). Organic phases were combined, washed with saturated brine (5 mL × 2), dried over anhydrous magnesium sulfate, filtered, and concentrated under reduced pressure to obtain a crude product, Compound **20-4** (30 mg with a yield of 62%).

**Step 4:** Compound **20-4** (30 mg, 66.3 µmol, 1 eq.) and a hydrochloride of Compound **1-4** (29.0 mg, 0.133 mmol, 2 eq.) were dissolved in DMF (2 mL), added with HATU (75.6 mg, 0.199 mmol, 3 eq.) and DIPEA (25.7 mg, 0.199 mmol, 3 eq.), and stirred and reacted at 25°C for 2 h. The reaction was quenched with water (10 mL) and extracted with ethyl acetate (5 mL × 3). Organic phases were combined, washed with saturated brine (5 mL × 2), dried over anhydrous magnesium sulfate, filtered, and concentrated under reduced pressure. The obtained crude product was purified through preparative thin-layer chromatography (SiO₂, ethyl acetate) to obtain Compound **20** (3 mg with a yield of 7%). ¹H NMR (400 MHz, CDCl₃) δ 7.91 (d, *J =* 2.4 Hz, 1H), 7.60 (d, *J =* 2.4 Hz, 1H), 7.58-7.53 (m, 2H), 7.50-7.47 (m, 1H), 6.65 (s, 1H), 6.59-6.53 (m, 1H), 4.24-4.17 (m, 1H), 4.13-4.02 (m, 3H), 2.50 (s, 3H), 1.37 (s, 2H), 1.18-1.14 (m, 2H). MS-ESI: calcd for [M+H]⁺ 616, found 616.

### Example 021 Preparation of N-(2-(3-cyano-3-fluoroazetidin-1-yl)-2-oxoethyl)-4-(5-(3,5-dichlorophenyl)-5-(trifluoromethyl)-4,5-dihydroisoxazol-3-yl)-2-methylbenzamide (21)

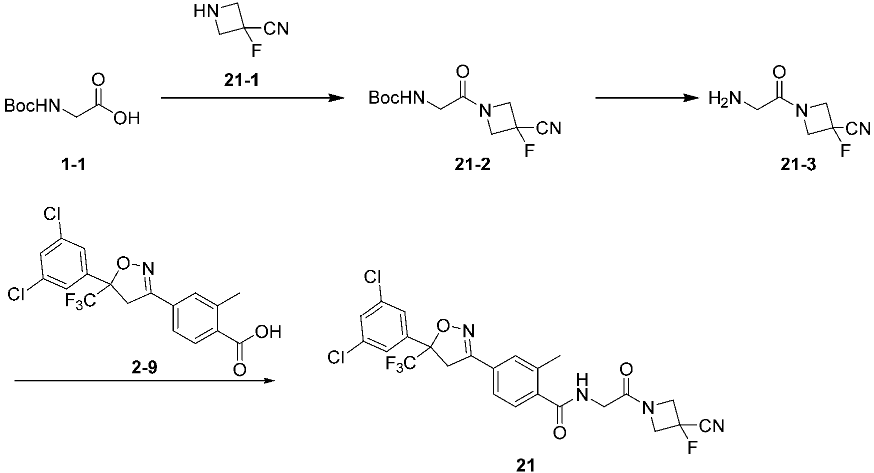

**Step 1:** Compound **1-1** (230 mg, 1.32 mmol, 1 eq.) and an oxalate of Compound **21-1** (250 mg, 1.32 mmol, 1 eq.) were dissolved in DCM (3 mL), added with DIPEA (510 mg, 3.95 mmol, 3 eq.) and T₄P (1.42 g, 1.972 mmol, 50% ethyl acetate solution, 1.5 eq.), and stirred and reacted at 25°C for 2 h. The reaction was quenched with water (10 mL) and extracted with ethyl acetate (10 mL × 3). Combined organic phases were washed with saturated NaHCO₃ (10 mL × 2), dried over anhydrous magnesium sulfate, and filtered. The filtrate was concentrated under reduced pressure to obtain a crude product, Compound **21-2** (300 mg with a yield of 89%).

**Step 2:** Compound **21-2** (300 mg, 1.166 mmol, 1 eq.) was dissolved in DCM (5 mL), added with TFA (1 mL), and stirred and reacted at 25°C for 2 h. The reaction solution was concentrated under reduced pressure to obtain a crude product, a trifluoroacetate of Compound **21-3** (120 mg with a yield of 37%).

**Step 3:** Compound **2-9** (50 mg, 0.120 mmol, 1 eq.) and the trifluoroacetate of Compound **21-3** (48.5 mg, 0.179 mmol, 1.5 eq.) were dissolved in DMF (2 mL), added with HATU (68.2 mg, 0.180 mmol, 1.5 eq.) and DIPEA (61.8 mg, 0.478 mmol, 4 eq.), and stirred and reacted at 25°C for 12 h. The reaction was quenched with water (5 mL) and extracted with ethyl acetate (5 mL × 2). Organic phases were combined, washed with saturated brine (5 mL × 3), dried over anhydrous magnesium sulfate, filtered, and concentrated under reduced pressure. The obtained crude product was purified through preparative thin-layer chromatography (SiO₂, ethyl acetate) to obtain Compound **21** (14 mg with a yield of 15%). ¹H NMR (400 MHz, CDCl₃) δ 7.58-7.41 (m, 6H), 6.67 (t, *J =* 4.4 Hz, 1H), 4.89-4.77 (m, 1H), 4.74-4.58 (m, 2H), 4.55-4.43 (m, 1H), 4.15-4.03 (m, 3H), 3.75-3.67 (m, 1H), 2.49 (s, 3H). MS-ESI: calcd for [M+H]⁺ 557, found 557.

### Example 022 Preparation of 4-(5-(3,5-dichlorophenyl)-5-(trifluoromethyl)-4,5-dihydroisoxazol-3-yl)-N-(2-(3,3-difluoroazetid in-1-yl)-2-oxoethyl)-2-methylbenzamide (22)

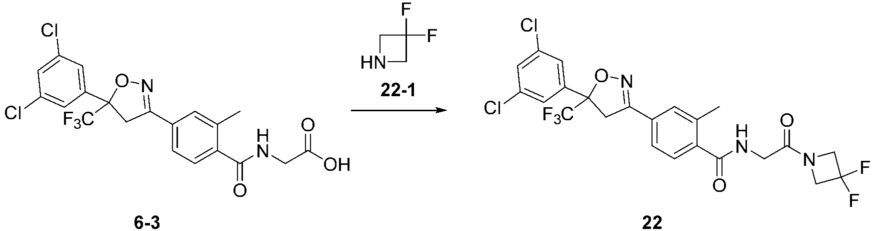

**Step 1:** Compound **6-3** (50.0 mg, 105 µmol, 1.0 eq.), a hydrochloride of Compound **22-1** (27.3 mg, 210 µmol, 1.5 eq.), HATU (60.0 mg, 158 µmol, 1.5 eq.), and DIPEA (54.4 mg, 421 µmol, 4.0 eq.) were dissolved in DMF (2 mL) and stirred at 20°C for 1 h. The system was added with water (2 mL) and ethyl acetate (2 mL) and separated into layers. The aqueous phase was extracted with ethyl acetate (1 mL × 2). Organic phases were combined, washed with water (1 mL × 3), washed with saturated brine (1 mL), dried over anhydrous magnesium sulfate, filtered, and concentrated under reduced pressure. The obtained crude product was purified though preparative thin-layer chromatography (SiO₂, n-heptane:ethyl acetate = 1:1) to obtain Compound 22 (25 mg with a yield of 43%). ¹H NMR (400 MHz, CDCl₃) δ 7.59-7.37 (m, 6H), 6.60 (t, *J =* 4.4 Hz, 1H), 4.64-4.40 (m, 4H), 4.21-4.00 (m, 3H), 3.72 (d, *J =* 17.2 Hz, 1H), 2.51 (s, 3H). MS-ESI: calcd for [M+H]⁺ 550, found 550.

### Example 023 Preparation of 4-(5-(3,5-dichlorophenyl)-5-(trifluoromethyl)-4,5-dihydroisoxazol-3-yl)-2-methyl-N-(2-oxo-2-(3 -(trifluoromethyl)azetidin-1-yl)ethyl)benzamide (23)

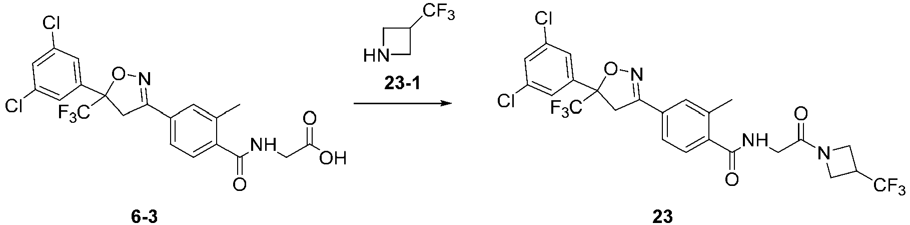

**Step 1:** Compound **22-1** (50.0 mg, 105 µmol, 1.0 eq.), a hydrochloride of Compound 23-1 (34.0 mg, 210 µmol, 1.5 eq.), HATU (60.0 mg, 158 µmol, 1.5 eq.), and DIPEA (54.4 mg, 421 µmol, 4.0 eq.) were dissolved in DMF (2 mL) and reacted at 20°C for 1 h. The system was added with water (2 mL) and ethyl acetate (2 mL) and separated into layers. The aqueous phase was extracted with ethyl acetate (1 mL × 2). Organic phases were combined, washed with water (1 mL), washed with saturated brine (1 mL), and concentrated under reduced pressure. The obtained crude product was purified though preparative thin-layer chromatography (SiO₂, n-heptane:ethyl acetate = 1:1) to obtain Compound 23 (25 mg with a yield of 41%). ¹H NMR (400 MHz, CDCl₃) δ 7.61-7.40 (m, 6H), 6.62 (t, *J =* 4.4 Hz, 1H), 4.51-4.34 (m, 1H), 4.33-4.24 (m, 2H), 4.20-4.02 (m, 4H), 3.72 (d, *J =* 17.2 Hz, 1H), 3.52-3.25 (m, 1H), 2.51 (s, 3H). MS-ESI: calcd for [M+H]⁺ 582, found 582.

### Example 024 Preparation of N-(2-((1-cyanocyclopropyl)amino)-2-oxoethyl)-4-(5-(3,5-dichlorophenyl)-5-(trifluoromethyl)-4, 5-dihydroisoxazol-3-yl)-2-methylbenzamide (24)

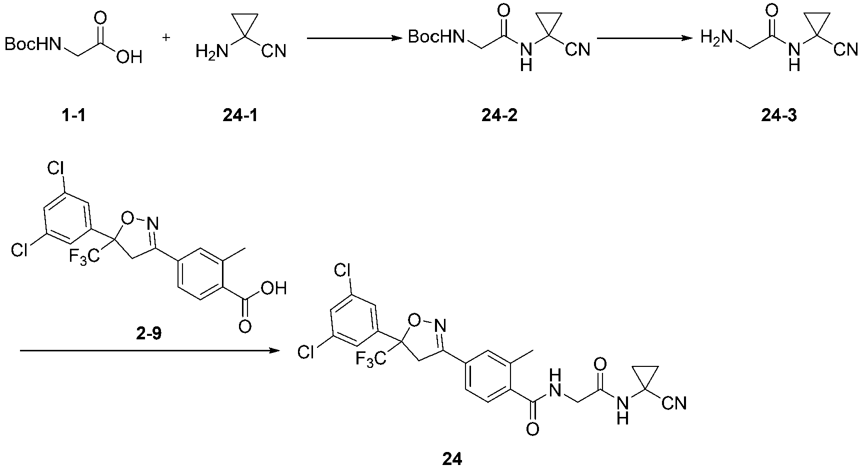

**Step 1:** Compound 1-1 (400 mg, 2.28 mmol, 1.0 eq.), a hydrochloride of Compound 24-1 (271 mg, 2.28 mmol, 1.0 eq.), HATU (1.04 g, 2.74 mmol, 1.2 eq.), and DIPEA (1.88 g, 6.85 mmol, 3.0 eq.) were dissolved in DMF (9.8 mL) and reacted at 20°C for 1 h. The reaction was quenched with water (10 mL) and extracted with ethyl acetate (10 mL × 2). Organic phases were washed with saturated brine (15 mL × 2), dried over anhydrous magnesium sulfate, filtered, and concentrated under reduced pressure to obtain Compound **24-2** (520 mg with a yield of 95%).

**Step 2:** Compound **24-2** (520 mg, 2.17 mmol, 1.0 eq.) was dissolved in 1,4-dioxane (5 mL), and a 1,4-dioxane solution of hydrogen chloride (4 M, 5 mL) was added dropwise and reacted at 20°C for 12 h. The system was concentrated under reduced pressure to obtain a crude product, a hydrochloride of Compound **24-3** (350 mg with a yield of 92%).

**Step 3:** Compound **2-9** (100 mg, 239 µmol, 1.0 eq.), the hydrochloride of Compound **24-3** (66.6 mg, 478 µmol, 2.0 eq.), HATU (136 mg, 359 µmol, 1.5 eq.), and DIPEA (124 mg, 956 µmol, 4.0 eq.) were dissolved in DMF (2 mL) and reacted at 20°C for 1 h. The system was added with water (10 mL) and ethyl acetate (10 mL) and extracted with ethyl acetate (10 mL × 2). Organic phases were combined, washed with water (10 mL), washed with saturated brine (5 mL), dried over anhydrous magnesium sulfate, filtered, and concentrated under reduced pressure. The crude product was separated and purified through preparative high-performance liquid chromatography (column: X-Bridge C18, 50 × 4.6 mm I.D., 3.5 µm; mobile phase: [0.1% formic acid aqueous solution-acetonitrile]; gradient: 20%-95%, 3 min) to obtain Compound **24** (10 mg with a yield of 8%). ¹H NMR (400 MHz, CDCl₃) δ 7.61-7.43 (m, 6H), 6.76 (t, *J =* 4.8 Hz, 1H), 4.73 (br, s, 1H), 4.15-4.11 (m, 3H), 3.73 (d, *J =* 17.2 Hz, 1H), 2.52 (s, 3H), 1.73-1.47 (m, 2H), 1.36-1.30 (m, 2H). MS-ESI: calcd for [M+H]⁺ 539, found 539.

### Example 025 Preparation of 4-(5-(3,5-dichlorophenyl)-5-(trifluoromethyl)-4,5-dihydroisoxazol-3-yl)-N-(2-(3-fluoro-3-(triflu oromethyl)azetidin-1-yl)-2-oxoethyl)-2-methylbenzamide (25)

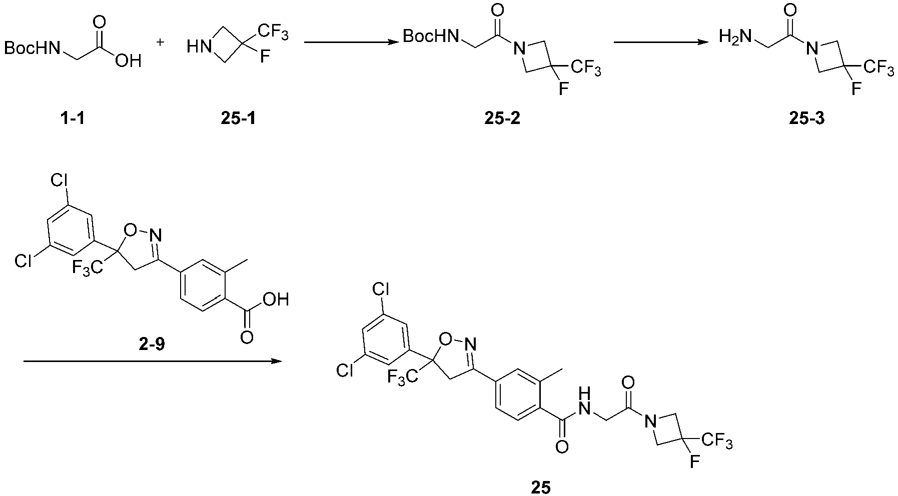

**Step 1:** Compound **1-1** (500 mg, 2.85 mmol, 1.0 eq.), a hydrochloride of Compound **25-1** (769 mg, 4.28 mmol, 1.5 eq.), HATU (1.63 g, 4.28 mmol, 1.5 eq.), and DIPEA (1.48 g, 11.4 mmol, 4.0 eq.) were dissolved in DMF (5 mL) and reacted at 20°C for 1 h. The system was added with water (10 mL) and ethyl acetate (10 mL) and extracted with ethyl acetate (10 mL × 2). Organic phases were combined, washed with water (10 mL), washed with saturated brine (10 mL × 2), dried over anhydrous magnesium sulfate, filtered, and concentrated under reduced pressure to obtain a crude product, Compound **25-2** (856 mg with a yield of 100%). ¹H NMR (400 MHz, CDCl₃) δ 4.59-4.23 (m, 4H), 3.85-3.78 (m, 2H), 1.47 (s, 9H).

**Step 2:** Compound **25-2** (856 mg, 2.85 mmol, 1.0 eq.) was dissolved in dichloromethane (1 mL), and trifluoroacetic acid (1.30 g, 11.4 mmol, 4.0 eq.) was added dropwise and reacted at 20°C for 16 h. The system was concentrated under reduced pressure to obtain a crude product, a trifluoroacetate of Compound **25-3** (895 mg with a yield of 100%).

**Step 3:** Compound **2-9** (50.0 mg, 120 µmol, 1.0 eq.), the trifluoroacetate of Compound **25-3** (56.3 mg, 179 µmol, 1.5 eq.), HATU (68.2 mg, 179 µmol, 1.5 eq.), and DIPEA (61.8 mg, 478 µmol, 4.0 eq.) were dissolved in DMF (2 mL) and reacted at 20°C for 1 h. The system was added with water (1 mL) and ethyl acetate (1 mL) and extracted with ethyl acetate (1 mL × 2). Organic phases were combined, washed with water (1 mL), washed with saturated brine (1 mL), dried over anhydrous magnesium sulfate, filtered, and concentrated under reduced pressure. The obtained crude product was purified through preparative thin-layer chromatography (SiO₂, n-heptane:ethyl acetate = 3:1) to obtain Compound **25** (20 mg with a yield of 28%). ¹H NMR (400 MHz, CDCl₃) δ 7.62-7.36 (m, 6H), 6.57 (t, *J =* 4.4 Hz, 1H), 4.73-4.33 (m, 4H), 4.22-4.01 (m, 3H), 3.72 (d, *J=* 17.2 Hz, 1H), 2.51 (s, 3H). MS-ESI: calcd for [M+H]⁺ 600, found 600.

### Example 026 Preparation of 4-(5-(3,5-dichlorophenyl)-5-(trifluoromethyl)-4,5-dihydroisoxazol-3-yl)-N-(2-(3-fluoro-3-(fluoro methyl)azetidin-1-yl)-2-oxoethyl)-2-methylbenzamide (26)

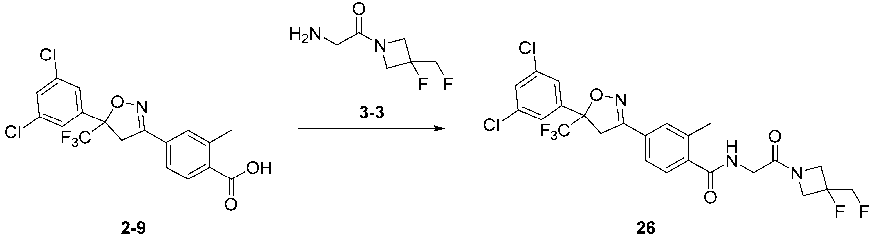

**Step 1:** Compound **2-9** (50.0 mg, 120 µmol, 1.0 eq.), a trifluoroacetate of Compound 3-3 (49.9 mg, 179 µmol, 1.5 eq.), HATU (68.2 mg, 179 µmol, 1.5 eq.), and DIPEA (61.8 mg, 478 µmol, 4.0 eq.) were dissolved in DMF (2 mL) and reacted at 20°C for 1 h. The system was added with water (1 mL) and ethyl acetate (1 mL) and extracted with ethyl acetate (1 mL × 2). Organic phases were combined, washed with water (1 mL), washed with saturated brine (1 mL), dried over anhydrous magnesium sulfate, filtered, and concentrated under reduced pressure. The obtained crude product was purified through preparative thin-layer chromatography (SiO₂, n-heptane:ethyl acetate = 3:1) to obtain Compound **26** (20 mg with a yield of 30%). ¹H NMR (400 MHz, CDCl₃) δ 7.74-7.37 (m, 6H), 6.63 (t, *J =* 4.0 Hz, 1H), 4.79-4.54 (m, 2H), 4.46-4.21 (m, 4H), 4.10-4.08 (m, 3H), 3.72 (d, *J =* 17.2 Hz, 1H), 2.51 (s, 3H). MS-ESI: calcd for [M+H]⁺ 564, found 564.

### Example 027 Preparation of 4-(5-(3-chloro-5-fluorophenyl)-5-(trifluoromethyl)-4,5-dihydroisoxazol-3-yl)-2-methyl-N-(2-oxo -2-((1-(trifluoromethyl)cyclopropyl)amino)ethyl)benzamide (27)

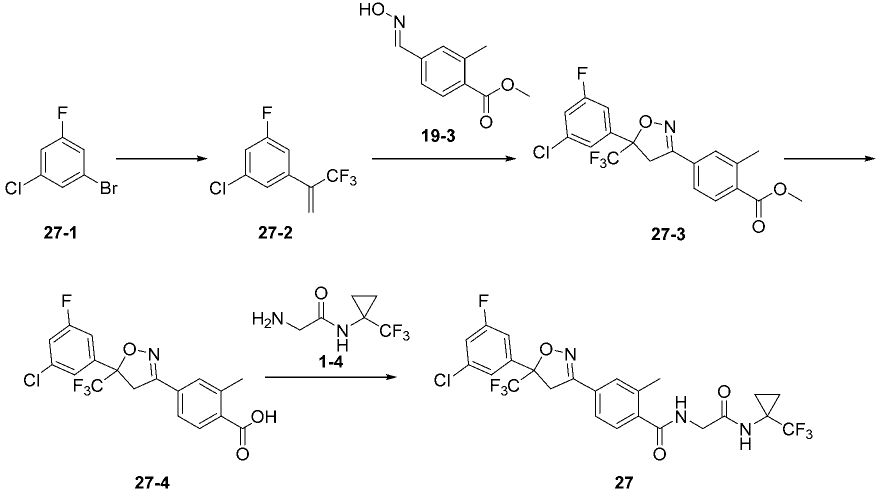

**Step 1:** Compound **27-1** (1.00 g, 4.78 mmol, 1.0 eq.), potassium carbonate (1.98 g, 14.3 mmol, 3.0 eq.), 1-(trifluoromethyl)vinyl boronic acid hexylene glycol ester (1.17 g, 5.25 mmol, 1.1 eq.), and Pd(dppf)Cl₂ (349 mg, 477 µmol, 0.1 eq.) were added to 1,4-dioxane (10 mL) and water (2 mL) and stirred at 90°C for 16 h. The system was filtered, spin-dried to remove the solvents, added with water (10 mL) and ethyl acetate (10 mL), and extracted with ethyl acetate (10 mL × 2). Organic phases were combined, washed with water (10 mL), washed with saturated brine (10 mL), dried over anhydrous magnesium sulfate, filtered, and concentrated under reduced pressure. The crude product was separated and purified through column chromatography (SiO₂, ethyl acetate/n-heptane, 0-1%) to obtain Compound **27-2** (540 mg with a yield of 50%). ¹H NMR (400 MHz, CDCl₃) δ 7.27 (s, 1H), 7.20-7.04 (m, 2H), 6.07 (s, 1H), 5.85 (s, 1H).

**Step 2:** Compound **19-3** (200 mg, 1.04 mmol, 1.0 eq.) and NCS (138 mg, 1.04 mmol, 1.0 eq.) were dissolved in DMF (5 mL) and stirred for 1 h under nitrogen protection, and Compound **27-2** (279 mg, 1.24 mmol, 1.2 eq.) and triethylamine (126 mg, 1.24 mmol, 1.2 eq.) were added and stirred at 20°C for 15 h. The system was added with water (5 mL) and ethyl acetate (5 mL) and extracted with ethyl acetate (5 mL × 2). Organic phases were combined, washed with water (5 mL), washed with saturated brine (5 mL), dried over anhydrous magnesium sulfate, filtered, and concentrated under reduced pressure. The crude product was separated and purified through column chromatography (SiO₂, ethyl acetate/n-heptane, 0-20%) to obtain Compound **27-3** (90 mg with a yield of 21%). ¹H NMR (400 MHz, CDCl₃) δ 8.03-7.93 (m, 1H), 7.58-7.54 (m, 2H), 7.43 (s, 1H), 7.28-7.25 (m, 1H), 7.23-7.14 (m, 1H), 4.12 (d, *J* = 17.1 Hz, 1H), 3.94 (s, 3H), 3.73 (d, *J=* 17.6 Hz, 1H), 2.65 (s, 3H).

**Step 3:** Compound **27-3** (90 mg, 216 µmol, 1.0 eq.) and NaOH (22 mg, 541 µmol, 2.5 eq.) were dissolved in THF (2 mL) and water (2 mL) and stirred at 20°C for 16 h. The system was added with water (5 mL) and methyl tert-butyl ether (5 mL) and separated into layers. The aqueous phase was extracted with ethyl acetate (5 mL), adjusted to a pH of 3-4 with a 1 M HCl aqueous solution, and extracted with ethyl acetate (5 mL × 3). Organic phases were combined, washed with saturated brine (5 mL), dried over anhydrous magnesium sulfate, filtered, and concentrated under reduced pressure to obtain a crude product, Compound **27-4** (40 mg with a yield of 46%).

**Step 4:** Compound **27-4** (35.0 mg, 87.1 µmol, 1.0 eq.), Compound **1-4** (28.6 mg, 131 µmol, 1.5 eq.), HATU (49.7 mg, 131 µmol, 1.5 eq.), and DIPEA (45.0 mg, 348 µmol, 4.0 eq.) were dissolved in DMF (2 mL) and reacted at 20°C for 1 h. The system wad added with water (1 mL) and ethyl acetate (1 mL) and extracted with ethyl acetate (1 mL × 2). Organic phases were combined, washed with water (1 mL), washed with saturated brine (1 mL), dried over anhydrous magnesium sulfate, filtered, and concentrated under reduced pressure. The obtained crude product was purified through preparative thin-layer chromatography (SiO₂, n-heptane:ethyl acetate = 1:3) to obtain Compound **27** (30 mg with a yield of 61%). ¹H NMR (400 MHz, CDCl₃) δ 7.60-7.35 (m, 4H), 7.22-7.10 (m, 2H), 7.28-7.25 (m, 1H), 6.83 (t, *J =* 5.2 Hz, 1H), 4.20-4.02 (m, 3H), 3.72 (d, *J=* 17.2 Hz, 1H), 2.49 (s, 3H), 1.44-1.29 (m, 2H), 1.21-1.11 (m, 2H). MS-ESI: calcd for [M+H]⁺ 566, found 566.

### Example 028 Preparation of 4-(5-(3,4-dichloro-5-fluorophenyl)-5-(trifluoromethyl)-4,5-dihydroisoxazol-3-yl)-2-methyl-N-(2-oxo-2-((1-(trifluoromethyl)cyclopropyl)amino)ethyl)benzamide (28)

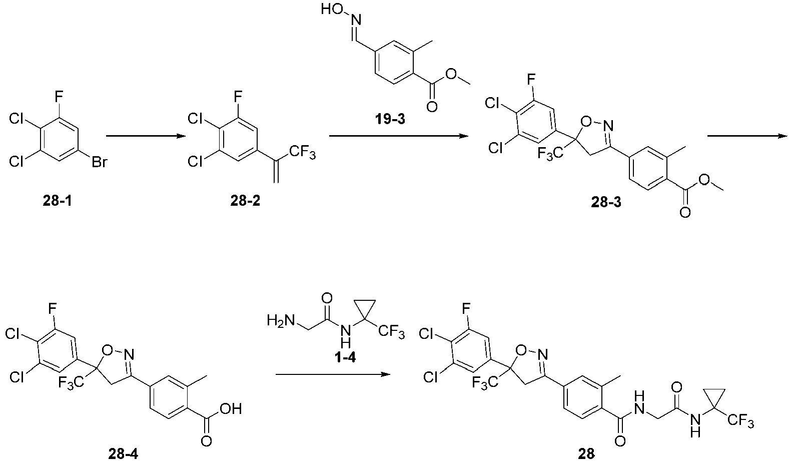

**Step 1:** Compound **28-1** (1.00 g, 4.10 mmol, 1.0 eq.), potassium carbonate (1.70 g, 12.3 mmol, 3.0 eq.), 1-(trifluoromethyl)vinyl boronic acid hexylene glycol ester (1.00 g, 4.51 mmol, 1.1 eq.), and Pd(dppf)Cl₂ (300 mg, 410 µmol, 0.1 eq.) were added to 1,4-dioxane (10 mL) and water (2 mL) and stirred at 90°C for 16 h. The system was filtered, spin-dried to remove the solvents, added with water (10 mL) and ethyl acetate (10 mL), and extracted with ethyl acetate (10 mL × 2). Organic phases were combined, washed with water (10 mL), washed with saturated brine (10 mL), dried over anhydrous magnesium sulfate, filtered, and concentrated under reduced pressure. The crude product was separated and purified through column chromatography (SiO₂, ethyl acetate/n-heptane, 0-1%) to obtain Compound **28-2** (580 mg with a yield of 55%). ¹H NMR (400 MHz, CDCl₃) δ 7.40 (s, 1H), 7.22 (d, *J=* 9.2 Hz, 1H), 6.09 (s, 1H), 5.87 (s, 1H).

**Step 2:** Compound **19-3** (200 mg, 1.04 mmol, 1.0 eq.) and NCS (138 mg, 1.04 mmol, 1.0 eq.) were dissolved in DMF (5 mL) and stirred for 1 h under nitrogen protection, and Compound **28-2** (322 mg, 1.24 mmol, 1.2 eq.) and triethylamine (126 mg, 1.24 mmol, 1.2 eq.) were added and stirred at 20°C for 15 h. The system was added with water (5 mL) and ethyl acetate (5 mL) and extracted with ethyl acetate (5 mL × 2). Organic phases were combined, washed with water (5 mL), washed with saturated brine (5 mL), dried over anhydrous magnesium sulfate, filtered, and concentrated under reduced pressure. The crude product was separated and purified through column chromatography (SiO₂, ethyl acetate/n-heptane, 0-20%) to obtain Compound **28-3** (100 mg with a yield of 21%). ¹H NMR (400 MHz, CDCl₃) δ 7.98 (d, *J =* 8.8 Hz, 1H), 7.57 (s, 2H), 7.43 (s, 1H), 7.21-7.15 (m, 1H), 4.12 (d, *J =* 17.2 Hz, 1H), 3.94 (s, 3H), 3.73 (d, *J=* 17.6 Hz, 1H), 2.65 (s, 3H).

**Step 3:** Compound **28-3** (100 mg, 222 µmol, 1.0 eq.) and NaOH (22 mg, 555 µmol, 2.5 eq.) were dissolved in THF (2 mL) and water (2 mL) and stirred at 60°C for 16 h. THF was spin-removed. The system was added with water (5 mL) and ethyl acetate (5 mL) and separated into layers. The aqueous phase was extracted with ethyl acetate (5 mL). Organic phases were adjusted to a pH of 3-4 with a 1 M HCL aqueous solution and spin-dried. The aqueous phase was adjusted to a pH of 3-4 with the 1 M HCL aqueous solution and extracted with ethyl acetate (5 mL × 3). Organic phases were combined, washed with saturated brine (5 mL), dried over anhydrous magnesium sulfate, filtered, and concentrated under reduced pressure to obtain a crude product, Compound **28-4** (90 mg with a yield of 93%).

**Step 4:** Compound **28-4** (40.0 mg, 91.7 µmol, 1.0 eq.), a hydrochloride of Compound 1-4 (30.1 mg, 138 µmol, 1.5 eq.), HATU (52.3 mg, 138 µmol, 1.5 eq.), and DIPEA (47.4 mg, 367 µmol, 4.0 eq.) were dissolved in DMF (2 mL) and reacted at 20°C for 1 h. The system was added with water (1 mL) and ethyl acetate (1 mL) and extracted with ethyl acetate (1 mL × 2). Organic phases were combined, washed with water (1 mL), washed with saturated brine (1 mL), dried over anhydrous magnesium sulfate, filtered, and concentrated under reduced pressure. The obtained crude product was purified through preparative thin-layer chromatography (SiO₂, n-heptane:ethyl acetate = 1:3) to obtain Compound **28** (20 mg with a yield of 36%). ¹H NMR (400 MHz, CDCl₃) δ 7.61-7.37 (m, 5H), 6.97 (s, 1H), 6.74 (t, *J =* 5.2 Hz, 1H), 4.22-3.99 (m, 3H), 3.71 (d, *J=* 17.2 Hz, 1H), 2.49 (s, 3H), 1.46-1.31 (m, 2H), 1.21-1.11 (m, 2H)). MS-ESI: calcd for [M+H]⁺ 600, found 600.

### Example 029 Preparation of 4-(5-(3,4-dichloro-5-(trifluoromethyl)phenyl)-5-(trifluoromethyl)-4,5-dihydroisoxazol-3-yl)-2-m ethyl-N-(2-oxo-2-((1-(trifluoromethyl)cyclopropyl)amino)ethyl)benzamide (29)

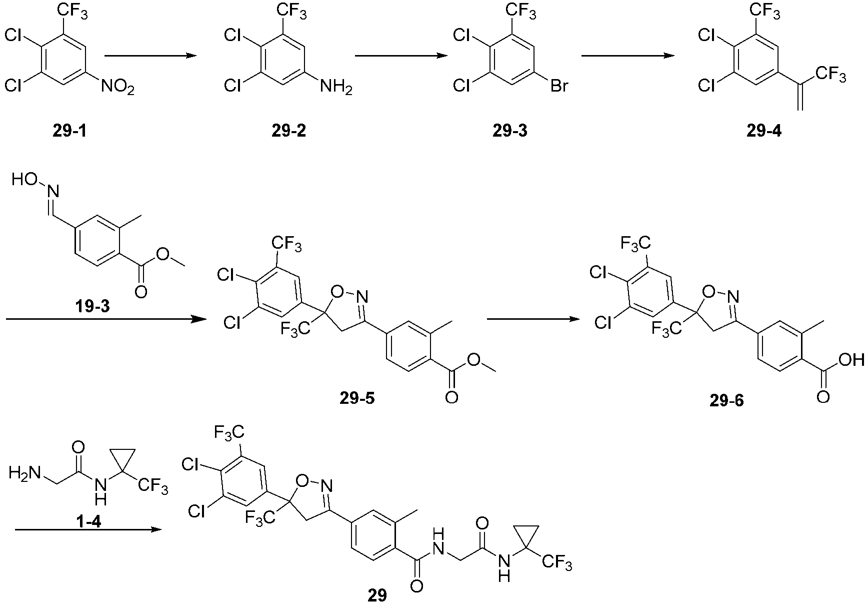

**Step 1:** Compound **29-1** (10.0 g, 38.5 mmol, 1 eq.) was dissolved in methanol (100 mL), added with Pd/C (1.00 g, 10% purity), purged with hydrogen three times, and stirred at 20°C for 16 h under a hydrogen balloon atmosphere. The system was filtered and concentrated under reduced pressure. The obtained crude product was purified through column chromatography (ethyl acetate/n-heptane = 0-12%) to obtain Compound **29-2** (6.05 g with a yield of 68%). ¹H NMR (400 MHz, CDCl₃) δ 6.96 (s, 1H), 6.93 (s, 1H).

**Step 2:** Compound **29-2** (3.00 g, 13.0 mmol, 1.0 eq.) was dissolved in 20% dilute sulfuric acid (10 mL) and acetonitrile (10 mL). An aqueous solution of sodium nitrite (1.35 g, 1 mL, 19.6 mmol, 1.5 eq.) was added dropwise at 0-10°C in an ice-water bath. After addition, the system was stirred for 0.5 h. A 20% dilute sulfuric acid solution (5 mL) of cuprous bromide (5.61 g, 39.1 mmol, 3.0 eq.) was added dropwise at 0-10°C, gas and heat were discharged, and the system was naturally heated to 20°C and stirred for 15.5 h under a nitrogen atmosphere. The system was extracted with ethyl acetate (10 mL × 3). Organic phases were combined, washed with water (10 mL), washed with saturated brine (10 mL), dried over anhydrous magnesium sulfate, filtered, and concentrated under reduced pressure. The obtained crude product was purified through column chromatography (ethyl acetate/n-heptane = 0-1%) to obtain Compound **29-3** (1.95 g with a yield of 51%). ¹H NMR (400 MHz, CDCl₃) δ = 7.85-7.84 (d, *J* = 2.0 Hz, 1H), 7.78-7.77 (d, *J =* 2.0 Hz, 1H).

**Step 3:** Compound **29-3** (1.00 g, 3.40 mmol, 1.0 eq.), potassium carbonate (1.41 g, 10.2 mmol, 3.0 eq.), 1-(trifluoromethyl)vinyl boronic acid hexylene glycol ester (831 mg, 3.74 mmol, 1.1 eq.), and Pd(dppf)Cl₂ (249 mg, 340 µmol, 0.1 eq.) were added to dioxane (10 mL) and water (2 mL) and stirred at 90°C for 16 h. The system was filtered, the filter cake was washed with ethyl acetate (10 mL × 2), and the filtrate was concentrated under reduced pressure. The crude product was separated and purified through column chromatography (ethyl acetate/n-heptane = 0-1%) to obtain Compound **29-4** (800 mg with a yield of 76%). ¹H NMR (400 MHz, CDCl₃) δ 7.77 (s, 1H), 7.71 (s, 1H), 6.15 (d, *J=* 0.8 Hz, 1H), 5.92-5.84 (m, 1H).

**Step 4:** Compound **19-3** (200 mg, 1.04 mmol, 1.0 eq.) and NCS (138 mg, 1.04 mmol, 1.0 eq.) were dissolved in DMF (5 mL) and stirred for 1 h under nitrogen protection, and Compound **29-4** (320 mg, 1.04 mmol, 1.0 eq.) and triethylamine (126 mg, 1.24 mmol, 1.2 eq.) were added and stirred at 20°C for 1 h. The system was added with water (5 mL) and ethyl acetate (5 mL) and extracted with ethyl acetate (5 mL × 2). Organic phases were combined, washed with water (5 mL), washed with saturated brine (5 mL), dried over anhydrous magnesium sulfate, filtered, and concentrated under reduced pressure. The crude product was separated and purified through column chromatography (ethyl acetate/n-heptane = 0-5%) to obtain Compound **29-5** (190 mg with a yield of 37%). ¹H NMR (400 MHz, CDCl₃) δ 8.05-7.92 (m, 2H), 7.87 (s, 1H), 7.65-7.42 (m, 2H), 4.17 (d, *J =* 17.2 Hz, 1H), 3.94 (s, 3H), 3.74 (d, *J =* 17.2 Hz, 1H), 2.65 (s, 3H).

**Step 5:** Compound **29-5** (90 mg, 180 µmol, 1.0 eq.) and NaOH (22 mg, 540 µmol, 3.0 eq.) were dissolved in THF (2 mL) and water (2 mL) and stirred at 55°C for 16 h. The aqueous phase was adjusted to a pH of 3-4 with a 1 N HCl aqueous solution and extracted with ethyl acetate (5 mL × 2). Organic phases were combined, washed with saturated brine (5 mL), dried over anhydrous magnesium sulfate, filtered, and concentrated under reduced pressure to obtain Compound **29-6** (80.0 mg, crude product).

**Step 6:** Compound **29-6** (80.0 mg, 165 µmol, 1.0 eq.), a hydrochloride of Compound **1-4** (54.0 mg, 247 µmol, 1.5 eq.), HATU (93.8 mg, 247 µmol, 1.5 eq.), and DIPEA (85.1 mg, 658 µmol, 4.0 eq.) were dissolved in DMF (2 mL) and reacted at 20°C for 1 h. The system was added with water (1 mL) and ethyl acetate (1 mL) and extracted with ethyl acetate (1 mL × 2). Organic phases were combined, washed with water (1 mL), washed with saturated brine (1 mL), dried over anhydrous magnesium sulfate, filtered, and concentrated under reduced pressure. The obtained crude product was purified through preparative thin-layer chromatography (n-heptane:ethyl acetate = 1:3) to obtain Compound **29** (50.0 mg with a yield of 47%). ¹H NMR (400 MHz, CDCl₃) δ = 7.97 (d, *J =* 1.6 Hz, 1H), 7.86 (s, 1H), 7.61-7.44 (m, 3H), 7.26 (s, 1H), 6.90 (t, *J* = 5.2 Hz, 1H), 4.23-4.10 (m, 3H), 3.73 (d, *J =* 17.2 Hz, 1H), 2.48 (s, 3H), 1.43-1.31 (m, 2H), 1.18- 1.04 (m, 2H). MS-ESI: calcd for [M+H]⁺ 650, found 650.

### Example 030 Preparation of 4-(5-(3,5-bis(trifluoromethyl)phenyl)-5-(trifluoromethyl)-4,5-dihydroisoxazol-3-yl)-2-methyl-N-(2-oxo-2-((1-(trifluoromethyl)cyclopropyl)amino)ethyl)benzamide (30)

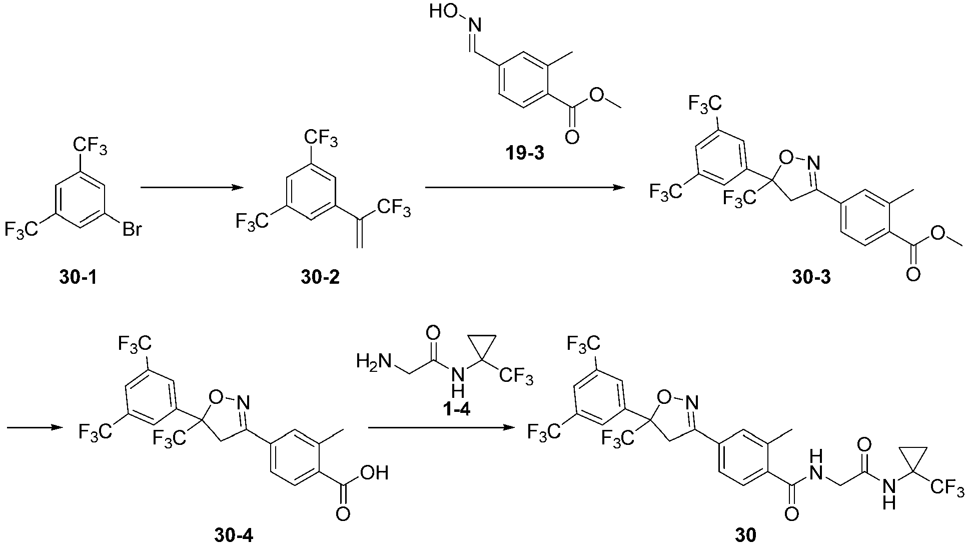

**Step 1:** Compound **30-1** (2.50 g, 8.53 mmol, 1.0 eq.), potassium carbonate (3.54 g, 25.6 mmol, 3.0 eq.), 1-(trifluoromethyl)vinyl boronic acid hexylene glycol ester (2.27 g, 10.2 mmol, 1.2 eq.), and Pd(dppf)Cl₂ (624 mg, 853 µmol, 0.1 eq.) were added to dioxane (40 mL) and water (10 mL) and stirred at 90°C for 12 h. The reaction solution was concentrated under reduced pressure. The crude product was separated and purified through column chromatography (n-heptane, 100%) to obtain Compound **30-2** (2.00 g with a yield of 76%). ¹H NMR (400 MHz, CDCl₃) δ 7.93 (s, 1H), 7.90 (s, 2H), 6.19 (s, 1H), 5.94 (d, *J=* 1.2 Hz, 1H).

**Step 2:** Compound **19-3** (200 mg, 1.04 mmol, 1.0 eq.) and NCS (138 mg, 1.04 mmol, 1.0 eq.) were dissolved in DMF (5 mL) and stirred for 1 h under nitrogen protection, and Compound **30-2** (383 mg, 1.24 mmol, 1.2 eq.) and triethylamine (126 mg, 1.24 mmol, 1.2 eq.) were added and stirred at 20°C for 1 h. The system was added with water (5 mL) and ethyl acetate (5 mL) and extracted with ethyl acetate (5 mL × 2). Organic phases were combined, washed with water (5 mL), washed with saturated brine (5 mL), dried over anhydrous magnesium sulfate, filtered, and concentrated under reduced pressure. The crude product was separated and purified through column chromatography (ethyl acetate/n-heptane = 0-5%) to obtain Compound **30-3** (270 mg with a yield of 52%). ¹H NMR (400 MHz, CDCl₃) δ 8.11 (s, 2H), 8.02-7.93 (m, 2H), 7.58 (m, 2H), 4.24 (d, *J =* 17.1 Hz, 1H), 3.94 (s, 3H), 3.78 (d, *J =* 17.1 Hz, 1H), 2.66 (s, 3H).

**Step 3:** Compound **30-3** (90 mg, 180 µmol, 1.0 eq.) and NaOH (22 mg, 540 µmol, 3.0 eq.) were dissolved in THF (2 mL) and water (2 mL) and stirred at 20°C for 16 h. The aqueous phase was adjusted to a pH of 3-4 with a 1 N HCl aqueous solution and extracted with ethyl acetate (5 mL × 2). Organic phases were combined, washed with saturated brine (5 mL), dried over anhydrous magnesium sulfate, filtered, and concentrated under reduced pressure to obtain Compound **30-4** (80.0 mg, crude product).

**Step 4:** Compound **30-4** (80.0 mg, 165 µmol, 1.0 eq.), a hydrochloride of Compound **1-4** (54.1 mg, 247 µmol, 1.5 eq.), HATU (94.0 mg, 247 µmol, 1.5 eq.), and DIPEA (85.2 mg, 659 µmol, 4.0 eq.) were dissolved in DMF (2 mL) and reacted at 20°C for 1 h. The system was added with water (1 mL) and ethyl acetate (1 mL) and extracted with ethyl acetate (1 mL × 2). Organic phases were combined, washed with water (1 mL), washed with saturated brine (1 mL), dried over anhydrous magnesium sulfate, filtered, and concentrated under reduced pressure. The obtained crude product was purified through preparative thin-layer chromatography (n-heptane:ethyl acetate = 1:3) to obtain Compound **30** (30.0 mg with a yield of 47%). ¹H NMR (400 MHz, CDCl₃) δ 8.11 (s, 2H), 7.99 (s, 1H), 7.64-7.39 (m, 3H), 7.01 (s, 1H), 6.77 (t, *J* = 5.2 Hz, 1H), 4.23 (d, *J=* 17.2 Hz, 1H), 4.15 (d, *J =* 5.2 Hz, 2H), 3.78 (d, *J =* 17.2 Hz, 1H), 2.50 (s, 3H), 1.43-1.34 (m, 2H), 1.21-1.11 (m, 2H). MS-ESI: calcd for [M+H]⁺ 650, found 650.

### Example 031 Preparation of 4-(5-(3-fluoro-5-(trifluoromethyl)phenyl)-5-(trifluoromethyl)-4,5-dihydroisoxazol-3-yl)-2-methy 1-N-(2-oxo-2-((1-(trifluoromethyl)cyclopropyl)amino)ethyl)benzamide (31)

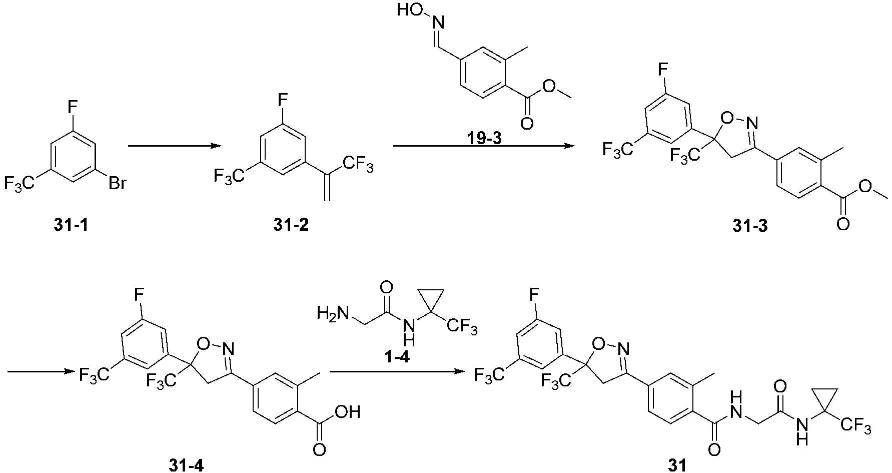

**Step 1:** Compound **31-1** (2.00 g, 8.23 mmol, 1.0 eq.), potassium carbonate (3.41 g, 24.7 mmol, 3.0 eq.), 1-(trifluoromethyl)vinyl boronic acid hexylene glycol ester (2.19 g, 9.88 mmol, 1.2 eq.), and Pd(dppf)Cl₂ (602 mg, 823 µmol, 0.1 eq.) were added to dioxane (10 mL) and water (2 mL) and stirred at 90°C for 16 h. The reaction solution was concentrated under reduced pressure. The crude product was separated and purified through column chromatography (n-heptane, 100%) to obtain Compound **31-2** (2.00 g with a yield of 94%). ¹H NMR (400 MHz, CDCl₃) δ 7.52 (s, 1H), 7.38 (d, *J=* 8.8 Hz, 2H), 6.12 (d, *J=* 1.2 Hz, 1H), 5.90 (d, *J=* 1.6 Hz, 1H).

**Step 2:** Compound **19-3** (200 mg, 1.04 mmol, 1.0 eq.) and NCS (138 mg, 1.04 mmol, 1.0 eq.) were dissolved in DMF (5 mL) and stirred for 1 h under nitrogen protection, and Compound **31-2** (320 mg, 1.24 mmol, 1.2 eq.) and triethylamine (126 mg, 1.24 mmol, 1.2 eq.) were added and stirred at 20°C for 1 h. The system was added with water (5 mL) and ethyl acetate (5 mL) and extracted with ethyl acetate (5 mL × 2). Organic phases were combined, washed with water (5 mL), washed with saturated brine (5 mL), dried over anhydrous magnesium sulfate, filtered, and concentrated under reduced pressure. The crude product was separated and purified through column chromatography (ethyl acetate/n-heptane = 0-5%) to obtain Compound **31-3** (200 mg with a yield of 43%). ¹H NMR (400 MHz, CDCl₃) δ 7.99 (d, *J =* 8.8 Hz, 1H), 7.69 (s, 1H), 7.66-7.54 (m, 3H), 7.44 (d, *J* = 8.0 Hz, 1H), 4.18 (d, *J=* 17.4 Hz, 1H), 3.94 (s, 3H), 3.76 (d, *J=* 17.2 Hz, 1H), 2.65 (s, 3H).

**Step 3:** Compound **31-3** (90 mg, 180 µmol, 1.0 eq.) and NaOH (22 mg, 540 µmol, 3.0 eq.) were dissolved in THF (2 mL) and water (2 mL) and stirred at 20°C for 16 h. The aqueous phase was adjusted to a pH of 3-4 with a 1 N HCl aqueous solution and extracted with ethyl acetate (5 mL × 2). Organic phases were combined, washed with saturated brine (5 mL), dried over anhydrous magnesium sulfate, filtered, and concentrated under reduced pressure to obtain Compound **31-4** (80.0 mg, crude product).

**Step 4:** Compound **31-4** (80.0 mg, 184 µmol, 1.0 eq.), a hydrochloride of Compound **1-4** (60.3 mg, 276 µmol, 1.5 eq.), HATU (105 mg, 276 µmol, 1.5 eq.), and DIPEA (95.0 mg, 735 µmol, 4.0 eq.) were dissolved in DMF (2 mL) and reacted at 20°C for 1 h. The system was added with water (1 mL) and ethyl acetate (1 mL) and extracted with ethyl acetate (1 mL × 2). Organic phases were combined, washed with water (1 mL), washed with saturated brine (1 mL), dried over anhydrous magnesium sulfate, filtered, and concentrated under reduced pressure. The obtained crude product was purified through preparative thin-layer chromatography (n-heptane:ethyl acetate = 1:3) to obtain Compound **31** (30.0 mg with a yield of 50%). ¹H NMR (400 MHz, CDCl₃) δ 7.69 (s, 1H), 7.62-7.40 (m, 5H), 7.07 (s, 1H), 6.79 (t, *J* = 5.2 Hz, 1H), 4.26-4.06 (m, 3H), 3.75 (d, *J=* 17.2 Hz, 1H), 2.49 (s, 3H), 1.45-1.31 (m, 2H), 1.21-1.07 (m, 2H). MS-ESI: calcd for [M+H]⁺ 600, found 600.

### Example 032 Preparation of 4-(5-(3-chloro-5-methylphenyl)-5-(trifluoromethyl)-4,5-dihydroisoxazol-3-yl)-2-methyl-N-(2-ox o-2-((1-(trifluoromethyl)cyclopropyl)amino)ethyl)benzamide (32)

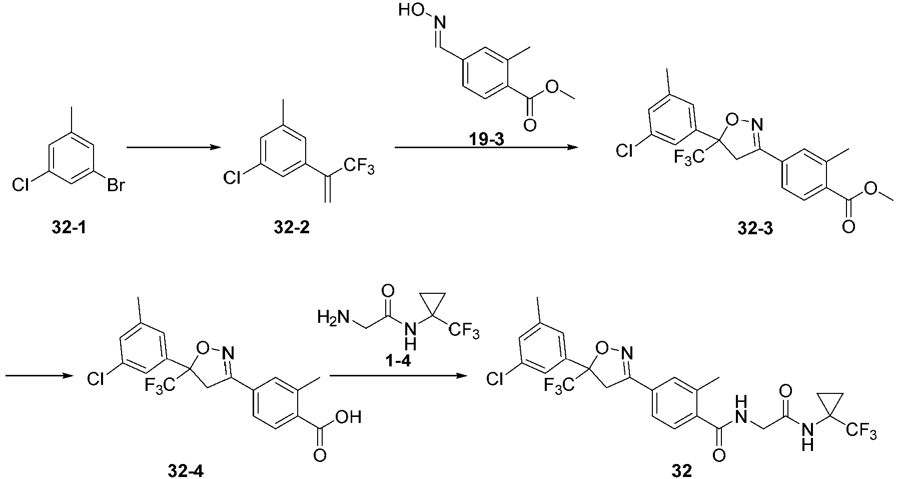

**Step 1:** Compound **32-1** (1.85 g, 9.00 mmol, 1 eq.) and 1-(trifluoromethyl)vinyl boronic acid hexylene glycol ester (2.00 g, 9.00 mmol, 1 eq.) were dissolved in dioxane (40 mL) and water (10 mL), added with Pd(dppf)Cl₂ (659 mg, 0.900 mmol, 0.1 eq.) and potassium carbonate (3.74 g, 27.0 mmol, 3 eq.), and stirred and reacted at 90°C for 12 h under nitrogen protection. The reaction solution was concentrated under reduced pressure to obtain a crude product. The crude product was purified through column chromatography (n-heptane) to obtain Compound **32-2** (1.00 g with a yield of 50%). ¹H NMR (400 MHz, CDCl₃) δ 7.26 (s, 1H), 7.21 (s, 1H), 7.15 (s, 1H), 5.99 (q, *J =* 1.2 Hz, 1H), 5.78 (q, *J =* 1.2 Hz, 1H), 2.37 (s, 3H).

**Step 2:** Compound **19-3** (140 mg, 0.726 mmol, 1 eq.) was dissolved in DMF (5 mL), added with NCS (145 mg, 1.09 mmol, 1.5 eq.), and stirred and reacted at 25°C for 2 h. Then, Compound **32-2** (160 mg, 0.726 mmol, 1 eq.) and triethylamine (220 mg, 2.18 mmol, 3 eq.) were dissolved in DMF (2 mL), added dropwise to the above reaction solution, and stirred and reacted at 25°C for 12 h. The reaction was quenched with water (10 mL) and extracted with ethyl acetate (5 mL × 3). Organic phases were combined, washed with saturated brine (5 mL × 2), dried over anhydrous magnesium sulfate, filtered, and concentrated under reduced pressure. The obtained crude product was purified through column chromatography (n-heptane:ethyl acetate = 9:1) to obtain Compound **32-3** (200 mg with a yield of 67%). ¹H NMR (400 MHz, CDCl₃) δ 7.96 (d, *J =* 8.8 Hz, 1H), 7.58-7.53 (m, 2H), 7.40 (s, 1H), 7.32 (s, 1H), 7.23 (s, 1H), 4.08 (d, *J =* 17.4 Hz, 1H), 3.92 (s, 3H), 3.73 (d, *J=* 17.4 Hz, 1H), 2.63 (s, 3H), 2.39 (s, 3H).

**Step 3:** Compound **32-3** (200 mg, 0.486 mmol, 1 eq.) was dissolved in THF (1.5 mL), methanol (1 mL), and water (0.5 mL), added with lithium hydroxide monohydrate (61.1 mg, 1.46 mmol, 3 eq.), and stirred and reacted at 25°C for 12 h. The reaction solution was adjusted to a pH of about 3 with 1 M HCl and extracted with ethyl acetate (5 mL × 2). Organic phases were combined, washed with saturated brine (5 mL × 2), dried over anhydrous magnesium sulfate, filtered, and concentrated under reduced pressure to obtain Compound **32-4** (50 mg with a yield of 26%).

**Step 4:** Compound **32-4** (50 mg, 0.126 mmol, 1 eq.) and a hydrochloride of Compound **1-4** (41.2 mg, 0.189 mmol, 1.5 eq.) were dissolved in DMF (2 mL), added with HATU (71.7 mg, 0.189 mmol, 1.5 eq.) and DIPEA (48.7 mg, 0.377 mmol, 3 eq.), and stirred and reacted at 25°C for 2 h. The reaction was quenched with water (10 mL) and extracted with ethyl acetate (5 mL × 3). Organic phases were combined, washed with saturated brine (5 mL × 2), dried over anhydrous magnesium sulfate, filtered, and concentrated under reduced pressure. The obtained crude product was purified through preparative thin-layer chromatography (ethyl acetate) to obtain Compound **32** (58.8 mg with a yield of 83%). ¹H NMR (400 MHz, CDCl₃) δ 7.55-7.43 (m, 4H), 7.40 (s, 1H), 7.31 (s, 1H), 7.23 (s, 1H), 7.02 (t, *J =* 4.8 Hz, 1H), 4.16-4.11 (m, 2H), 4.07 (d, *J =* 17.2 Hz, 1H), 3.73 (d, *J =* 17.2 Hz, 1H), 2.45 (s, 3H), 2.39 (s, 3H), 1.35-1.28 (m, 2H), 1.11 (s, 2H). MS-ESI: calcd for [M+H]⁺ 562, found 562.

### Example 033 Preparation of 4-(5-(3-chloro-5-methoxyphenyl)-5-(trifluoromethyl)-4,5-dihydroisoxazol-3-yl)-2-methyl-N-(2-o xo-2-((1-(trifluoromethyl)cyclopropyl)amino)ethyl)benzamide (33)

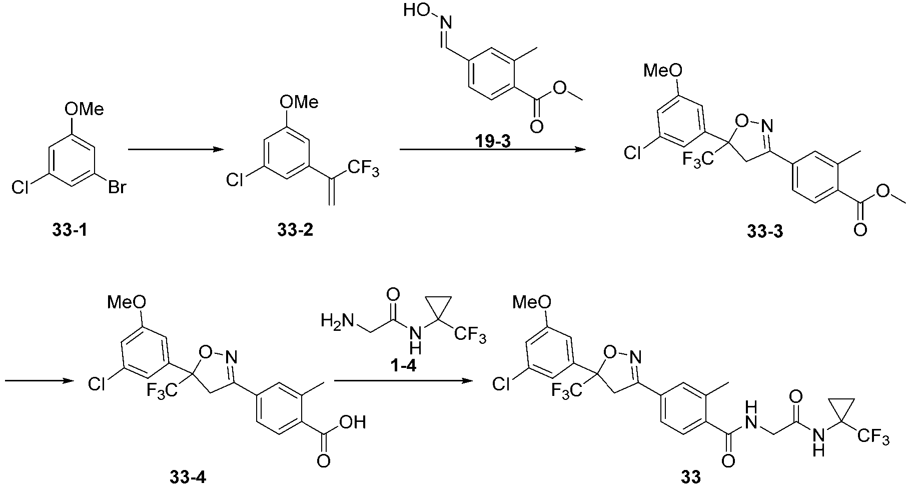

**Step 1:** Compound **33-1** (1.00 g, 4.52 mmol, 1.0 eq.), potassium carbonate (1.87 g, 13.5 mmol, 3.0 eq.), 1-(trifluoromethyl)vinyl boronic acid hexylene glycol ester (1.10 mg, 4.97 mmol, 1.1 eq.), and Pd(dppf)Cl₂ (330 mg, 452 µmol, 0.1 eq.) were added to dioxane (10 mL) and water (2 mL) and stirred at 90°C for 16 h. The system was filtered, the filter cake was washed with ethyl acetate (10 mL × 2), and the filtrate was concentrated under reduced pressure. The crude product was separated and purified through column chromatography (n-heptane, 100%) to obtain Compound **33-2** (860 mg with a yield of 80%). ¹H NMR (400 MHz, CDCl₃) δ 7.11-7.02 (m, 1H), 6.99-6.92 (m, 1H), 6.92-6.85 (m, 1H), 6.01 (d, *J* = 1.2 Hz, 1H), 5.81 (d, *J =* 1.6 Hz, 1H), 3.84 (s, 3H).

**Step 2:** Compound **19-3** (200 mg, 1.04 mmol, 1.0 eq.) and NCS (166 mg, 1.24 mmol, 1.2 eq.) were dissolved in DMF (5 mL) and stirred for 1 h under nitrogen protection, and Compound **33-2** (245 mg, 1.04 mmol, 1.0 eq.) and triethylamine (126 mg, 1.24 mmol, 1.2 eq.) were added and stirred at 20°C for 1 h. The system was added with water (5 mL) and ethyl acetate (5 mL) and extracted with ethyl acetate (5 mL × 2). Organic phases were combined, washed with water (5 mL), washed with saturated brine (5 mL), dried over anhydrous magnesium sulfate, filtered, and concentrated under reduced pressure. The crude product was separated and purified through column chromatography (ethyl acetate/n-heptane = 0-5%) to obtain Compound **33-3** (240 mg with a yield of 54%). ¹H NMR (400 MHz, CDCl₃) δ 7.97 (d, *J* = 8.4 Hz, 1H), 7.65-7.47 (m, 2H), 7.18 (s, 1H), 7.10 (s, 1H), 6.96 (t, *J =* 2.0 Hz, 1H), 4.09 (d, *J* = 17.2 Hz, 1H), 3.97-3.84 (m, 6H), 3.75 (d, *J* = 17.2 Hz, 1H), 2.64 (s, 3H).

**Step 3:** Compound **33-3** (240 mg, 561 µmol, 1.0 eq.) and NaOH (67.3 mg, 1.68 mmol, 3.0 eq.) were dissolved in THF (5 mL) and water (5 mL) and stirred at 20°C for 6 h. The reaction solution was concentrated under reduced pressure. The aqueous phase was adjusted to a pH of 3 with a 1 N HCl aqueous solution and extracted with ethyl acetate (5 mL × 2). Organic phases were combined, washed with saturated brine (5 mL), dried over anhydrous magnesium sulfate, filtered, and concentrated under reduced pressure to obtain Compound **33-4** (190 mg, crude product).

**Step 4:** Compound **33-4** (100 mg, 242 µmol, 1.0 eq.), a hydrochloride of Compound **1-4** (79.2 mg, 363 µmol, 1.5 eq.), HATU (138 mg, 363 µmol, 1.5 eq.), and DIPEA (125 mg, 967 µmol, 4.0 eq.) were dissolved in DMF (2 mL) and reacted at 20°C for 1 h. The system was added with water (1 mL) and ethyl acetate (1 mL) and extracted with ethyl acetate (1 mL × 2). Organic phases were combined, washed with water (1 mL), washed with saturated brine (1 mL), dried over anhydrous magnesium sulfate, filtered, and concentrated under reduced pressure. The obtained crude product was purified through preparative thin-layer chromatography (n-heptane:ethyl acetate = 1:3) to obtain Compound **33** (40.0 mg with a yield of 29%). ¹H NMR (400 MHz, CDCl₃) δ 7.59-7.45 (m, 3H), 7.33 (s, 1H), 7.18 (s, 1H), 7.10 (s, 1H), 6.96 (t, *J* = 2.0 Hz, 1H), 6.92 (t, *J* = 5.2 Hz, 1H), 4.16 (d, *J =* 5.2 Hz, 2H), 4.08 (d, *J =* 17.2 Hz, 1H), 3.86 (s, 3H), 3.74 (d, *J =* 17.2 Hz, 1H), 2.48 (s, 3H), 1.40-1.31 (m, 2H), 1.18-1.09 (m, 2H). MS-ESI: calcd for [M+H]⁺ 578, found 578.

### Example 034 Preparation of 4-(5-(3,5-dichlorophenyl)-5-(trifluoromethyl)-4,5-dihydroisoxazol-3-yl)-N,2-dimethyl-N-(2-oxo-2-((1-(trifluoromethyl)cyclopropyl)amino)ethyl)benzamide (34)

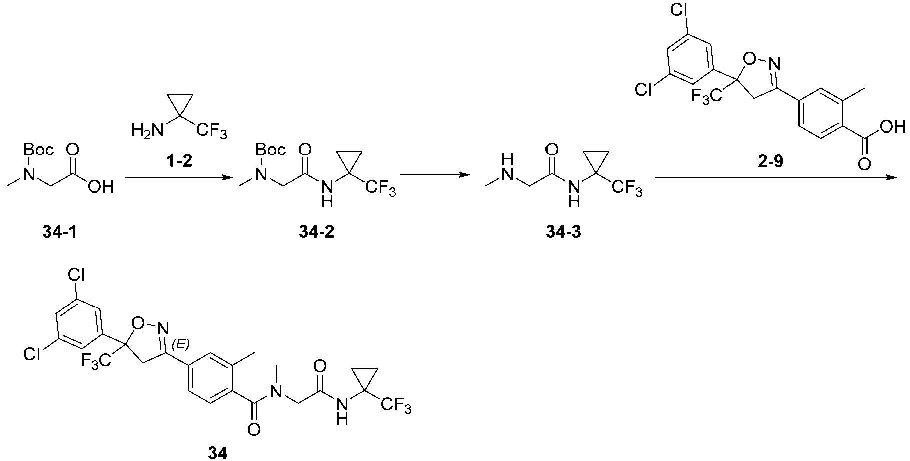

**Step 1:** Compound **34-1** (1.00 g, 5.28 mmol, 1 eq.) was dissolved in DMF (10 mL), added with HATU (2.41 g, 6.34 mmol, 1.2 eq.) and DIPEA (2.05 g, 15.8 mmol, 3 eq.), and stirred at 25°C for 0.5 h. Then, a hydrochloride of Compound 1-2 (939 mg, 5.81 mmol, 1.1 eq.) was added and stirred at 25°C for 0.5 h. The reaction solution was poured into water (30 mL) and extracted with ethyl acetate (30 mL × 3). Organic phases were washed with saturated brine (30 mL × 2), dried over anhydrous magnesium sulfate, filtered, and concentrated under reduced pressure to obtain Compound **34-2** (1.56 g, crude product).

**Step 2:** Compound **34-2** (1.56 g, 5.26 mmol, 1 eq.) was dissolved in DCM (8 mL), added with a dioxane solution of hydrogen chloride (4 M, 8 mL), and stirred at 25°C for 2 h. The reaction mixture was concentrated under reduced pressure to obtain a crude product. The crude product was triturated and purified with ethyl acetate (10 mL) to obtain a hydrochloride of Compound **34-3** (950 mg with a yield of 77%). ¹H NMR (400 MHz, DMSO-*d*₆) δ 9.45 (s, 1H), 9.07 (s, 2H), 3.71 (s, 2H), 2.53 (s, 3H), 1.32-1.25 (m, 2H), 1.13-0.96 (m, 2H).

**Step 3:** Compound **2-9** (70 mg, 167 µmol, 1 eq.) was dissolved in DMF (1 mL), added with HATU (76 mg, 200 µmol, 1.2 eq.) and DIPEA (64 mg, 502 µmol, 3 eq.), and stirred at 25°C for 0.5 h. Then, the hydrochloride of Compound **34-3** (43 mg, 184 µmol, 1.1 eq.) was added and stirred at 25°C for 0.5 h. The reaction solution was poured into water (10 mL) and extracted with ethyl acetate (10 mL × 3). Organic phases were washed with saturated brine (10 mL × 2), dried over anhydrous magnesium sulfate, filtered, and concentrated under reduced pressure. The crude product was purified through preparative thin-layer chromatography (SiO₂, ethyl acetate) to obtain Compound **34** (40.3 mg with a yield of 40%). ¹H NMR (400 MHz, CDCl₃) δ 7.61-7.51 (m, 4H), 7.45 (t, *J =* 1.6 Hz, 1H), 7.27 (d, *J =* 8.0 Hz, 1H), 7.01 (s, 1H), 4.21-4.04 (m, 3H), 3.72 (d, *J* = 17.6 Hz, 1H), 2.95 (s, 3H), 2.37 (s, 3H), 1.41-1.34 (m, 2H), 1.22-1.14 (m, 2H). MS-ESI: calcd for [M+H]⁺ 596, found 596.

### Example 035 Preparation of 4-(5-(3,5-dichlorophenyl)-5-(trifluoromethyl)-4,5-dihydroisoxazol-3-yl)-2-methyl-N-((R)-1-oxo-1-((1-(trifluoromethyl)cyclopropyl)amino)propan-2-yl)benzamide (35)

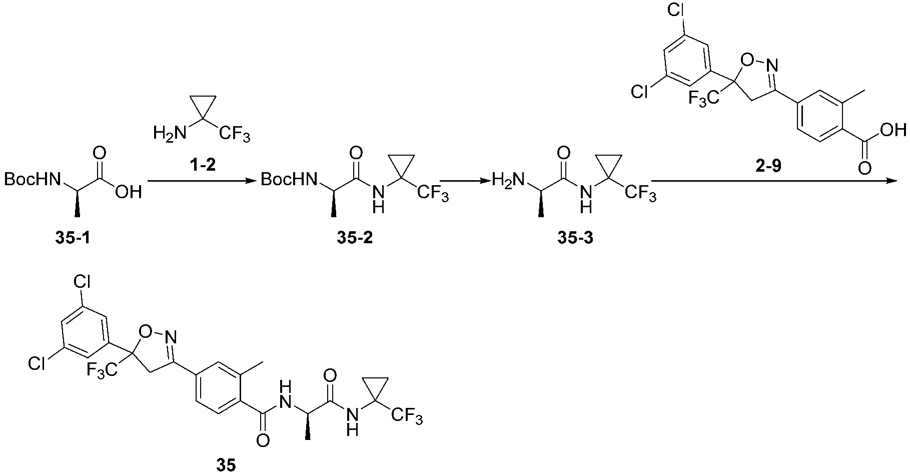

**Step 1:** HATU (2.41 g, 6.34 mmol, 1.2 eq.) and DIPEA (2.04 g, 15.8 mmol, 3 eq.) were added to a solution of Compound **35-1** (1.00 g, 5.28 mmol, 1 eq.) in DMF (10 mL) and stirred at 25°C for 0.5 h. Then, a hydrochloride of Compound **1-2** (939 mg, 5.81 mmol, 1.1 eq.) was added to the mixture and stirred at 25°C for 0.5 h. The reaction solution was poured into water (50 mL) and extracted with ethyl acetate (50 mL × 3). Organic phases were washed with saturated brine (50 mL × 2), dried over anhydrous magnesium sulfate, filtered, and concentrated under reduced pressure to obtain Compound **35-2** (2.0 g, crude product).

**Step 2:** Compound **35-2** (2.00 g, 6.75 mmol, 1 eq.) was dissolved in DCM (10 mL), added with a dioxane solution of hydrogen chloride (4 M, 10 mL), and stirred at 25°C for 2 h. The reaction mixture was concentrated under reduced pressure to obtain a crude product. The crude product was triturated with ethyl acetate (10 mL) to obtain a hydrochloride of Compound **35-3** (950 mg with a yield of 60%). ¹H NMR (400 MHz, DMSO-*d*₆) δ 9.37 (s, 1H), 8.29 (s, 3H), 3.80 (q, *J* = 7.2 Hz, 1H), 1.35 (d, *J* = 7.2 Hz, 3H), 1.32-1.22 (m, 2H), 1.14-0.97 (m, 2H).

**Step 3:** Compound **2-9** (70 mg, 167 µmol, 1 eq.) was dissolved in DMF (1 mL), added with HATU (76 mg, 200 µmol, 1.2 eq.) and DIPEA (64 mg, 502 µmol, 3 eq.), and stirred at 25°C for 0.5 h. Then, the hydrochloride of Compound **35-3** (42 mg, 184 µmol, 1.1 eq.) was added and stirred at 25°C for 0.5 h. The reaction solution was poured into water (5 mL) and extracted with ethyl acetate (5 mL × 3). Organic phases were washed with saturated brine (5 mL × 2), dried over anhydrous magnesium sulfate, filtered, and concentrated under reduced pressure. The crude product was purified through preparative thin-layer chromatography (SiO₂, n-heptane/ethyl acetate = 1:1) to obtain Compound **35** (41.8 mg with a yield of 41%). ¹H NMR (400 MHz, CDCl₃) δ 7.59-7.50 (m, 4H), 7.49-7.38 (m, 2H), 7.10 (s, 1H), 6.48 (dd, *J* = 3.2, 7.2 Hz, 1H), 4.68 (t, *J* = 7.2 Hz, 1H), 4.11 (dd, *J =* 1.2, 17.2 Hz, 1H), 3.72 (d, *J =* 17.6 Hz, 1H), 2.47 (s, 3H), 1.50 (d, *J* = 7.2 Hz, 3H), 1.39-1.31 (m, 2H), 1.14 (s, 2H). MS-ESI: calcd for [M+H]⁺ 569, found 596.

### Example 036 Preparation of 4-(5-(3,5-dichlorophenyl)-5-(trifluoromethyl)-4,5-dihydroisoxazol-3-yl)-2-methyl-N-((S)-1-oxo-1-((1-(trifluoromethyl)cyclopropyl)amino)propan-2-yl)benzamide (36)

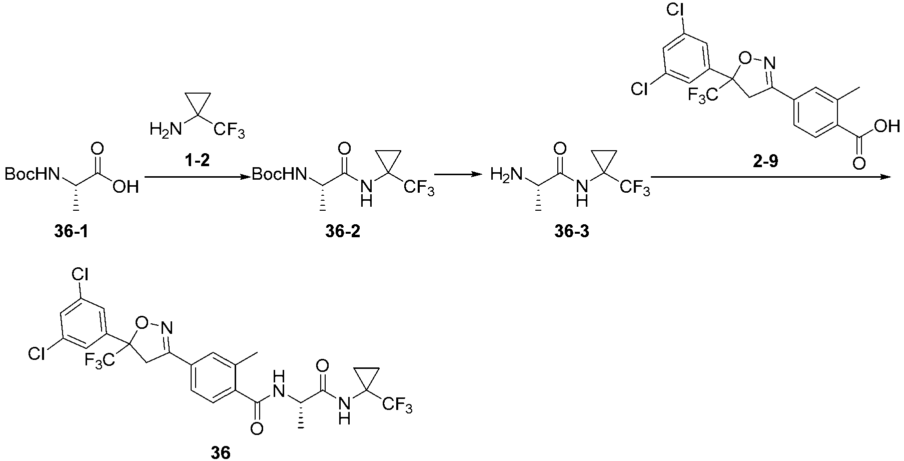

**Step 1:** Compound **36-1** (1.00 g, 5.28 mmol, 1 eq.) was dissolved in DMF (10 mL), added with HATU (2.41 g, 6.34 mmol, 1.2 eq.) and DIPEA (2.04 g, 15.8 mmol, 3 eq.), and stirred at 25°C for 0.5 h. Then, a hydrochloride of Compound 1-2 (939 mg, 5.81 mmol, 1.1 eq.) was added and stirred at 25°C for 0.5 h. The reaction solution was poured into water (50 mL) and extracted with ethyl acetate (50 mL × 3). Organic phases were washed with saturated brine (50 mL × 2), dried over anhydrous magnesium sulfate, filtered, and concentrated under reduced pressure to obtain Compound **36-2** (1.70 g, crude product).

**Step 2:** Compound **36-2** (1.70 g, 5.73 mmol, 1 eq.) was dissolved in DCM (10 mL), added with a dioxane solution of hydrogen chloride (4 M, 10 mL), and stirred at 25°C for 2 h. The reaction mixture was concentrated under reduced pressure to obtain a crude product. The crude product was triturated with ethyl acetate (10 mL) to obtain a hydrochloride of Compound 36-3 (830 mg with a yield of 62%). ¹H NMR (400 MHz, DMSO-*d*₆) δ 9.35 (s, 1H), 8.42-7.94 (m, 3H), 3.79 (d, *J* = 6.8 Hz, 1H), 1.34 (d, *J* = 6.8 Hz, 3H), 1.32-1.23 (m, 2H), 1.13-0.97 (m, 2H).

**Step 3:** Compound **2-9** (70 mg, 167 µmol, 1 eq.) was dissolved in DMF (1 mL), added with HATU (76.3 mg, 200 µmol, 1.2 eq.) and DIPEA (64 mg, 502 µmol, 3 eq.), and stirred at 25°C for 0.5 h. Then, Compound **36-3** (42 mg, 184 µmol, 1.1 eq.) was added and stirred at 25°C for 0.5 h. The reaction solution was poured into water (5 mL) and extracted with ethyl acetate (5 mL × 3). Organic phases were washed with saturated brine (5 mL × 2), dried over anhydrous magnesium sulfate, filtered, and concentrated under reduced pressure. The crude product was purified through preparative thin-layer chromatography (SiO₂, n-heptane/ethyl acetate = 1:1) to obtain Compound **36** (33.4 mg with a yield of 33%). ¹H NMR (400 MHz, CDCl₃) δ 7.58-7.48 (m, 4H), 7.47-7.37 (m, 2H), 7.22 (s, 1H), 6.57 (t, *J =* 6.8 Hz, 1H), 4.70 (q, *J =* 7.2 Hz, 1H), 4.11 (dd, *J* = 1.6, 17.2 Hz, 1H), 3.72 (d, *J* = 17.6 Hz, 1H), 2.46 (s, 3H), 1.50 (d, *J* = 7.2 Hz, 3H), 1.38-1.31 (m, 2H), 1.13 (s, 2H). MS-ESI: calcd for [M+H]⁺ 596, found 596.

### Example 037 Preparation of 4-(5-(3-chloro-5-(trifluoromethoxy)phenyl)-5-(trifluoromethyl)-4,5-dihydroisoxazol-3-yl)-2-met hyl-N-(2-oxo-2-((1-(trifluoromethyl)cyclopropyl)amino)ethyl)benzamide (37)

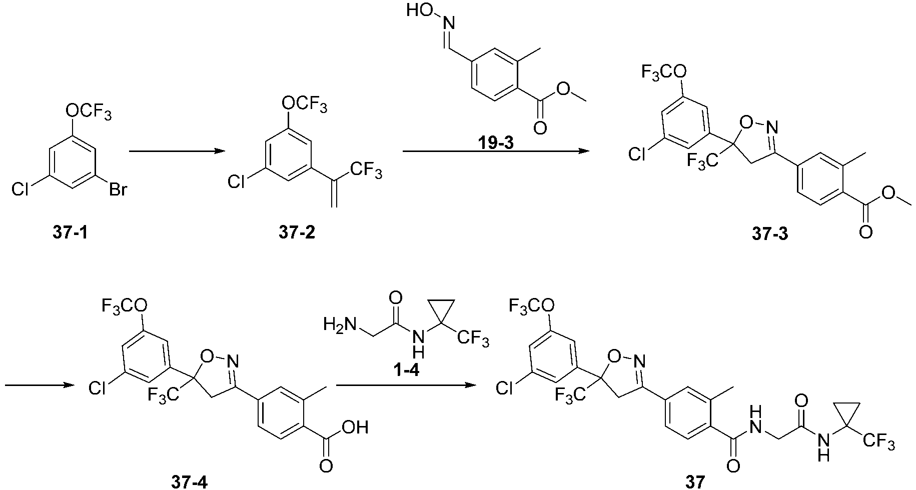

**Step 1:** Compound **37-1** (2.00 g, 7.26 mmol, 1 eq.) and 1-(trifluoromethyl)vinyl boronic acid hexylene glycol ester (1.93 g, 8.71 mmol, 1.2 eq.) were dissolved in dioxane (10 mL) and H₂O (2 mL), added with Pd(dppf)Cl₂ (531 mg, 726 µmol, 0.1 eq.) and K₂CO₃ (3.01 g, 21.7 mmol, 3 eq.), and stirred at 95°C for 16 h. The reaction solution was concentrated under reduced pressure to obtain a crude product. The crude product was separated and purified through column chromatography (SiO₂, n-heptane) to obtain Compound 37-3 (1.56 g with a yield of 73%). ¹H NMR (400 MHz, CDCl₃) δ 7.41 (s, 1H), 7.30 (s, 1H), 7.23 (s, 1H), 6.10 (s, 1H), 5.87 (d, *J =* 1.6Hz, 1H).

**Step 2:** Compound **19-3** (302 mg, 1.56 mmol, 1 eq.) and NCS (208 mg, 1.56 mmol, 1 eq.) were dissolved in DMF (5 mL) and stirred at 25°C for 2 h, and Compound **37-2** (500 mg, 1.72 mmol, 1.1 eq.) and triethylamine (189 mg, 1.87 mmol, 1.2 eq.) were added and stirred at 25°C for 2 h. The reaction solution was poured into water (10 mL) and extracted with ethyl acetate (10 mL × 3). Organic phases were washed with saturated brine (10 mL × 2), dried over anhydrous magnesium sulfate, filtered, and concentrated under reduced pressure. The crude product was separated and purified through column chromatography (SiO₂, ethyl acetate/n-heptane, 0-6%) to obtain Compound **37-3** (170 mg with a yield of 22%). ¹H NMR (400 MHz, CDCl₃) δ 7.98 (d, *J =* 8.8 Hz, 1H), 7.62-7.53 (m, 3H), 7.42 (s, 1H), 7.34 (d, *J =* 0.8 Hz, 1H), 4.14 (d, *J =* 17.2 Hz, 1H), 3.93 (s, 3H), 3.74 (d, *J =* 17.2 Hz, 1H), 2.65 (s, 3H).

**Step 3:** Compound **37-3** (170 mg, 352 µmol, 1 eq.) was dissolved in THF (2 mL) and H₂O (0.5 mL), added with LiOH.H₂O (44 mg, 1.05 mmol, 3 eq.), and stirred at 25°C for 16 h. The reaction mixture was adjusted to a pH of 5-7 with 1 N HCl and extracted with ethyl acetate (10 mL × 2). Organic phases were washed with brine (10 mL), dried over anhydrous magnesium sulfate, filtered, and concentrated under reduced pressure to obtain Compound **37-4** (160 mg, crude product).

**Step 4:** Compound **37-4** (100 mg, 213 µmol, 1 eq.) was dissolved in DMF (1 mL), added with HATU (98 mg, 257 µmol, 1.20 eq.) and DIPEA (83 mg, 642 µmol, 3.00 eq.), and stirred at 25°C for 0.5 h. Then, Compound **1-4** (51 mg, 233 µmol, 1.09 eq.) was added and stirred at 25°C for 0.5 h. The reaction solution was poured into water (10 mL) and extracted with ethyl acetate (10 mL × 3). Organic phases were washed with saturated brine (10 mL × 2), dried over anhydrous magnesium sulfate, filtered, and concentrated under reduced pressure. The crude product was separated and purified through preparative high-performance liquid chromatography (column: Xtimate C18, 30 × 150 mm I.D., 5 µm; mobile phase: A for H₂O (0.1% TFA) and B for CH₃CN; gradient: (63%-83%, 10 min, 100%, 2 min, 63%, 3 min)) to obtain Compound **37** (37.24 mg with a yield of 27%).¹H NMR (400 MHz, CDCl₃) δ 7.63-7.46 (m, 4H), 7.41 (s, 1H), 7.34 (s, 1H), 7.18 (s, 1H), 6.86 (t, *J =* 5.2 Hz, 1H), 4.21-4.04 (m, 3H), 3.73 (d, *J =* 17.6 Hz, 1H), 2.49 (s, 3H), 1.42-1.32 (m, 2H), 1.21-1.09 (m, 2H). MS-ESI: calcd for [M+H]⁺ 632, found 632.

### Example 038 Preparation of 4-(5-(3,5-dichlorophenyl)-5-(trifluoromethyl)-4,5-dihydroisoxazol-3-yl)-N-(2-oxo-2-((1-(trifluor omethyl)cyclopropyl)amino)ethyl)benzamide (38)

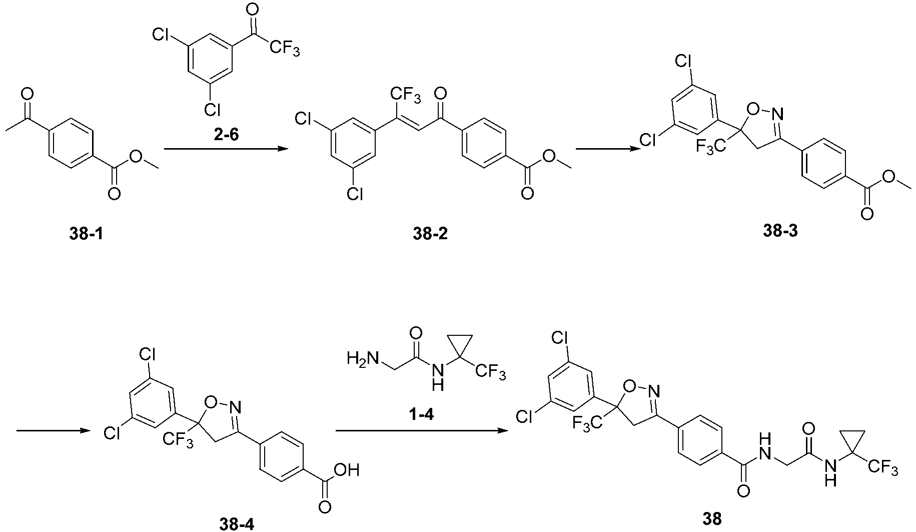

**Step 1:** Compound **38-1** (5.00 g, 28.1 mmol, 1 eq.) and Compound **2-6** (7.00 g, 28.8 mmol, 1 eq.) were dissolved in trifluoromethylbenzene (50 mL) and toluene (50 mL), added with cesium carbonate (914 mg, 2.81 mmol, 0.1 eq.), and stirred and reacted at 110°C for 12 h. The reaction was quenched with water (50 mL) and extracted with ethyl acetate (50 mL × 2). Organic phases were combined, washed with saturated brine (50 mL), dried over anhydrous magnesium sulfate, filtered, and concentrated under reduced pressure. The obtained crude product was purified through column chromatography (n-heptane:ethyl acetate = 20:1) to obtain Compound **38-2** (6.00 g with a yield of 53%). ¹H NMR (400 MHz, CDCl₃) δ 8.12 (d, *J =* 8.4 Hz, 2H), 7.88 (d, *J* = 8.4 Hz, 2H), 7.40 (d, *J =* 1.2 Hz, 1H), 7.33 (t, *J =* 1.6 Hz, 1H), 7.16 (d, *J =* 1.6 Hz, 2H), 3.96 (s, 3H).

**Step 2:** Sodium hydroxide (1.20 g, 29.8 mmol, 4 eq.), NH₂OH.HCl (3.10 g, 22.3 mmol, 3 eq.), and tetrabutylammonium bromide (1.16 g, 0.744 mmol, 0.1 eq.) were dissolved in water (50 mL) and stirred and reacted at 0°C for 0.5 h. Compound **38-2** (3.00 g, 7.44 mmol, 1 eq.) was dissolved in toluene (50 mL), added dropwise to the above reaction solution, and stirred and reacted at 25°C for 12 h. The reaction was quenched with a saturated aqueous ammonium chloride solution (50 mL) and extracted with ethyl acetate (50 mL × 3). Organic phases were combined, washed with saturated brine (50 mL × 2), dried over anhydrous magnesium sulfate, filtered, and concentrated under reduced pressure. The obtained crude product was purified through column chromatography (n-heptane:ethyl acetate = 9:1) to obtain Compound **38-3** (200 mg with a yield of 6%). ¹H NMR (400 MHz, CDCl₃) δ 8.10 (d, *J =* 8.4 Hz, 2H), 7.74 (d, *J =* 8.4 Hz, 2H), 7.52 (s, 2H), 7.44 (d, *J =* 1.6 Hz, 1H), 3.99-3.93 (m, 4H), 3.73 (d, *J =* 17.2 Hz, 1H).

**Step 3:** Compound **38-3** (200 mg, 0.478 mmol, 1 eq.) was dissolved in THF (1.5 mL), methanol (1 mL), and water (0.5 mL), added with lithium hydroxide monohydrate (60.2 mg, 1.44 mmol, 3 eq.), and stirred and reacted at 25°C for 6 h. The reaction solution was adjusted to a pH of about 3 with 1 N HCl and extracted with ethyl acetate (10 mL × 2). Organic phases were combined, washed with saturated brine (10 mL × 2), dried over anhydrous magnesium sulfate, filtered, and concentrated under reduced pressure to obtain Compound **38-4** (190 mg, crude product).

**Step 4:** Compound **38-4** (200 mg, 0.495 mmol, 1 eq.) and a hydrochloride of Compound **1-4** (162 mg, 0.742 mmol, 1.5 eq.) were dissolved in DMF (3 mL), added with HATU (282 mg, 0.742 mmol, 1.5 eq.) and DIPEA (192 mg, 1.49 mmol, 3 eq.), and stirred and reacted at 25°C for 2 h. The reaction was quenched with water (10 mL) and extracted with ethyl acetate (10 mL × 3). Organic phases were combined, washed with saturated brine (10 mL × 2), dried over anhydrous magnesium sulfate, filtered, and concentrated under reduced pressure. The obtained crude product was purified through preparative thin-layer chromatography (ethyl acetate) to obtain Compound **38** (37.5 mg with a yield of 13%). ¹H NMR (400 MHz, CDCl₃) δ 7.88 (d, *J =* 8.4 Hz, 2H), 7.72 (d, *J =* 8.4 Hz, 2H), 7.67-7.61 (m, 2H), 7.52 (d, *J =* 1.6 Hz, 2H), 7.44 (t, *J* = 1.6 Hz, 1H), 4.19 (d, *J =* 4.8 Hz, 2H), 4.11 (d*, J =* 17.2 Hz, 1H), 3.73 (d, *J =* 17.2 Hz, 1H), 1.37-1.31 (m, 2H), 1.16-1.10 (m, 2H). MS-ESI: calcd for [M+H]⁺ 568, found 568.

### Example 39 (Comparative Example 1) Preparation of N-(2-(cyclopropylamino)-2-oxoethyl)-4-(5-(3,5-dichlorophenyl)-5-(trifluoromethyl)-4,5-dihydro isoxazol-3-yl)-2-methylbenzamide

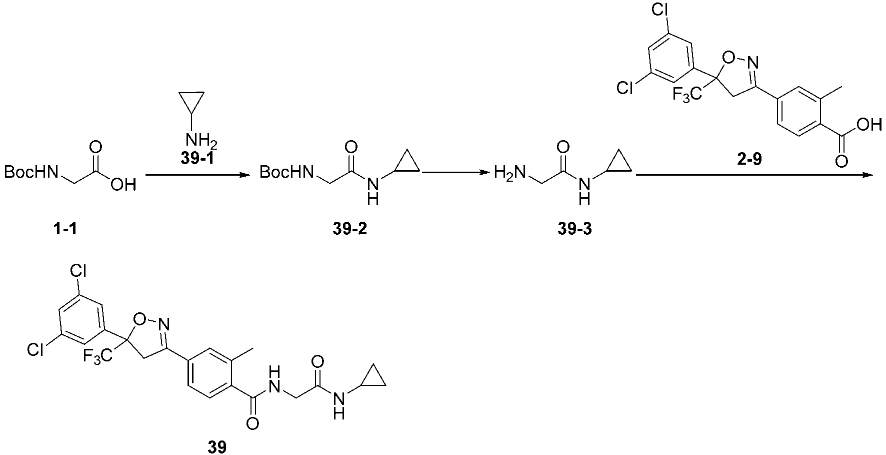

**Step 1:** Compound **1-1** (2.00 g, 11.4 mmol, 1.0 eq.) was dissolved in dichloromethane (50 mL), added with Compound 39-1 (978 mg, 17.1 mmol, 1.5 eq.), T₄P (12.3 g, 17.1 mmol, 50% ethyl acetate solution, 1.5 eq.), and DIPEA (5.90 g, 45.7 mmol, 4.0 eq.), and stirred at 25°C for 1 h. The system was added with water (5 mL) and ethyl acetate (5 mL) and separated into layers. The aqueous phase was extracted with ethyl acetate (5 mL × 2). Organic phases were combined, washed with water (5 mL × 3), washed with saturated brine (5 mL), dried over anhydrous magnesium sulfate, filtered, and concentrated under reduced pressure to obtain a crude product, Compound **39-2** (1.72 g with a yield of 70%).

**Step 2:** Compound **39-2** (1.70 g, 7.93 mmol, 1.0 eq.) was dissolved in DCM (2 mL), added with a dioxane solution of hydrogen chloride (4 M, 10 mL), and stirred at 25°C for 16 h. After the reaction was completed, methyl *tert*-butyl ether (5 mL) was added, stirred for 1 h, and filtered to obtain a hydrochloride of Compound **39-3** (920 mg with a yield of 85%).

**Step 3:** Compound **2-9** (70.0 mg, 167 µmol, 1.0 eq.), the hydrochloride of Compound **39-3** (37.8 mg, 251 µmol, 1.5 eq.), HATU (95.5 mg, 251 µmol, 1.5 eq.), and DIPEA (86.5 mg, 670 µmol, 4.0 eq.) were dissolved in DMF (2 mL) and reacted at 20°C for 1 h. The system was added with water (1 mL) and ethyl acetate (1 mL) and separated into layers. The aqueous phase was extracted with ethyl acetate (1 mL × 2). Organic phases were combined, washed with water (1 mL), washed with saturated brine (1 mL), dried over anhydrous magnesium sulfate, filtered, and concentrated under reduced pressure. The crude product was purified though preparative thin-layer chromatography (n-heptane:ethyl acetate = 1:20) to obtain Compound **39** (35 mg with a yield of 41%). ¹H NMR (400 MHz, DMSO-*d₆*) δ 8.49 (t, *J =* 6.0 Hz, 1H), 8.02 (d, *J =* 4.0 Hz, 1H), 7.83 (t, *J =* 1.6 Hz, 1H), 7.67-7.57 (m, 4H), 7.48 (d, *J =* 8.4 Hz, 1H), 4.50-4.23 (m, 2H), 3.78 (d, *J =* 6.0 Hz, 2H), 2.70-2.60 (m, 1H), 2.40 (s, 3H), 0.71-0.56 (m, 2H), 0.48-0.37 (m, 2H). MS-ESI: calcd for [M+H]⁺ 514, found 514.

### Example 040 Preparation of 2-chloro-N-(2-(cyclopropylamino)-2-oxoethyl)-4-(5-(3,5-dichlorophenyl)-5-(trifluoromethyl)-4, 5-dihydroisoxazol-3-yl)-6-methylbenzamide (40)

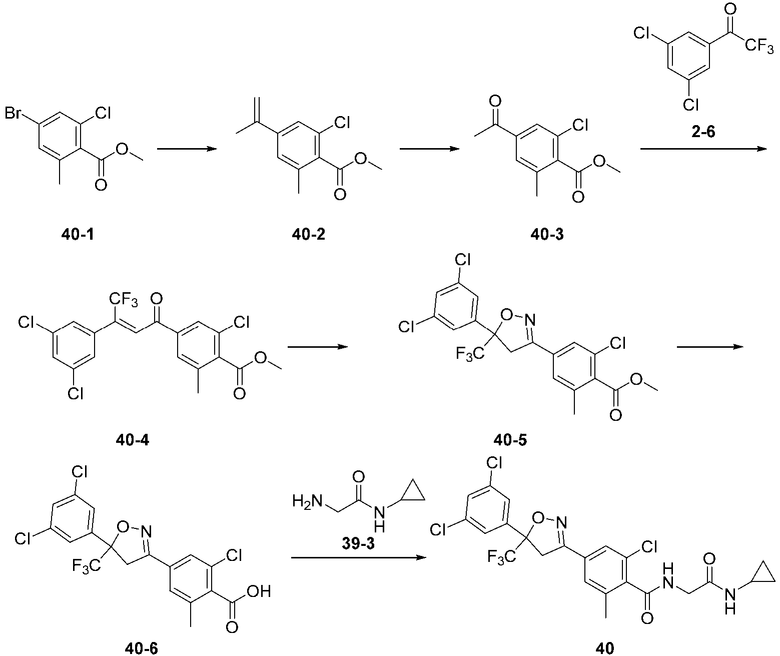

**Step 1:** Compound **40-1** (4.00 g, 15.2 mmol, 1 eq.) and potassium isopropenyltrifluoroborate (2.46 g, 16.6 mmol, 1.1 eq.) were dissolved in dioxane (20 mL) and H₂O (10 mL), added with K₂CO₃ (6.28 g, 45.4 mmol, 2.99 eq.) and Pd(dppf)Cl₂ (1.11 g, 1.52 mmol, 0.1 eq.), and stirred at 60°C for 20 h. The reaction solution was concentrated under reduced pressure, added with ethyl acetate (20 mL), and filtered. Organic phases were washed with saturated brine (10 mL × 1), dried over magnesium sulfate, filtered, and spin-dried. The crude product was separated and purified through column chromatography (SiO₂, ethyl acetate/n-heptane, 1-3%) to obtain Compound **40-2** (2.7 g with a yield of 79%).

**Step 2:** Compound **40-2** (2.70 g, 12.0 mmol, 1 eq.) was dissolved in THF (20 mL) and H₂O (10 mL), added with potassium osmate (374 mg, 1.20 mmol, 0.1 eq.) and sodium periodate (5.91 g, 27.6 mmol, 2.30 eq.), and stirred at 20°C for 20 h. The system was added with an aqueous solution of sodium thiosulfate (20 mL) and separated into layers. The aqueous phase was extracted with ethyl acetate (10 mL × 2). Organic phases were combined, washed with water (10 mL × 2), washed with saturated brine (10 mL × 2), dried over magnesium sulfate, filtered, and concentrated under reduced pressure. The crude product was separated and purified through column chromatography (ethyl acetate/n-heptane, 1-3%) to obtain Compound **40-3** (1.05 g with a yield of 39%).

**Step 3:** Compound **40-3** (1.05 g, 4.63 mmol, 1 eq.) and Compound 2-6 (1.69 g, 6.95 mmol, 1.5 eq.) were dissolved in toluene (10 mL), added with Cs₂CO₃ (302 mg, 927 µmol, 0.2 eq.), and stirred at 110°C for 16 h. The reaction solution was concentrated under reduced pressure, added with ethyl acetate (15 mL), filtered, and concentrated under reduced pressure. The crude product was separated and purified through column chromatography (SiO₂, ethyl acetate/n-heptane, 1-3%) to obtain Compound **40-4** (1.45 g with a yield of 69%).

**Step 4:** NH₂OH·HCl (558 mg, 8.03 mmol, 2.50 eq.), tetrabutylammonium bromide (103 mg, 320 µmol, 0.1 eq.), and NaOH (385 mg, 9.63 mmol, 3.0 eq.) were dissolved in H₂O (2 mL), added with a solution of Compound **40-4** (1.45 g, 3.21 mmol, 1 eq.) in THF (10 mL), and stirred at 20°C for 15.5 h. After the reaction was completed, the reaction was quenched with NH₄Cl (20 mL) and extracted with ethyl acetate (25 mL × 2). Organic phases were combined, washed with saturated brine (25 mL × 2), dried over magnesium sulfate, filtered, and concentrated under reduced pressure. The crude product was subjected to column chromatography (ethyl acetate/n-heptane, 2-5%) to obtain Compound **40-5** (1.35 g with a yield of 90%).

**Step 5:** Compound **40-5** (200 mg, 429 µmol, 1 eq.) was dissolved in THF (2 mL) and H₂O (2 mL), added with LiOH.H₂O (36 mg, 858 µmol, 2.00 eq.), and stirred at 70°C for 16 h. The reaction solution was concentrated under reduced pressure. The aqueous phase was adjusted to a pH of 3-4 with a 1 mol/L hydrochloric acid aqueous solution and extracted with ethyl acetate (10 mL × 2). Organic phases were washed with saturated brine (10 mL × 2), dried over magnesium sulfate, filtered, and concentrated under reduced pressure to obtain Compound **40-6** (190 mg with a yield of 98%). MS-ESI: calcd for [M+H]⁺ 452, found 452.

**Step 6:** Compound **40-6** (85 mg, 188 µmol, 1 eq.) was dissolved in DMF (1 mL), added with HATU (107 mg, 281 µmol, 1.5 eq.) and DIPEA (97 mg, 751 µmol, 4.0 eq.), and stirred at 25°C for 0.5 h. Then, a hydrochloride of Compound 39-3 (43 mg, 286 µmol, 1.52 eq.) was added and stirred at 20°C for 1 h. The reaction was quenched with water (10 mL) and extracted with ethyl acetate (10 mL × 2). Organic phases were combined, washed with water (10 mL × 2), washed with saturated brine (10 mL × 2), dried over magnesium sulfate, filtered, and concentrated under reduced pressure. The crude product was purified through preparative thin-layer chromatography (SiO₂, n-heptane/ethyl acetate = 1:6) to obtain Compound **40** (15 mg with a yield of 15%). ¹H NMR (400 MHz, DMSO-*d*₆ ) δ 8.76 (t, *J =* 5.9 Hz, 1H), 7.99 (d, *J* = 3.9 Hz, 1H), 7.83 (t, *J =* 1.8 Hz, 1H), 7.65-7.54 (m, 4H), 4.47-4.29 (m, 2H), 3.81 (d, *J =* 5.9 Hz, 2H), 2.75-2.57 (m, 1H), 2.37 (s, 3H), 0.69-0.57 (m, 2H), 0.50-0.33 (m, 2H). MS-ESI: calcd for [M+H]⁺ 548, found 548.

### Example 041 Preparation of N-(2-(cyclopropylamino)-2-oxoethyl)-4-(5-(3,5-dichlorophenyl)-5-(trifluoromethyl)-4,5-dihydro isoxazol-3-yl)-5-fluoro-2-methylbenzamide (41)

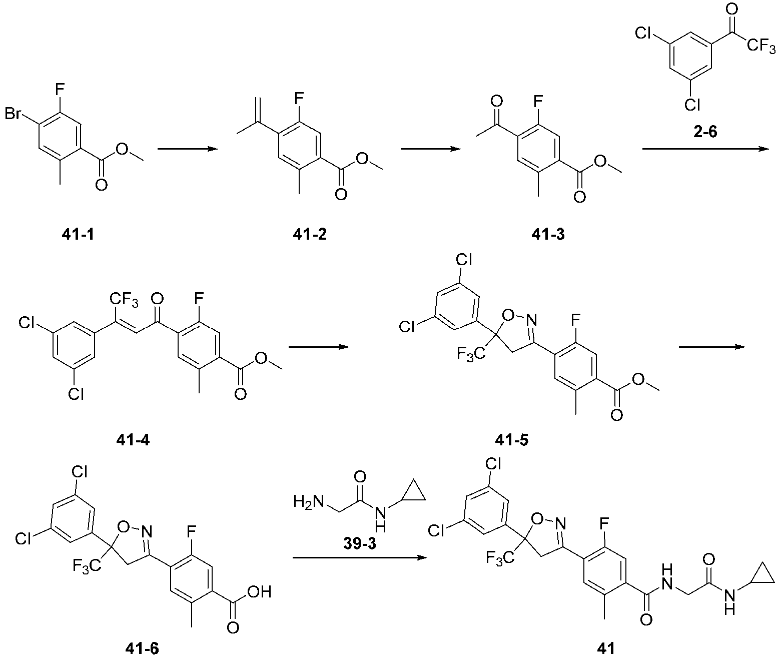

**Step 1:** Compound **41-1** (1.00 g, 4.05 mmol, 1 eq.) and potassium isopropenyltrifluoroborate (660 mg, 4.46 mmol, 1.10 eq.) were dissolved in dioxane (10 mL) and H₂O (5 mL), added with K₂CO₃ (1.68 g, 12.2 mmol, 3.00 eq.) and Pd(dppf)Cl₂ (296 mg, 405 µmol, 0.1 eq.), and stirred at 80°C for 20 h. The reaction solution was concentrated under reduced pressure, added with ethyl acetate (20 mL), and filtered. Organic phases were washed with saturated brine (10 mL × 1), dried over magnesium sulfate, filtered, and spin-dried. The crude product was separated and purified through column chromatography (SiO₂, ethyl acetate/n-heptane, 1-5%) to obtain Compound **41-2** (0.7 g with a yield of 83%).

**Step 2:** Compound **41-2** (700 mg, 3.36 mmol, 1 eq.) was dissolved in THF (7 mL) and H₂O (7 mL), added with potassium osmate (105 mg, 337 µmol, 0.1 eq.) and sodium periodate (1.65 g, 7.71 mmol, 2.30 eq.), and stirred at 20°C for 20 h. The system was added with an aqueous solution of sodium thiosulfate (20 mL) and separated into layers. The aqueous phase was extracted with ethyl acetate (10 mL × 2). Organic phases were combined, washed with water (10 mL × 2), washed with saturated brine (10 mL × 2), dried over magnesium sulfate, filtered, and concentrated under reduced pressure. The crude product was separated and purified through column chromatography (ethyl acetate/n-heptane, 3-5%) to obtain Compound **41-3** (0.4 g with a yield of 57%).

**Step 3:** Compound **41-3** (300 mg, 1.43 mmol, 1 eq.) and Compound **2-6** (522 mg, 2.15 mmol, 1.5 eq.) were dissolved in toluene (20 mL), added with Cs₂CO₃ (465 mg, 1.43 mmol, 1.0 eq.), and stirred at 110°C for 16 h. The reaction solution was concentrated under reduced pressure, added with ethyl acetate (35 mL), washed with saturated brine (15 mL × 2), dried over magnesium sulfate, filtered, and concentrated under reduced pressure. The crude product was separated and purified through column chromatography (SiO₂, ethyl acetate/n-heptane, 0-3%) to obtain Compound **41-4** (195 mg with a yield of 31%).

**Step 4:** NH₂OH·HCl (88 mg, 1.27 mmol, 2.50 eq.), tetrabutylammonium bromide (17 mg, 52.7 µmol, 0.1 eq.), and NaOH (61 mg, 1.53 mmol, 3.0 eq.) were dissolved in H₂O (1 mL), added with a solution of Compound **41-4** (220 mg, 506 µmol, 1 eq.) in THF (5 mL), and stirred at 20°C for 15.5 h. After the reaction was completed, the reaction was quenched with NH₄Cl (20 mL) and extracted with ethyl acetate (25 mL × 2). Organic phases were combined, washed with saturated brine (25 mL × 2), dried over magnesium sulfate, filtered, and concentrated under reduced pressure. The crude product was subjected to column chromatography (ethyl acetate/n-heptane, 2-10%) to obtain Compound **41-5** (185 mg with a yield of 81%).

**Step 5:** Compound **41-5** (200 mg, 444 µmol, 1 eq.) was dissolved in THF (5 mL) and H₂O (5 mL), added with LiOH.H₂O (53 mg, 1.33 mmol, 3.00 eq.), and stirred at 20°C for 16 h. The reaction solution was concentrated under reduced pressure. The aqueous phase was adjusted to a pH of 3-4 with a 1 mol/L hydrochloric acid aqueous solution and extracted with ethyl acetate (10 mL × 2). Organic phases were washed with saturated brine (10 mL × 2), dried over magnesium sulfate, filtered, and concentrated under reduced pressure to obtain Compound **41-6** (180 mg with a yield of 93%).

**Step 6:** Compound **41-6** (70 mg, 160 µmol, 1 eq.) was dissolved in DMF (1 mL) and added with HATU (92 mg, 242 µmol, 1.5 eq.) and DIPEA (83 mg, 642 µmol, 4.0 eq.). Then, a hydrochloride of Compound **39-3** (36 mg, 239 µmol, 1.5 eq.) was added and stirred at 20°C for 1 h. The reaction was quenched with water (10 mL) and extracted with ethyl acetate (10 mL × 2). Organic phases were combined, washed with water (10 mL × 2), washed with saturated brine (10 mL × 2), dried over magnesium sulfate, filtered, and concentrated under reduced pressure. The crude product was purified through preparative thin-layer chromatography (SiO₂, n-heptane/ethyl acetate = 1:4) to obtain Compound **41** (5 mg with a yield of 6%). ¹H NMR (400 MHz, CD₃OD) *δ* 7.72 (d, *J* = 7.1 Hz, 1H), 7.67-7.56 (m, 3H), 7.38 (d, *J* = 11.0 Hz, 1H), 4.44-4.24 (m, 1H), 4.13-3.91 (m, 3H), 2.82-2.60 (m, 1H), 2.44 (s, 3H), 0.85-0.65 (m, 2H), 0.60-0.47 (m, 2H). MS-ESI: calcd for [M+H]⁺ 532, found 532.

### Example 042 Preparation of 4-(5-(3,5-dichlorophenyl)-5-(trifluoromethyl)-4,5-dihydroisoxazol-3-yl)-5-fluoro-2-methyl-N-(2-oxo-2-(2,2,2-trifluoroethyl)amino)ethyl)benzamide (42)

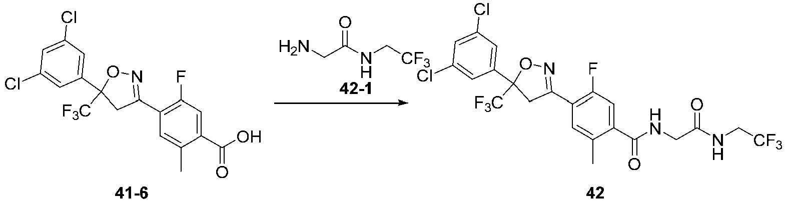

**Step 1:** Compound **41-6** (70 mg, 160 µmol, 1 eq.) was dissolved in DMF (1 mL) and added with HATU (92 mg, 242 µmol, 1.5 eq.) and DIPEA (83 mg, 642 µmol, 4.0 eq.). Then, a hydrochloride of Compound **42-1** (46 mg, 239 µmol, 1.5 eq.) was added and stirred at 20°C for 1 h. The reaction was quenched with water (10 mL) and extracted with ethyl acetate (10 mL × 2). Organic phases were combined, washed with water (10 mL × 2), washed with saturated brine (10 mL × 2), dried over magnesium sulfate, filtered, and concentrated under reduced pressure. The crude product was purified through preparative thin-layer chromatography (SiO₂, n-heptane/ethyl acetate = 1:2) to obtain Compound **42** (40 mg with a yield of 43%). ¹H NMR (400 MHz, CDCl₃) *δ* 7.79 (d, *J* = 7.1 Hz, 1H), 7.59-7.37 (m, 3H), 7.24 (d, *J* = 10.8 Hz, 1H), 7.06-6.70 (m, 2H), 4.34-4.11 (m, 3H), 4.02-3.90 (m, 2H), 3.79 (d, *J* = 17.4 Hz, 1H), 2.44 (s, 3H). MS-ESI: calcd for [M+H]⁺ 574, found 574.

### Example 043 Preparation of N-(2-(cyclopropylamino)-2-oxoethyl)-4-(5-(3,5-dichlorophenyl)-5-(trifluoromethyl)-4,5-dihydro isoxazol-3-yl)-3-fluoro-2-methylbenzamide (43)

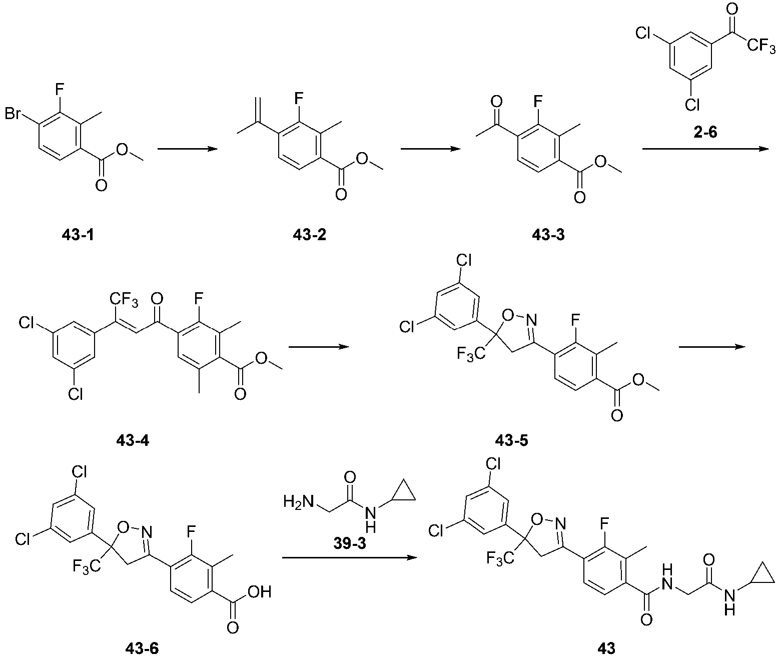

**Step 1:** Compound **43-1** (1.00 g, 4.05 mmol, 1 eq.) and potassium isopropenyltrifluoroborate (899 mg, 6.08 mmol, 1.5 eq.) were dissolved in dioxane (10 mL) and H₂O (2 mL), added with K₂CO₃ (1.68 g, 12.2 mmol, 3.00 eq.) and Pd(dppf)Cl₂ (296 mg, 405 µmol, 0.1 eq.), and stirred at 90°C for 2 h. The reaction solution was poured into water (50 mL) and extracted with ethyl acetate (50 mL × 3). Organic phases were washed with saturated brine (50 mL × 2), dried over anhydrous MgSO₄, filtered, and concentrated under reduced pressure. The crude product was separated and purified through column chromatography (SiO₂, ethyl acetate/n-heptane, 0-4%) to obtain Compound **43-2** (758 mg with a yield of 90%).

**Step 2:** Compound **43-2** (750 mg, 3.60 mmol, 1 eq.) was dissolved in THF (20 mL) and H₂O (5 mL), added with potassium osmate (112 mg, 360 µmol, 0.1 eq.) and sodium periodate (2.31 g, 10.8 mmol, 3.0 eq.), and stirred at 25°C for 16 h. The reaction was quenched with an aqueous solution of sodium thiosulfate (20 mL), added with water (30 mL), and extracted with ethyl acetate (30 mL × 3). Organic phases were combined, washed with brine (30 mL), dried over magnesium sulfate, and concentrated under reduced pressure. The crude product was separated and purified through column chromatography (ethyl acetate/n-heptane, 0-5%) to obtain Compound **43-3** (608 mg with a yield of 80%).

**Step 3:** Compound **43-3** (600 mg, 2.85 mmol, 1 eq.) and Compound 2-6 (833 mg, 3.43 mmol, 1.2 eq.) were dissolved in toluene (20 mL), added with Cs₂CO₃ (558 mg, 1.71 mmol, 0.6 eq.), and stirred at 110°C for 16 h. The reaction solution was poured into water (10 mL) and extracted with ethyl acetate (20 mL × 3). Organic phases were washed with saturated brine (20 mL × 2), dried over anhydrous MgSO₄, filtered, and concentrated under reduced pressure. The crude product was separated and purified through column chromatography (SiO₂, ethyl acetate/n-heptane, 0-6%) to obtain Compound **43-4** (200 mg with a yield of 16%).

**Step 4:** NH₂OH·HCl (96 mg, 1.38 mmol, 3.0 eq.), tetrabutylammonium bromide (15 mg, 46.5 µmol, 0.1 eq.), and NaOH (74 mg, 1.85 mmol, 4.0 eq.) were dissolved in H₂O (2 mL) and stirred at 0°C for 10 min. A solution of Compound **43-4** (200 mg, 460 µmol, 1 eq.) in THF (0.5 mL) was added and stirred at 25°C for 20 min. The reaction was quenched with a saturated ammonium chloride solution (10 mL), poured into water (10 mL), and extracted with ethyl acetate (10 mL × 3). Organic phases were washed with saturated brine (10 mL × 2), dried over anhydrous magnesium sulfate, filtered, and concentrated under reduced pressure. The crude product was subjected to column chromatography (ethyl acetate/n-heptane, 0-6%) to obtain Compound **43-5** (70 mg with a yield of 34%).

**Step 5:** Compound **43-5** (70 mg, 155 µmol, 1 eq.) was dissolved in THF (1 mL), methanol (0.5 mL), and H₂O (0.5 mL), added with LiOH.H₂O (20 mg, 477 µmol, 3.07 eq.), and stirred at 25°C for 16 h. The reaction mixture was adjusted to a pH of 5-7 with 1 N HCl and extracted with ethyl acetate (10 mL × 2). Organic phases were washed with brine (10 mL), dried over magnesium sulfate, filtered, and concentrated under reduced pressure to obtain a crude product, Compound **43-6** (70 mg).

**Step 6:** Compound **43-6** (35 mg, 80.2 µmol, 1 eq.) was dissolved in DMF (1 mL) and added with HATU (37 mg, 97.3 µmol, 1.21 eq.) and DIPEA (32 mg, 248 µmol, 3.09 eq.). Then, a hydrochloride of Compound **39-3** (15 mg, 99.6 µmol, 1.24 eq.) was added and stirred at 95°C for 16 h. The reaction solution was poured into water (10 mL) and extracted with ethyl acetate (10 mL × 3). Organic phases were washed with saturated brine (10 mL × 2), dried over anhydrous magnesium sulfate, filtered, and concentrated under reduced pressure. The crude product was purified through preparative thin-layer chromatography (SiO₂, n-heptane/ethyl acetate = 1:2) to obtain Compound **43** (10 mg with a yield of 23%). ¹H NMR (400 MHz, DMSO-*d*₆) δ 8.61 (t, *J =* 6.0 Hz, 1H), 8.05 (d, *J* = 3.9 Hz, 1H), 7.82 (t, *J =* 2.0 Hz, 1H), 7.70-7.62 (m, 3H), 7.31 (d, *J* = 8.1 Hz, 1H), 4.40-4.32 (m, 1H), 4.32-4.24 (m, 1H), 3.79 (d, *J* = 6.1 Hz, 2H), 2.71-2.60 (m, 1H), 2.31 (d, *J* = 2.4 Hz, 3H), 0.69-0.58 (m, 2H), 0.48-0.36 (m, 2H). MS-ESI: calcd for [M+H]⁺ 532, found 532.

### Example 044 Preparation of 4-(5-(3,5-dichlorophenyl)-5-(trifluoromethyl)-4,5-dihydroisoxazol-3-yl)-3-fluoro-2-methyl-N-(2-oxo-2-(2,2,2-trifluoroethyl)amino)ethyl)benzamide (44)

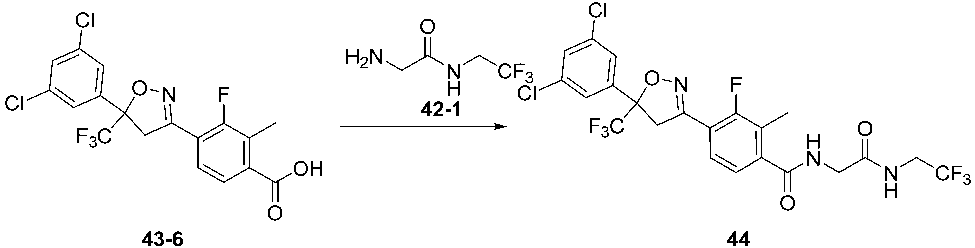

**Step 1:** Compound **43-6** (35 mg, 80.2 µmol, 1 eq.) was dissolved in DMF (1 mL) and added with HATU (37 mg, 97.3 µmol, 1.21 eq.) and DIPEA (32 mg, 248 µmol, 3.09 eq.). Then, a hydrochloride of Compound **42-1** (19 mg, 98.7 µmol, 1.23 eq.) was added and stirred at 25°C for 0.5 h. The reaction solution was poured into water (10 mL) and extracted with ethyl acetate (10 mL × 3). Organic phases were washed with saturated brine (10 mL × 2), dried over anhydrous magnesium sulfate, filtered, and concentrated under reduced pressure. The crude product was purified through preparative thin-layer chromatography (SiO₂, n-heptane/ethyl acetate = 1:2) to obtain Compound **44** (9.83 mg with a yield of 21%). ¹H NMR (400 MHz, DMSO-*d*₆) *δ* 8.75 (t, *J* = 6.0 Hz, 1H), 8.66 (t, *J =* 6.2 Hz, 1H), 7.82 (t, *J =* 1.8 Hz, 1H), 7.72-7.59 (m, 3H), 7.33 (d, *J =* 8.1 Hz, 1H), 4.43-4.22 (m, 2H), 4.04-3.87 (m, 4H), 2.31 (d, *J =* 2.4 Hz, 3H). MS-ESI: calcd for [M+H]⁺ 574, found 574.

### Biological Test and Evaluation

### Test Example 1 Mite killing efficacy test with an in vitro model

Mite collection: Live mites were gently picked up from a cage box with cotton swabs or small tweezers, 17-26 live mites per group.

Drug treatment and detection method: 100 µL of a drug solution with a different concentration (drug solution prepared by 3% DMSO solution + 97% water and having a target final concentration of 30 µM) was aspirated with a pipette and added to a 24-well plate for each group, ensuring full contact between the drug and the mites. The mites were separately incubated at normal temperature for 5 min, transferred from each plate to another dry 24-well plate with the top sealed, and air-dried for about 1 h. Mite actions were observed, and the number of dead mites/the total number of mites was recorded. Then, the 24-well plate containing the mites was cultured at room temperature and relatively high humidity. The number of dead mites/the total number of mites was counted separately after 1 h, 2 h, 4 h, 6 h, 8 h, 22 h, and 24 h (time points after drug addition), and mite mortality rates were calculated. The project ended 24 h after drug addition.

At a corresponding time point, a mite mortality rate greater than or equal to 90% is denoted as "+++", a mite mortality rate less than 90% and greater than or equal to 50% is denoted as "++", and a mite mortality rate less than 50% is denoted as "+". Table 1 lists the experimental results.

**Table 1**

| Example No. | Mite Mortality Rate | | Example No. | Mite Mortality Rate | |
|---|---|---|---|---|---|
| | 4 h | 24 h | | 4 h | 24 h |
| Example 001 | + | +++ | Example 024 | + | +++ |
| Example 002 | + | +++ | Example 025 | + | + |
| Example 003 | + | +++ | Example 026 | ++ | +++ |
| Example 004 | + | +++ | Example 027 | ++ | +++ |
| Example 005 | ++ | +++ | Example 028 | ++ | +++ |
| Example 006 | + | +++ | Example 029 | +++ | +++ |
| Example 007 | ++ | +++ | Example 030 | +++ | +++ |
| Example 008 | +++ | +++ | Example 031 | +++ | +++ |
| Example 009 | + | + | Example 032 | + | +++ |
| Example 010 | ++ | +++ | Example 033 | + | +++ |
| Example 011 | +++ | +++ | Example 034 | + | +++ |
| Example 012 | + | + | Example 035 | + | ++ |
| Example 013 | +++ | +++ | Example 036 | + | + |
| Example 014 | + | + | Example 037 | ++ | +++ |
| Example 015 | + | + | Example 038 | + | +++ |
| Example 016 | + | + | Example 39 (Comparative Example 1) | +++ | +++ |
| Example 017 | + | ++ | Example 040 | + | + |
| Example 018 | + | ++ | Example 041 | +++ | +++ |
| Example 019 | ++ | +++ | Example 042 | +++ | +++ |
| Example 020 | + | ++ | Example 043 | + | ++ |
| Example 021 | + | + | Example 044 | +++ | +++ |
| Example 022 | + | ++ | Fluralaner | +++ | +++ |
| Example 023 | + | ++ | | | |

### Test Example 2 Fruit fly killing efficacy test with an in vitro model

1. Drug preparation: Test samples were prepared in a vehicle of 3% DMSO + 97% water to obtain solutions with final concentrations of 30 µM/mL and 100 µM/mL.
2. Experimental animals: Each group included more than 50 fruit flies.
3. Administration method and statistics of test effects: A piece of filter paper was placed in an empty fruit fly vial, 200 µL of a 5% glucose solution containing 30 µM or 100 µM test substance or vehicle control was added dropwise onto the filter paper, and 7-day-old fruit flies were transferred into the vial containing the test substance or the vehicle control. At time points after drug administration, the number of dead fruit flies was recorded, and a survival curve was plotted. Table 2 lists the experimental results.

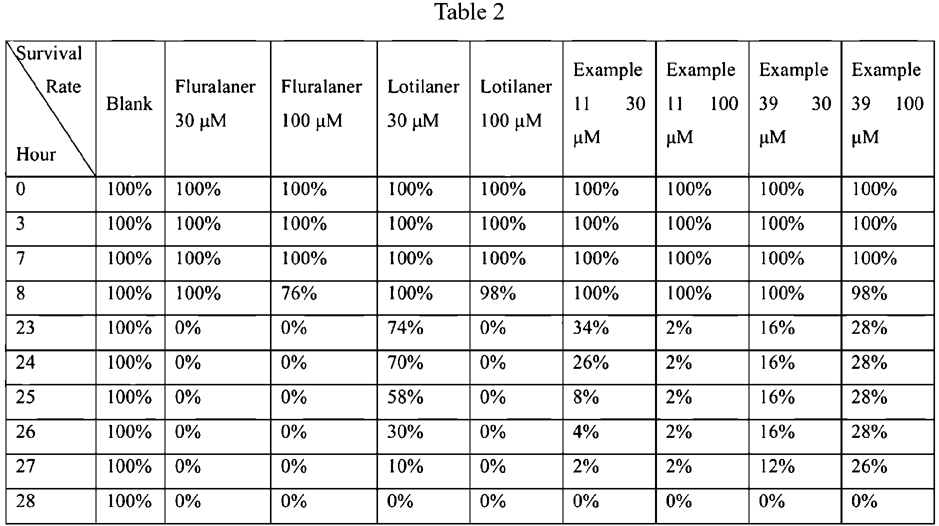

### Test Example 3 Killing efficacy test against agricultural pests

Solution preparation: 2 mg of a test compound was weighed and dissolved in 200 µL of acetone, and the sample was diluted with an aqueous solution containing 0.1% Tween 80 to a required concentration to obtain a test solution.
1. Diamondback moth test: The test sample was prepared into solutions with final concentrations of 0.0156 mg/L, 0.125 mg/L, and 1 mg/L. Fresh and clean rapeseed leaves were cut into small pieces of 3 cm × 3 cm. The leaf pieces were immersed in the solutions with different concentrations for 10s, taken out, and air-dried naturally. The treated leaf pieces were placed into Petri dishes lined with moistened filter paper. 20 third-instar larvae of the diamondback moth were introduced into each Petri dish. The Petri dishes were placed in a constant-temperature room, and mortality rates were checked after 72 h.
2. Aphid test: The test sample was prepared into solutions with final concentrations of 2 mg/L, 10 mg/L, and 50 mg/L. Fresh cotton leaves of uniform size were immersed in the solutions with different concentrations for 10s, air-dried, and placed backside up on culture plates. 100 wingless adult aphids were introduced into each culture plate. The culture plates were sealed with paper strips and placed in a constant-temperature room. Mortality rates were checked after 72 h.
3. Mite test: The test sample was prepared into solutions with final concentrations of 0.02 mg/L, 0.2 mg/L, and 2 mg/L. Filter paper treated with the test solutions was placed in Petri dishes, and about 30 mites were placed at the center of each Petri dish. 0.05 g of mite feed was added to the center of each Petri dish after 30 min. The Petri dishes were placed in a constant-temperature room, and mortality rates were checked after 72 h.
4. Borer test: The test sample was prepared into solutions with final concentrations of 0.125 mg/L, 0.5 mg/L, and 2 mg/L. Fresh leaves were cut, immersed in the solutions with different concentrations for 10s, taken out, and air-dried. The treated leaves were placed in Petri dishes, and 30 borer larvae were introduced into each Petri dish. The Petri dishes were placed in a constant-temperature room, and mortality rates were checked after 72 h. Table 3 lists the experimental results.

**Table 3**

| **Agricultural Pest** | **DiamondBack Moth** | | **Aphid** | | **Mite** | | **Borer** | |
|---|---|---|---|---|---|---|---|---|
| Sample No. | Concentration (mg/L) | Corrected Mortality Rate (%) | Concentration (mg/L) | Corrected Mortality Rate (%) | Concentration (mg/L) | Corrected Mortality Rate (%) | Concentration (mg/L) | Corrected Mortality Rate (%) |
| Example 8 | 0.0156 | 5 | 2 | 86 | 0.02 | 62 | 0.125 | 2 |
| | 0.125 | 100 | 10 | 100 | 0.2 | 92 | 0.5 | 82 |
| | 1 | 100 | 50 | 100 | 2 | 100 | 2 | 100 |
| Example 11 | 0.0156 | 0 | 2 | 60 | 0.02 | 34 | 0.125 | 29 |
| | 0.125 | 100 | 10 | 100 | 0.2 | 86 | 0.5 | 93 |
| | 1 | 100 | 50 | 100 | 2 | 100 | 2 | 100 |
| Example 29 | 0.0156 | 0 | 2 | 5 | 0.02 | 54 | 0.125 | 0 |
| | 0.125 | 79 | 10 | 100 | 0.2 | 70 | 0.5 | 50 |
| | 1 | 100 | 50 | 100 | 2 | 100 | 2 | 100 |
| Example 30 | 0.0156 | 16 | 2 | 22 | 0.02 | 34 | 0.125 | 30 |
| | 0.125 | 63 | 10 | 86 | 0.2 | 66 | 0.5 | 100 |
| | 1 | 100 | 50 | 100 | 2 | 96 | 2 | 100 |

### Test Example 4 In vivo pharmacokinetic evaluation in mice

1. Drug preparation: 5% DMSO + 10% macrogol 15 hydroxystearate (BASF) + 85% saline (where drug solutions have a final concentration of 1.2 mg/mL for the IV group and 3 mg/mL for the PO group).
2. Experimental animals: Each group included three mice (ICR mice, SPF grade).
3. Administration method: The body weight was measured before administration, and the dose was calculated according to the body weight. The mice were administered through intravenous injection or oral gavage.
4. Blood collection: After administration, blood samples were collected in 0.083 h, 0.25 h, 0.5 h, 1 h, 2 h, 4 h, 6 h, 8 h, and 24 h. The blood samples were collected via the cheek. Each sample included about 0.05 mL of blood anticoagulated with EDTA-2K. The blood samples were placed on wet ice after collection.
5. Sample treatment: The blood samples were placed on ice after collection and centrifuged within 1 h to obtain plasma (centrifugation conditions: 6000 g, 3 min, 2-8°C). Plasma samples were stored in a -80°C refrigerator before analysis.
6. Data processing: Pharmacokinetic parameters were calculated using Phoenix WinNonlin 8.2.0 based on plasma concentration data at different time points. Table 4 lists the experimental results.

**Table 4**

| Test Drug | | **Compound 8** | **Compound 11** | **Example 39 (Comparative Example 1)** | **Fluralaner** |
|---|---|---|---|---|---|
| IV (6 mg/kg) | T_{1/2} (h) | 37.6 | 20.0 | 12.3 | 11.9 |
| | AUC^{1-24 h} (h × ng/mL) | 56120 | 48855 | 37091 | 22972 |
| | C₀ (ng/mL) | 10328 | 6797 | 7804 | 7151 |
| | Vₛₛ (L/kg) | 1.93 | 1.96 | 1.97 | 2.87 |
| | CL (mL/kg/min) | 0.713 | 1.18 | 2.06 | 3.49 |
| PO (30 | T_{1/2} (h) | 31.6 | 27.8 | 11.8 | 15.2 |
| mg/kg) | AUC^{1-24 h} (h × ng/mL) | 233172 | 231036 | 172618 | 93520 |
| | Tₘₐₓ (h) | 9.33 | 2.67 | 3.33 | 1.67 |
| | Cₘₐₓ (ng/mL) | 15333 | 15933 | 12313 | 8000 |
| | F (%) | 83.10 | 94.58 | 93.10 | 81.42 |

The results show that the example compounds according to the present application exhibit good pharmacokinetic properties in mice.

The applicant has stated that although the isoxazoline compound and the use thereof in the present application are described through the preceding embodiments, the present application is not limited to the preceding embodiments, which means that the implementation of the present application does not necessarily depend on the preceding embodiments. It is to be understood by those skilled in the art that any improvements made to the present application and equivalent replacements of raw materials of the product, additions of adjuvant ingredients, selections of specific manners, etc. in the present application all fall within the scope of the present application.

## Claims

1. An isoxazoline compound having a structure represented by Formula I: 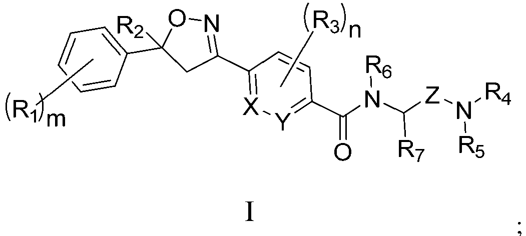 wherein
X is selected from -CH= or -N=;
Y is selected from -CH= or -N=;
Z is selected from 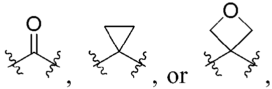 wherein the wavy line represents a linkage site of the group;
R₁ is selected from hydrogen, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, halogenated C₂ to C₆ alkynyl, halogen, cyano, nitro, -C(O)NRₐR_{b}, -C(O)Rₐ, -C(O)ORₐ, -ORₐ, -RₐOR_{b}, -OC(O)Rₐ, -OC(O)ORₐ, -OC(O)NRₐR_{b}, -NRₐR_{b}, -SRₐ, -S(O)Rₐ, -S(O)₂Rₐ, or a 3- to 10-membered saturated or unsaturated ring containing 0 to 3 heteroatoms, wherein the 3- to 10-membered saturated or unsaturated ring containing 0 to 3 heteroatoms is optionally substituted with 1 to 3 Rₐ;
R₂ is selected from hydrogen, halogen, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, or halogenated C₂ to C₆ alkynyl;
R₃ is selected from hydrogen, halogen, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, or halogenated C₂ to C₆ alkynyl;
R₄ is selected from hydrogen, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, halogenated C₂ to C₆ alkynyl, halogen, cyano, nitro, -C(O)NRₐR_{b}, -C(O)Rₐ, -C(O)ORₐ, -ORₐ, -RₐOR_{b}, -OC(O)Rₐ, -OC(O)ORₐ, -OC(O)NRₐR_{b}, -NRₐR_{b}, -SRₐ, -S(O)Rₐ, -S(O)₂Rₐ, or a 3- to 10-membered saturated or unsaturated ring containing 0 to 3 heteroatoms, wherein the 3- to 10-membered saturated or unsaturated ring containing 0 to 3 heteroatoms is optionally substituted with 1 to 3 Rₐ;
R₅ is selected from hydrogen, C₁ to C₆ alkyl, C₁ to C₆ alkyl substituted with 1 to 3 Rₐ, C₂ to C₆ alkenyl, C₂ to C₆ alkynyl, halogenated C₁ to C₆ alkyl, deuterated C₁ to C₆ alkyl, halogenated C₂ to C₆ alkenyl, halogenated C₂ to C₆ alkynyl, halogen, cyano, nitro, -C(O)NRₐR_{b}, -C(O)Rₐ, -C(O)ORₐ, -ORₐ, -RₐOR_{b}, -OC(O)Rₐ, -OC(O)ORₐ, -OC(O)NRₐR_{b}, -NRₐR_{b}, -SRₐ, -S(O)Rₐ, -S(O)₂Rₐ, or a 3- to 10-membered saturated or unsaturated ring containing 0 to 3 heteroatoms, wherein the 3- to 10-membered saturated or unsaturated ring containing 0 to 3 heteroatoms is optionally substituted with 1 to 3 Rₐ; or
R₄ and R₃, together with the nitrogen atom connecting them, form a 3- to 7-membered saturated or unsaturated ring optionally substituted with 1 to 8 Rₐ;
R₆ is selected from hydrogen, C₁ to C₆ alkyl, C₂ to C₆ alkenyl, halogenated C₁ to C₆ alkyl, C₁ to C₆ alkoxy, or deuterated C₁ to C₆ alkyl;
R₇ is selected from hydrogen, halogen, C₁ to C₆ alkyl, C₂ to C₆ alkenyl, C₂ to C₆ alkynyl, halogenated C₁ to C₆ alkyl, C₁ to C₆ alkoxy, deuterated C₁ to C₆ alkyl, halogenated C₂ to C₆ alkenyl, or halogenated C₂ to C₆ alkynyl;
Rₐ and R_{b} are each independently selected from hydrogen, deuterium, C₁ to C₆ alkyl, C₂ to C₆ alkenyl, C₂ to C₆ alkynyl, halogenated C₁ to C₆ alkyl, deuterated C₁ to C₆ alkyl, halogenated C₂ to C₆ alkenyl, halogenated C₂ to C₆ alkynyl, halogen, cyano, nitro, amino, carboxyl, oxo, hydroxyl, hydroxyalkyl, alkoxy, halogenated alkoxy, deuterated alkoxy, C₃ to C₆ cycloalkyl, halogenated C₃ to C₆ cycloalkyl, alkoxy-substituted C₃ to C₆ cycloalkyl, C₃ to C₆ cycloalkyl substituted with 1 to 3 R_{c}, C₃ to C₆ heterocyclyl, halogenated C₃ to C₆ heterocyclyl, alkyl-substituted C₃ to C₆ heteroaryl, -S(O)₂R_{c}, -OR_{c}OR_{d}, -R_{c}ORₐ, -C(O)R_{c}, or -OC(O)R_{c}, wherein R_{c} and R_{d} are each independently selected from halogen, C₁ to C₆ alkyl, or halogenated C₁ to C₆ alkyl;
m is 0, 1, 2, 3, 4, or 5; and
n is 0, 1, 2, 3, or 4.

2. The isoxazoline compound according to claim 1, wherein the isoxazoline compound has a structure represented by Formula Ia: 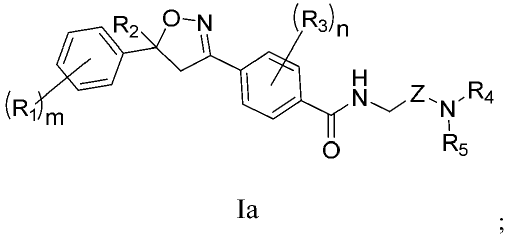 wherein Z, R₁, R₂, R₃, R₄, R₅, m, and n are defined the same as in claim 1.

3. The isoxazoline compound according to claim 1 or 2, wherein the isoxazoline compound has a structure represented by Formula Ib: 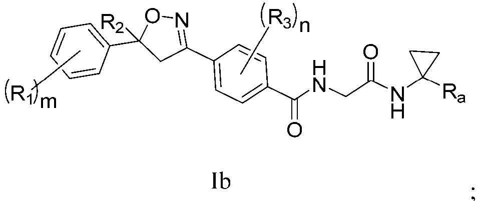
wherein R₁, R₂, R₃, m, and n are defined the same as in claim 1; and
Rₐ is selected from cyano or trifluoromethyl.

4. The compound of Formula I according to claim 1, wherein R₁ is selected from hydrogen, halogen, C₁ to C₆ alkyl, C₁ to C₆ alkoxy, halogenated C₁ to C₆ alkyl, or halogenated C₁ to C₆ alkoxy.

5. The compound of Formula I according to claim 1, wherein R₁ is selected from hydrogen, fluorine, chlorine, methyl, methoxy, trifluoromethyl, or trifluoromethoxy.

6. The compound of Formula I according to claim 1, wherein R₂ is selected from hydrogen, halogen, C₁ to C₆ alkyl, or halogenated C₁ to C₆ alkyl.

7. The compound of Formula I according to claim 1, wherein R₂ is trifluoromethyl.

8. The compound of Formula I according to claim 1, wherein R₃ is selected from hydrogen, halogen, C₁ to C₆ alkyl, or halogenated C₁ to C₆ alkyl.

9. The compound of Formula I according to claim 1, wherein R₃ is methyl, chlorine, or fluorine.

10. The compound of Formula I according to claim 1, wherein R₄ is hydrogen.

11. The compound of Formula I according to claim 1, wherein R₅ is selected from 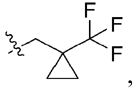 C₁ to C₆ alkyl, halogenated C₁ to C₆ alkyl, C₃ to C₆ cycloalkyl, halogenated C₃ to C₆ cycloalkyl, cyano-substituted C₃ to C₆ cycloalkyl, alkoxy-substituted C₃ to C₆ cycloalkyl, C₃ to C₆ cycloalkyl substituted with halogenated C₁ to C₆ alkyl, or C₃ to C₆ heterocyclyl substituted with halogenated C₁ to C₆ alkyl.

12. The compound of Formula I according to claim 1, wherein R₅ is selected from trifluoroethyl, cyclopropyl,

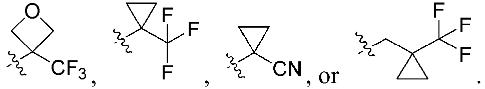

13. The compound of Formula I according to claim 1, wherein 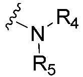 is selected from 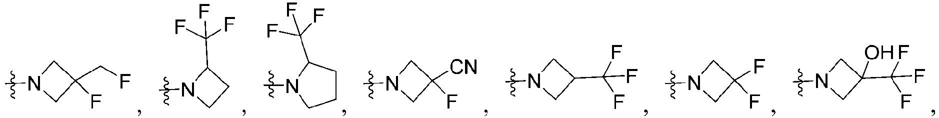 or

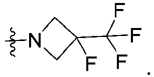

14. The compound of Formula I according to claim 1, wherein R₆ is selected from hydrogen or C₁ to C₆ alkyl.

15. The compound of Formula I according to claim 1, wherein R₇ is selected from hydrogen or C₁ to C₆ alkyl.

16. An isoxazoline compound selected from any one of the following compounds: 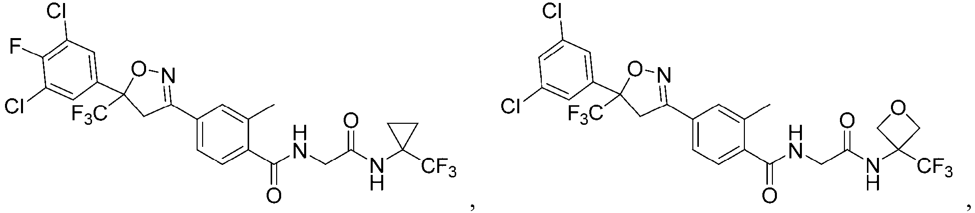 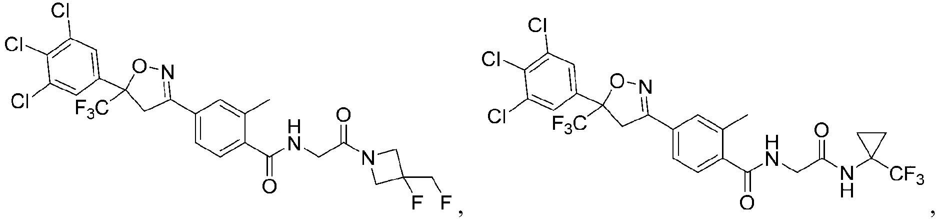 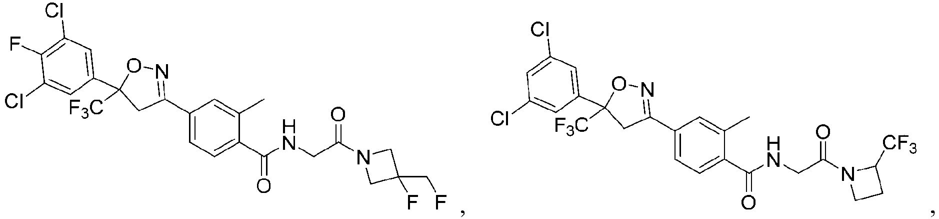 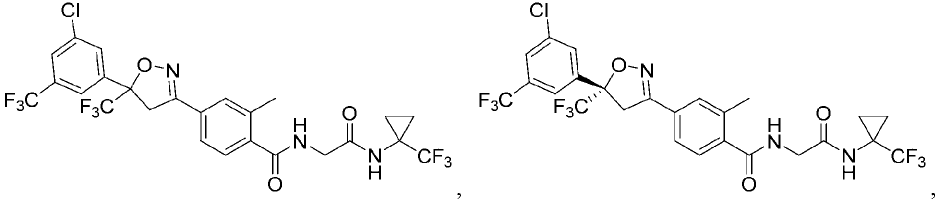 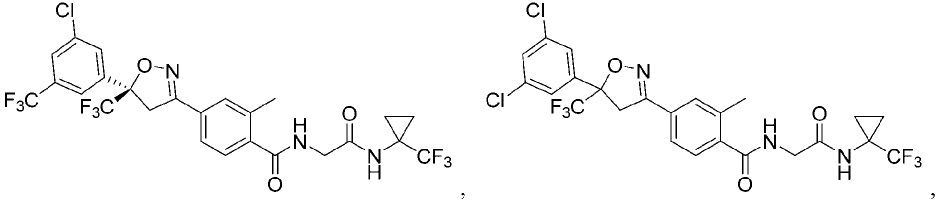 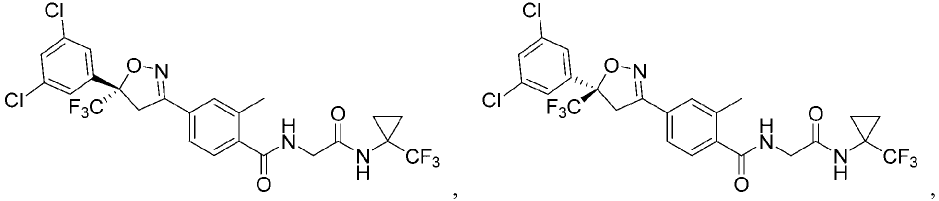 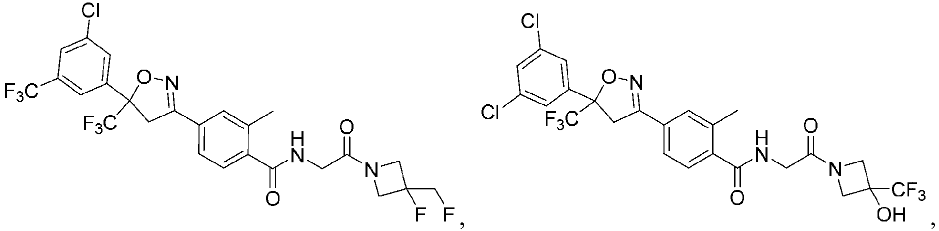 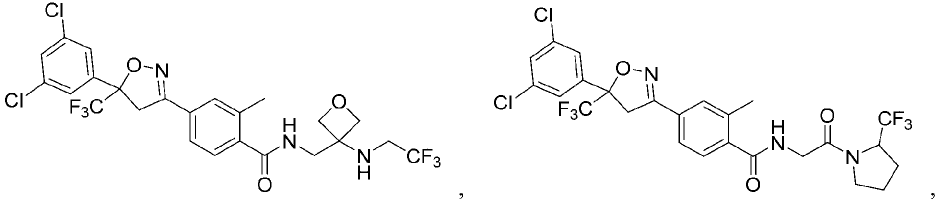 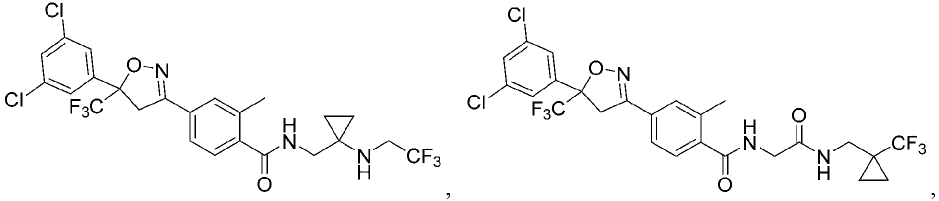 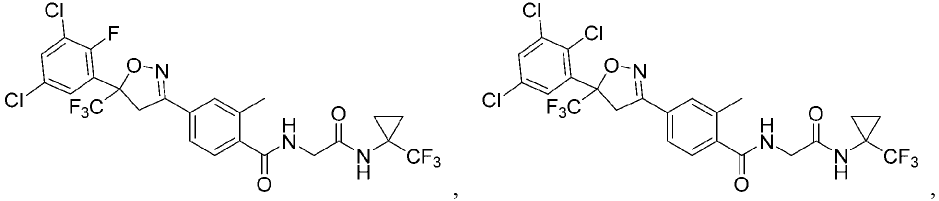 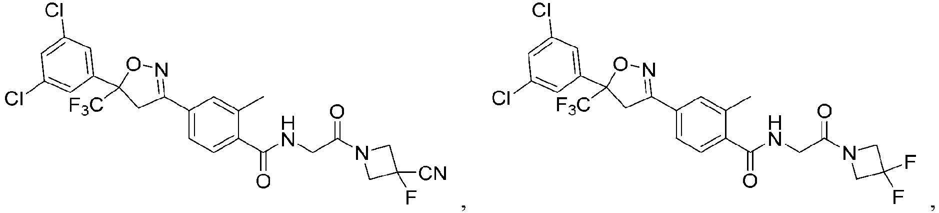 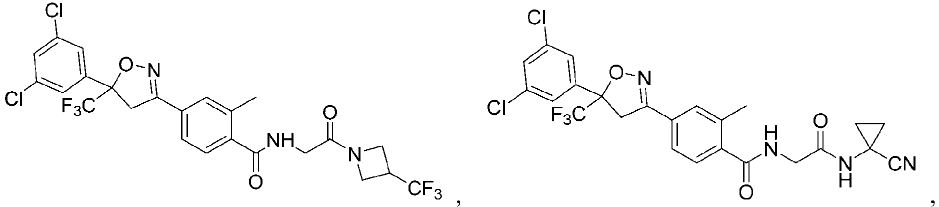 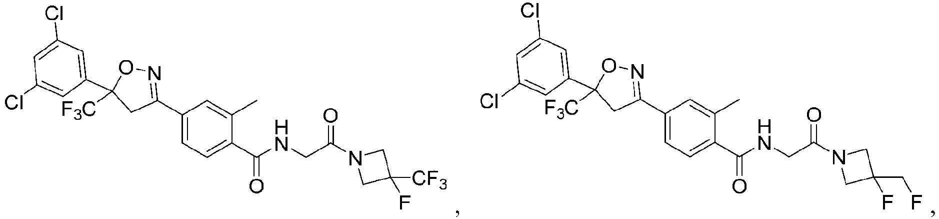 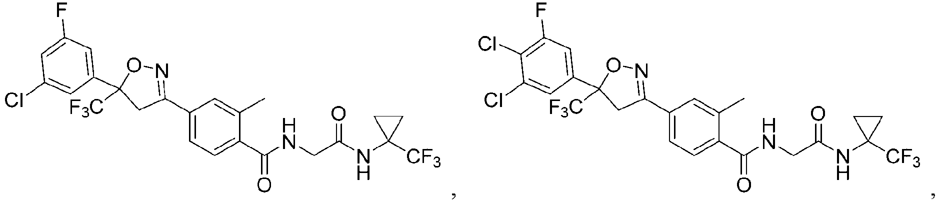 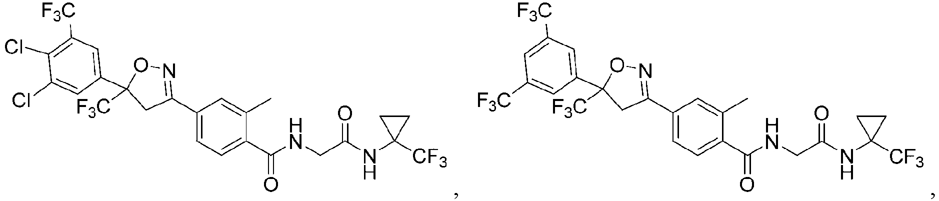 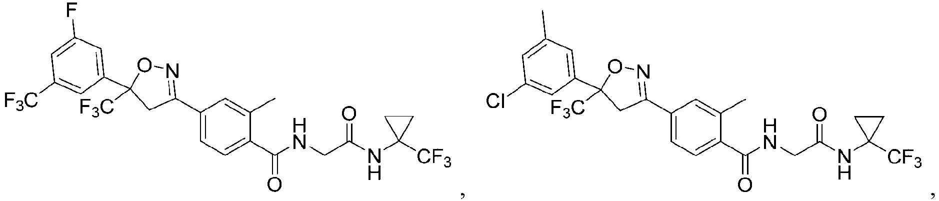 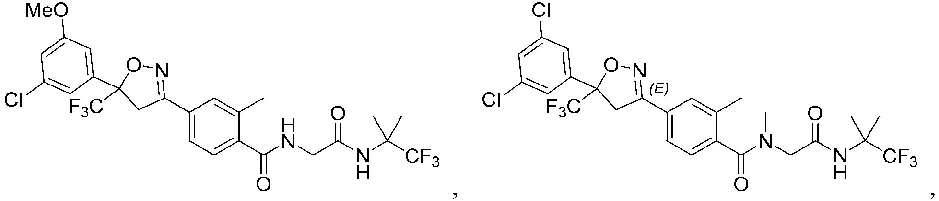 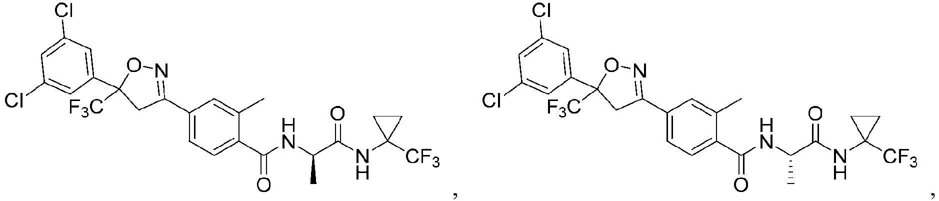 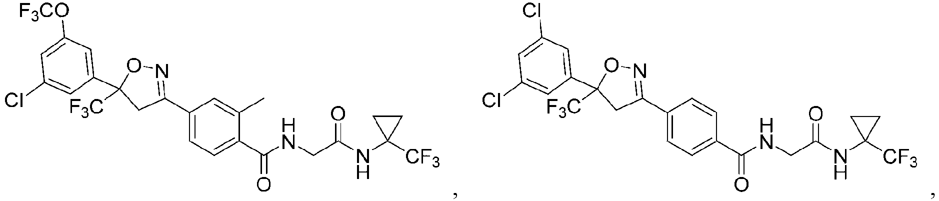 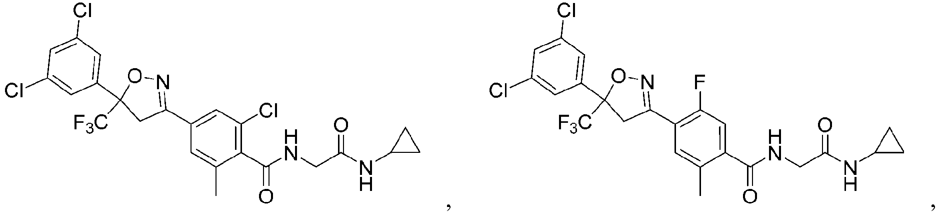 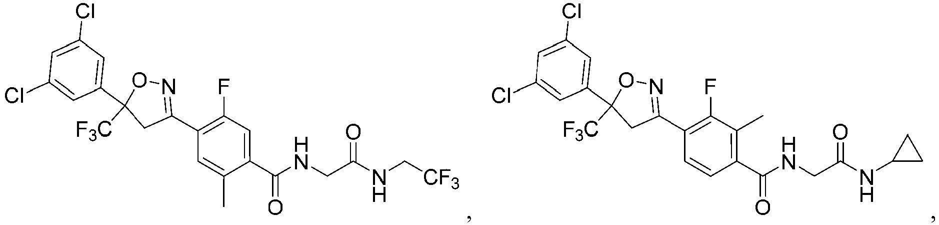 or

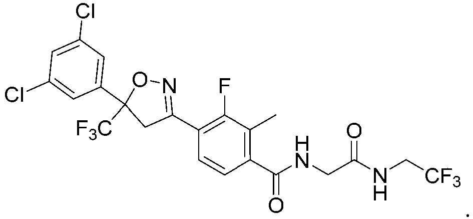

17. The isoxazoline compound according to claim 16, wherein the isoxazoline compound is selected from any one of the following compounds: 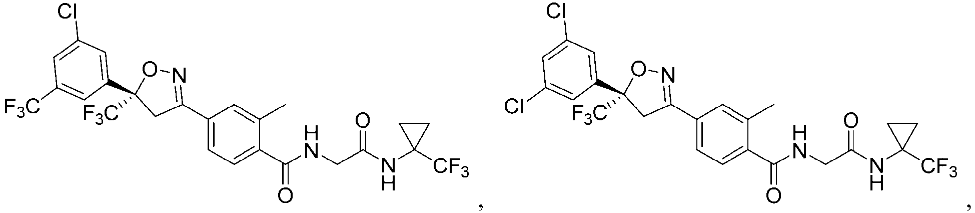 or

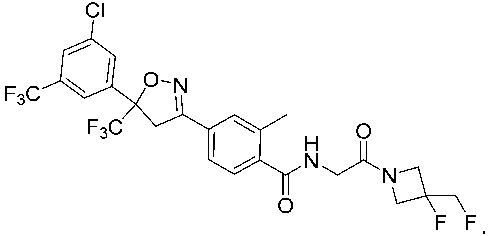

18. A tautomer, enantiomer, diastereomer, mesomer, racemate, or pharmaceutically acceptable salt of the isoxazoline compound according to any one of claims 1 to 17.

19. A pharmaceutical composition, comprising a therapeutically effective amount of the isoxazoline compound according to any one of claims 1 to 17 or a tautomer, enantiomer, diastereomer, mesomer, racemate, or pharmaceutically acceptable salt thereof and a pharmaceutically acceptable carrier or excipient.

20. Use of the isoxazoline compound according to any one of claims 1 to 17, the tautomer, enantiomer, diastereomer, mesomer, racemate, or pharmaceutically acceptable salt thereof according to claim 18, or the pharmaceutical composition according to claim 19 for controlling an infection of a human or an animal with ectoparasites.

21. A pesticide composition, comprising an active ingredient and optionally at least one agromedically acceptable carrier and/or adjuvant, wherein the active ingredient is the isoxazoline compound according to any one of claims 1 to 17 or the tautomer, enantiomer, diastereomer, mesomer, racemate, or pharmaceutically acceptable salt thereof according to claim 18, and a pharmaceutically acceptable carrier or excipient.

22. A pesticide formulation made of the isoxazoline compound according to any one of claims 1 to 17 or the tautomer, enantiomer, diastereomer, mesomer, racemate, or pharmaceutically acceptable salt thereof according to claim 18.

23. Use of the isoxazoline compound according to any one of claims 1 to 17 or the tautomer, enantiomer, diastereomer, mesomer, racemate, or pharmaceutically acceptable salt thereof according to claim 18 for preparation of a product for controlling agricultural pests.

24. The use for preparation of the product for controlling agricultural pests according to claim 23, wherein the agricultural pests are selected from at least one of mites, Lepidoptera, Diptera, Thysanoptera, Hemiptera, Isoptera, or Coleoptera.
